## Europäisches Patentamt

## European Patent Office

## Office européen des brevets

(19)

(11) Numéro de publication : **0 178 996**
**B1**

(12) **FASCICULE DE BREVET EUROPÉEN**

(45) Date de publication du fascicule du brevet :
13.12.89

(51) Int. Cl.⁴ : **C 12 N 15/00**, C 12 N  5/00,
C 12 N  7/00, C 12 P 21/02

(21) Numéro de dépôt : 85401999.9

(22) Date de dépôt : 15.10.85

(54) Vecteurs de clonage ou d'expression comportant le génome du virus de l'érythroblastose aviaire et cellules transfectées par ces vecteurs.

(30) Priorité : 15.10.84 FR 8415764

(43) Date de publication de la demande :
23.04.86 Bulletin 86/17

(45) Mention de la délivrance du brevet :
13.12.89 Bulletin 89/50

(84) Etats contractants désignés :
AT BE CH DE FR GB IT LI LU NL SE

(56) Documents cités :
EP-A- 0 105 141
EP-A- 0 155 198
US-A- 4 405 712
VIROLOGY, vol. 130, 1983, pages 155-178, Academic Press Inc.; L. SEALY et al.: "Site-specific mutagenesis of avian erythroblastosis virus: erb-B is required for oncogenicity"
Idem
PROC. NATL. ACAD. SCI. USA, vol. 80, août 1983, pages 4709-4713; A.D. MILLER et al.: "A transmissible retrovirus expressing human hypoxanthine phosphoribosyltransferase (HPRT): Gene transfer into cells obtained from humans deficient in HPRT"
Idem
M. INOUYE: "Experimental manipulation of gene expression", 1983, chapitre 8, pages 155-173, Academic Press, New York, US;R.C. MULLIGAN: "Construction of highly transmissible mammalian cloning vehicles derived from murine retroviruses"
Idem

(73) Titulaire : INSTITUT NATIONAL DE LA RECHERCHE AGRONOMIQUE (INRA)
145, Rue de l'Université
F-75341 Paris Cédex 07 (FR)

(72) Inventeur : Samarut, Jacques
169 bis, Route de Genas
F-69100 Villeurbanne (FR)
Inventeur : Verdier, Gérard
5 bis, rue de la Libération
F-69270 Fontaines S/Saone (FR)
Inventeur : Benchaibi, Miloud
7, rue Jean-Claude Vivant
F-69100 Villeurbanne (FR)
Inventeur : Savatier, Pierre
3, rue Béranger
F 69006 Lyon (FR)
Inventeur : Poncet, Didier
205, Avenue Lacassagne
F-69003 Lyon (FR)
Inventeur : Flamant, Frédéric
45, rue de Brest
F-69002 Lyon (FR)
Inventeur : Xiao, Jiao-Hao
Chambre 422 Cité Universitaire 11, rue Louvois
F-67084 Strasbourg Cedex (FR)
Inventeur : Thoraval, Pierrick
11 Avenue Roberto Rossellini
F-69100 Villeurbanne (FR)
Inventeur : Chambonnet, Frédérique
21 Impasse de la Vivaraize
F-42100 Saint-Etienne (FR)
Inventeur : Nigon, Victor
9, Avenue Condorcet
F-69100 Villeurbanne (FR)

EP 0 178 996 B1

PROCEEDINGS OF THE NATIONAL ACADEMY OF SCIENCES OF THE USA, vol. 79, no. 7, avril 1982, pages 2268-2272, Washington, US; J. DOEHMER et al.: "Introduction of rat growth hormone gene into mouse fibroblasts via a retroviral DNA vector: Expression and regulation"

CELL, vol. 32, janvier 1983, pages 227-238, MIT; L. FRYKBERG et al.: "Transforming capacities of avian erythroblastosis virus mutants deleted in the erbA or erbB oncogenes"

JOURNAL OF VIROLOGY, vol. 36, no. 2, novembre 1980, pages 575-585; B. VENNSTRÖM et al.: "Molecular cloning of the avian erythroblastosis virus genome and recovery of oncogenic virus by transfection of chicken cells"

(74) Mandataire : **Warcoin, Jacques et al**
Cabinet Régimbeau 26, avenue Kléber
F-75116 Paris (FR)

## Description

L'introduction stable de matériel génétique dans des organismes implique, en général, que ce transfert soit effectué dans des cellules précurseurs dont les potentialités de multiplication et de différenciation sont élevées. Ces cellules précurseurs peuvent soit être représentatives d'une voie de différenciation bien particulière, comme par exemple les cellules souches hématopoïétiques, soit totipotentes comme les cellules germinales. Dans tous les cas, la population des cellules cibles du transfert est très minoritaire dans des tissus considérés.

L'introduction stable de séquences génétiques dans ces cellules nécessite donc l'utilisation de techniques de transfert dont le rendement est élevé. Les méthodes classiques d'introduction de fragments d'ADN purifié dans des cellules en culture fournissent des taux d'incorporation faibles.

L'emploi de vecteurs composés comportant des génomes viraux a permis une nette amélioration de l'incorporation d'une séquence exogène dans une cellule en culture.

La présente invention concerne de nouveaux vecteurs de ce type et en particulier des vecteurs de transfert de gènes dérivés du virus de l'érythroblastose aviaire ou d'un virus apparenté.

Les rétrovirus doivent à leur structure particulière l'aptitude à s'intégrer facilement sous forme de provirus dans l'ADN génomique des cellules qu'ils infectent. Les éléments structuraux qui assurent cette intégration sont portés aux deux extrémités du provirus et sont appelés « Long Terminal Repeat » ou LTR.

Cette facilité d'insertion des rétrovirus au sein du génome des cellules qu'ils infectent a conduit à l'idée de leur utilisation comme vecteur potentiel pour le transfert de matériel génétique.

Le génome du rétrovirus de l'érythroblastose aviaire (AEV) renferme deux gènes onc appelés v-erbA et v-erbB et dont les expressions sont responsables du pouvoir oncogène de ce rétrovirus.

Dans un premier mode de mise en oeuvre de l'invention, le pouvoir oncogène du virus de l'AEV a été utilisé comme marqueur de sélection et son aptitude d'insertion pour le transfert dans des cellules en culture d'un gène exogène inséré dans ce rétrovirus.

Dans un second mode de mise en œuvre de l'invention, le pouvoir oncogène du virus de l'AEV est supprimé et le gène marqueur est un gène étranger, par exemple de résistance à un antibiotique tel que le G418.

Une étude rapide des caractéristiques des rétrovirus permettra de mieux comprendre l'invention.

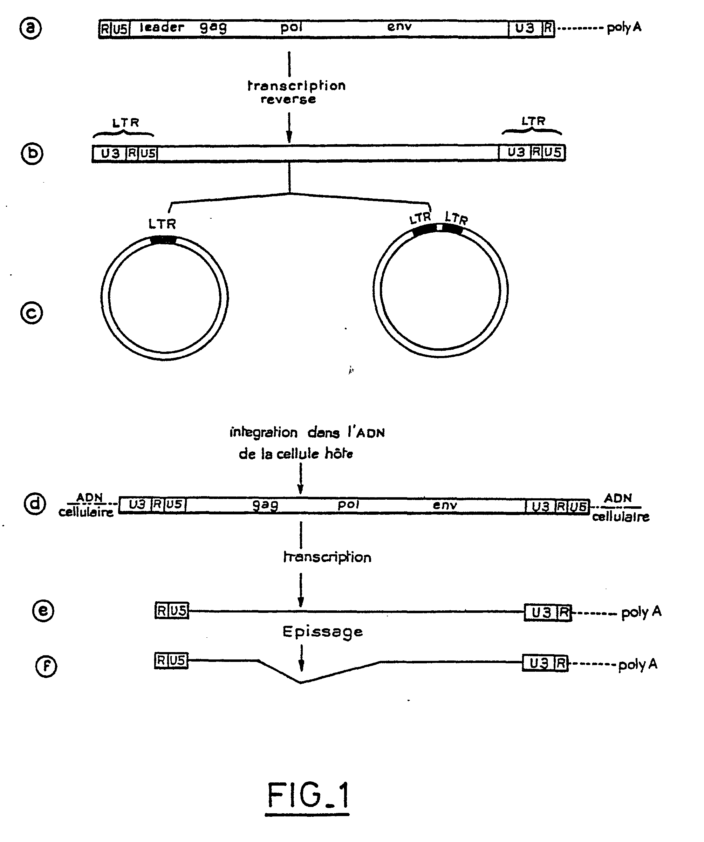

Le génome d'un rétrovirus non défectif pour la réplication (figure 1) est formé d'une molécule d'ARN présentant dans le sens de la transcription (5'→3') une séquence courte identique à chacune des extrémités et appelée R (entre 21 et 80 bases). Elle est suivie, dans l'ordre, d'une séquence unique appelée U5 (de 70 à 80 bases), d'un site de fixation d'un ARNt (TBS, environ 20 bases), et d'une séquence non codante (séquence « leader »). La molécule d'ARN se poursuit par une région codant pour trois gènes dont les produits de traduction sont indispensables pour la réplication du virus et qui sont gag (protéines structurales des virions), pol (polymérase réverse), env (enveloppe). Le génome se termine, dans l'ordre, par une séquence non codante, une séquence riche en purines (PU), une séquence unique appelée U3 (de 200 à 450 bases), et enfin la séquence R. Les séquences extrêmes répétées (R) ou uniques (U5 et U3) particulières aux rétrovirus semblent assez bien conservées dans ce groupe de virus et renferment les signaux impliqués dans le contrôle de l'expression du génome viral.

Certains rétrovirus tels que l'AEV, responsables de transformations néoplasiques entraînant des leucémies ou des tumeurs, doivent leur pouvoir transformant à la présence de séquences particulières « onc » dans leur génome. Ces séquences onc ont une origine cellulaire et ont été intégrées dans le génome viral à la suite de recombinaisons avec le génome des cellules hôtes du virus. Dans la plupart de ces virus, cette intégration s'accompagne de la perte totale ou partielle des gènes gag, pol et env. Ces virus sont par conséquent défectifs pour la réplication. Pour se répliquer, ces virus nécessitent la présence dans la même cellule hôte d'un virus assistant fonctionnel.

Les virus assistants (helper) sont capables de se répliquer. Leur génome ne renferme que les gènes fonctionnels gag, pol et env.

Le cycle d'infection par un rétrovirus débute par l'absorption des virions à la surface des cellules, suivie de la pénétration dans le cytoplasme. Les étapes représentant la réplication et le cycle viral d'un rétrovirus sont résumées dans la figure 1. Dans le cytoplasme de la cellule, l'ARN viral simple brin (a) est transcrit par la polymérase réverse, présente dans le virion, en une copie linéaire d'ADN double brin (b). La copie d'ADN résultant de cette transcription réverse est légèrement plus longue que la molécule d'ARN viral qui lui a servi de matrice. Cette différence est le résultat de l'addition d'une séquence U3 à l'extrémité 5' et d'une séquence U5 à l'extrémité 3'.

La combinaison dans l'ordre des séquences U3-R-U5 constitue une séquence répétée aux deux extrémités de la molécule d'ADN appelée LTR (Long Terminal Repeat). Les copies d'ADN viral renfermant une ou deux séquences LTR sont acheminées jusqu'au noyau où elles sont converties en molécules de formes circulaires (c). Certaines molécules circulaires ne présentent plus qu'une seule LTR. Ces molécules sont ensuite intégrées dans l'ADN génomique cellulaire (d). L'ADN viral est intégré dans l'ADN cellulaire de telle sorte qu'il se trouve encadré par une LTR à chaque extrémité et porte alors le nom d'ADN proviral ou provirus. Par la suite, nous appellerons « LTR gauche » d'un provirus la LTR située en amont du gène gag et « LTR droite » la LTR située en aval du gène env.

C'est le provirus qui sert de matrice pour la transcription de molécules d'ARN virales. La transcription est initiée au niveau de la séquence R de la LTR de gauche et s'arrête au-delà du signal de polyadénylation porté par la séquence U3 ou R de la LTR de droite. Les ARN obtenus après transcription du provirus sont à l'image des ARNm des cellules eucaryotiques, « capés » par un résidu 7mG terminal à l'extrémité 5', et pourvus d'une séquence polyadénylée à leur extrémité 3' terminale.

Les molécules d'ARN s'associent en dimères par des liaisons hydrogènes à leur extrémité 5' et sont encapsidées dans l'enveloppe protéique virale. Les particules virales forment un ensemble qui sédimente à 7OS. Elles sont rejetées à l'extérieur de la cellule productrice et vont infecter les cellules voisines. Cette libération de particules virales ne s'accompagne pas de la mort de la cellule.

L'utilisation du rétrovirus AEV comme vecteur de clonage ou d'expression d'un gène étranger (ci-après AEV vecteur) peut revêtir différentes formes compte tenu de son processus de développement.

La description ci-après se réfère plus particulièrement au virus AEV, mais l'invention concerne de façon générale les rétrovirus aviaires. Parmi les rétrovirus, l'invention est plus particulièrement intéressante pour les rétrovirus dans lesquels deux gènes peuvent être exprimés simultanément à partir du même promoteur, en particulier du promoteur contenu dans les LTR et qui dépendent de deux ARN génomique et sous-génomique.

Lorsque l'AEV vecteur est mis en oeuvre dans des conditions permettant sa réplication et la formation de virions, c'est-à-dire avec un virus assistant, l'infection de culture de cellules in vitro pourra être réalisée avec une efficacité considérable compte tenu de la multiplication des virions infectieux.

Mais, d'autre part, l'intégration sous forme de provirus permet d'envisager la modification permanente du génome d'un ensemble de cellules sans que le caractère infectieux persiste en l'absence de virus assistant.

La présente invention concerne donc l'utilisation de rétrovirus aviaires, en particulier de l'AEV, à titre de vecteurs de clonage ou d'expression d'un gène étranger.

Bien entendu, lorsque l'AEV est utilisé à titre de vecteur pour les besoins de la réalisation du vecteur ou pour des raisons pratiques d'utilisation, une partie du génome pourra avoir été délétée ou modifiée.

Néanmoins, on continuera de parler d'AEV dans la mesure où le virus obtenu continuera de garder les caractéristiques essentielles du virus de l'AEV sauvage, c'est-à-dire l'existence de séquences LTR qui assurent son intégration.

Les termes « vecteur de clonage ou d'expression » sont connus dans le domaine du génie génétique.

En outre, compte tenu du fait que l'AEV est un virus à ARN, la terminologie « virus » pourra parfois désigner le génome viral sous forme d'ADN, de même que dans certains cas le « gène » étranger pourra être sous forme d'ARN, en particulier lorsqu'il est inséré dans le génome d'un virion.

Les techniques permettant de passer d'une forme ADN à la forme ARN et réciproquement sont connues et sont la transcription et la transcription réverse.

Par « gène étranger » on entend désigner un gène qui ne se trouve pas dans le génome de l'AEV naturel et de préférence qui ne se trouve pas dans le génome d'un virus.

Le virus recombinant peut être utilisé tel quel sous forme de virion à ARN, mais le génome viral sous forme d'ADN comportant l'insertion d'un gène étranger, lui aussi sous forme d'ADN et correspondant à la transcription reverse de l'ARN du virus, peut être intégré dans un vecteur à ADN tel qu'un plasmide, un cosmide ou un phage par exemple.

La caractéristique essentielle de ces vecteurs est que le gène étranger est inséré entre les deux séquences LTR du rétrovirus, en particulier de l'AEV.

Les rétrovirus en cause sont, le plus souvent, défectifs, c'est pourquoi lorsque le vecteur en cause doit être répliqué, il sera utilisé en présence d'un virus assistant fonctionnel qui renfermera au moins les gènes fonctionnels manquant pour la réplication de l'AEV, c'est-à-dire en général les gènes gag, pol et env.

De façon générale, le ou les gènes étrangers doivent donc être insérés de manière à préserver l'initiation et l'arrêt de la transcription, le site d'addition de polyA dans la LTR et les signaux d'épissage.

Comme cela a été dit précédemment, ce type de vecteur pourra être utilisé sous deux formes essentielles suivant la nature du gène marqueur utilisé.

En effet, le gène marqueur peut mettre à profit les propriétés oncogènes de l'AEV, on aura alors des vecteurs oncogènes plus particulièrement utilisables pour les cultures cellulaires, ou bien l'activité oncogène aura été supprimée et remplacée par un gène marqueur de résistance à une drogue analogue à un antibiotique tel que le G418, on aura alors un vecteur non oncogène utilisable par exemple pour certains transferts de gènes « in vivo ».

## Les vecteurs oncogènes

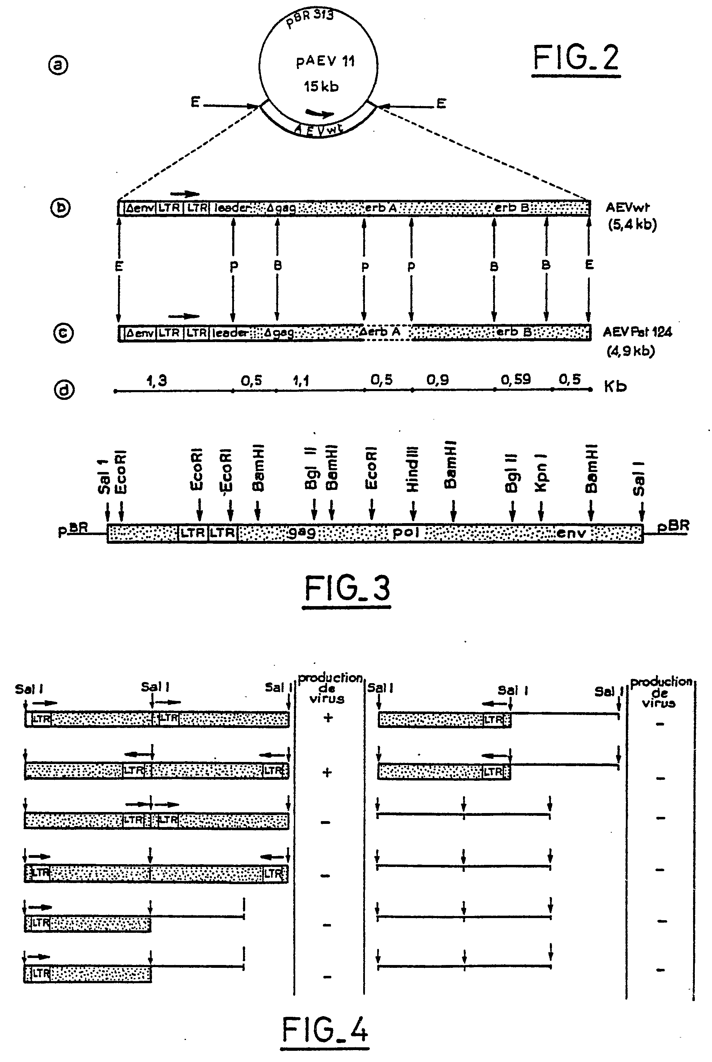

Le génome de l'AEV, schématisé à la figure 2, b, est constitué d'une séquence « leader » située immédiatement en aval de la région U5. Cette séquence est suivie d'un gène gag tronqué dans sa moitié 3'. Le gène pol est absent.

Les gènes onc v-erbA et v-erbB couvrent environ 3 kb. Ces deux gènes sont séparés par une séquence de jonction renfermant un site accepteur d'épissage.

Le génome de l'AEV est donc totalement dépourvu du gène pol et partiellement des gènes env et gag. La réplication du virus nécessite de ce fait l'infection simultanée par un virus assistant. Dans ce travail le virus assistant est RAV-2 dont le génome est représenté sur la figure 3.

Par recombinaison génétique « in vitro » il a été possible de créer des délétions respectivement dans les gènes v-erbA et v-erbB. De l'analyse du pouvoir transformant des virus mutants ainsi créés il ressort que :

● le gène v-erbB assure à lui seul la transformation des fibroblastes « in vitro » et l'induction de sarcomes « in vivo » ;

● le gène v-erbA seul semble incapable d'induire aucune transformation « in vitro », les résultats des analyses « in vivo » concernant le gène v-erbA restent ambigus ;

● la délétion du gène v-erbA seul entraîne une réduction du pouvoir transformant sur les précurseurs érythrocytiques « in vitro ».

Ce pouvoir oncogène de l'AEV, localisé essentiellement sur erbB, peut être utilisé comme un moyen de contrôle de la transformation de cellules exposées à ce virus. De ce point de vue, le caractère transformant des rétrovirus en général et de l'AEV en particulier est à rapprocher de celui résultant des gènes codant pour l'expression de marqueurs dominants (gène xgprt ou gène Neo<sup>r</sup>) qui sont mis en oeuvre dans le second mode de réalisation de l'invention. L'avantage de cette transformation oncogène induite par un rétrovirus est qu'elle entraîne des changements physiologiques qui confèrent aux cellules transformées un pouvoir multiplicatif accru. Notamment l'aptitude particulière des fibroblastes transformés à se développer dans l'agar mou offre une méthode de sélection des cellules ayant incorporé et exprimant le génome viral.

Dans le but de préserver la fonction du gène v-erbB indispensable au développement du pouvoir oncogène, les gènes étrangers sont insérés soit dans le gène v-erbA soit en aval du gène v-erbB et avant la séquence LTR droite.

Le choix de ces sites d'insertion doit tenir compte, en plus de la préservation du gène v-erbB, de celle d'autres zones « sensibles » du virus (initiation et arrêt de la trancription, site d'addition de polyA dans la LTR, signaux d'épissage).

Les expériences réalisées ont montré que le gène étranger pouvait être orienté dans l'un ou l'autre sens par rapport au sens de lecture du génome viral.

L'insertion du gène étranger dans le site EcoRI (2) de l'AEV a conduit à deux vecteurs actifs, AEVdelta (28) et AEVdelta (17), incorporant le gène de la globine delta humaine, qui peuvent être utilisés en remplaçant le gène de la globine par un autre gène.

Dans les expériences réalisées, les éléments d'expression du gène étranger n'ont pas été étudiés systématiquement, mais il est clair que le gène étranger peut être précédé d'éléments d'expression qui lui seront propres, indépendamment des éléments d'expression existant déjà dans le virus.

Pour des raisons de commodité, les constructions ont été réalisées tout d'abord sur des plasmides qui comportent une séquence composite d'ADN correspondant au transcrit réverse d'un virus tel que décrit précédemment.

Bien entendu, ce plasmide peut comporter une partie de plasmide bactérien avec, par exemple, une origine de réplication dans les procaryotes et par exemple un caractère de résistance à un antibiotique, comme cela est connu, mais il peut s'agir également d'un phage ou d'un cosmide.

Des fibroblastes de poulet en culture transfectés par les virus recombinants sous forme de clones d'ADN ont donné lieu à une production de particules virales infectieuses avec des titres très élevés.

Les analyses des ADN des provirus et des ARN issus des particules virales ont montré que les virus recombinants conservent et transmettent le fragment d'ADN du gène humain de globine delta.

Choix de gène humain de globine delta

A titre d'exemple de gène étranger, on a utilisé les gènes de globine, en particulier de la globine delta humaine, car on peut imaginer qu'il pourra aisément être mis en évidence après son incorporation dans le génome de cellules de poulet. Le choix particulier du gène de globine delta repose sur divers critères :

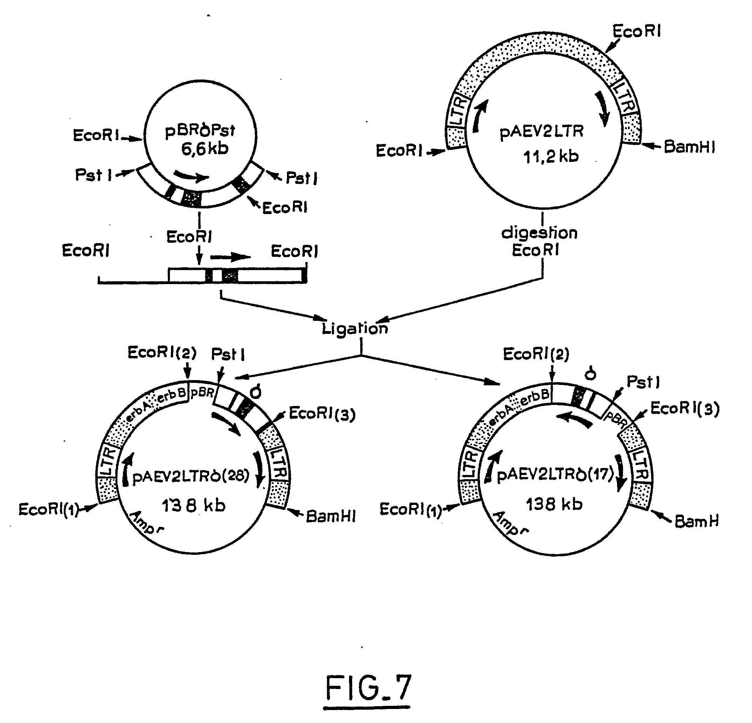

● la cartographie et la séquence ont été établies et un clone deltaPst (figure 7) existe ;

● la taille du fragment deltaPst n'excède pas 2,3 kb, un fragment de taille beaucoup plus grande entraînerait une augmentation de la taille du génome viral qui pourrait inhiber son encapsidation dans les particules virales ;

● cette séquence présente enfin une cartographie de restriction qui facilite son insertion dans le génome de l'AEV.

Les vecteurs non oncogènes

Dans ce second type de vecteur, le gène v-erbB ne s'exprime plus, de préférence parce qu'il a été au moins partiellement délété. Ainsi, dans les vecteurs qui seront étudiés en détail, les 1,6 kb de séquences nucléotides d'origine du gène erbB sont éliminés et remplacés par le gène structural Néo<sup>r</sup> du transposon procaryotique Tn5. Il en résulte trois ADN recombinant pXJ1, pXJ2 et pXJ3.

Bien entendu, l'utilisation du gène structural Neo<sup>r</sup> n'est décrite qu'à titre d'exemple, il est bien

EP 0 178 996 B1

entendu possible d'utiliser d'autres gènes de résistance comme gène marqueur.

Le clone pXJ1 comporte une seule copie du gène Neo$^r$ s'orientant dans le même sens transcriptionnel que celui de l'AEV. Les séquences hétérologues introduites sont longues de 1,2 kb. Elles se composent d'une séquence issue de « polylinker » de clonage du phage mp11, des nucléotides dérivés d'un « linker » BglII, d'un fragment BglII-HincII du transposon bactérien Tn5 dans lequel se situe le gène Neo$^r$, et d'une courte séquence du gène tk de HSV portant notamment le site d'addition de séquences polyA.

Dans le clone pXJ3, deux copies du gène Neo$^r$ sont introduites à l'emplacement du gène erbB délété. Ces deux gènes identiques se disposent en tandem dans la même orientation transcriptionnelle que celle du gène erbB délété. Dans ce recombinant, les séquences hétérologues introduites sont de 2,4 kb. Les jonctions entre les séquences hétérologues et le vecteur rétroviral sont les mêmes que dans le cas de pXJ1.

En somme, la seule différence entre l'AEV sauvage et les vecteurs rétroviraux construits est que l'oncogène viral v-erbB est remplacé par le gène procaryotique Neo$^r$.

Lorsque les bactéries sont transformées par les vecteurs portant le gène Neo$^r$ (pXJ1, pXJ2 et pXJ3), elles acquièrent une résistance à la kanamycine.

Lorsque la concentration de kanamycine dépasse 50 µg/ml de culture, les cellules bactériennes transformées par pXJ1 et pXJ3 montrent une croissance 10 fois plus élevée que celle des cellules transformées par pXJ2.

Le clone pXJ5 dépourvu de gène Neo$^r$ n'induit aucune résistance à la kanamycine des bactéries transformées.

Ainsi, il est possible que la LTR de l'AEV contienne des séquences jouant un rôle de promoteur procaryotique. Cependant, on ne peut exclure la possibilité que le gène Neo$^r$ est activé par des promoteurs potentiels présents dans les séquences conservées de pBR322.

Ces vecteurs ont été introduits dans les cellules L de souris et dans les cellules QT6 de caille par transfection. Seuls les vecteurs pXJ1 et pXJ3 induisent la résistance des cellules transfectées à des doses de G418 supérieures à 300 µg/ml. Ces résultats démontrent que la transcription du gène Neo$^r$ est initiée au niveau du promoteur porté par la LTR gauche.

Le vecteur portant deux gènes Neo$^r$ en tandem induit la résistance des cellules transfectées avec une fréquence beaucoup plus faible que le vecteur pXJ1. Cette propriété pourrait provenir soit de la taille supérieure du vecteur pXJ3 qui réduirait sa capacité d'intégration dans le génome cellulaire, soit de l'existence de recombinaisons entre les deux séquences Neo$^r$ répétées conduisant à des délétions plus ou moins étendues de ces séquences. L'interaction entre la terminaison de la traduction du premier gène et l'initiation de celle du deuxième pourrait également exercer un effet sur l'expression de ces deux gènes.

Il est évident que le gène Neo$^r$, outre son utilisation à titre de marqueur, doit être considéré comme un exemple de l'expression du gène étranger dans un vecteur selon l'invention. Toutefois, dans ce type de vecteur, le gène Neo$^r$ sera utilisé comme marqueur et un autre gène étranger sera incorporé dans le vecteur.

D'autres vecteurs non oncogènes proviennent de l'AEV dans lequel les deux oncogènes v-herbA et v-erbB sont inactivés par délétion partielle ou totale.

Dans ce cas, il est possible d'insérer deux gènes étrangers qui pourront être exprimés simultanément, en particulier si l'un de ces gènes est situé entre les séquences d'épissage et si l'autre gène est situé à l'extérieur des zones d'épissage en aval de celles-ci. Ces deux gènes seront alors traduits à partir de l'ARN génomique pour l'un et à partir de l'ARN sous-génomique pour l'autre.

Les expériences ont mis en évidence le fait qu'il était possible de faire varier la quantité d'ARN génomique par rapport à la quantité d'ARN sous-génomique.

Ainsi, l'insertion du gène Neo$^r$ a été effectuée d'abord dans une séquence laissant subsister plusieurs codons d'initiation dans des phases de lecture différentes, ensuite dans une constuction ne comportant plus que des codons d'initiation dans une même phase de lecture. Il s'avère que cette dernière construction influence le rapport de production ARN génomique/ARN sous-génomique dans un sens favorable à la production d'ARN génomique, c'est-à-dire à l'expression du gène porté par le site placé en 5'. L'utilisation de ce vecteur a comporté la mise au point d'une méthode de titrage spécifique pour les virus aviaires portant le gène Neo.

Les vecteurs particulièrement intéressants selon l'invention sont ceux dans lesquels on a inséré un site de restriction unique entre les deux sites accepteurs et donneurs d'épissage et un site de restriction unique en aval de ces sites d'épissage ; dans ces conditions, il est possible d'insérer dans ces sites des gènes étrangers qui correspondront à un ARN génomique et un ARN sous-génomique lors de la transcription.

De préférence, ces sites seront placés pour que les gènes insérés puissent être en phase dans le cadre de lecture, on dit que les gènes sont synchronisés.

Ces gènes seront de préférence placés pour être sous la dépendance du promoteur du LTR 5'.

L'un des gènes en cause pourra, par exemple, être un gène de sélection tel que Neo$^r$ ou autre.

Comme cela ressort de la description précédente, les gènes étrangers insérés dans les vecteurs selon l'invention ont été exprimés souvent grâce à des éléments d'expression se trouvant dans le génome du virus, il est bien entendu possible d'insérer en amont du gène étranger des éléments d'expression

6

spécifiques qui assurent une expression accrue de la protéine étrangère ; ces éléments d'expression pourront être, par exemple, des promoteurs du virus, en particulier de virus aviaires.

Bien entendu, lorsque le vecteur est un plasmide, un cosmide ou un phage, il comportera également les éléments nécessaires à la réplication de ces derniers dans les cellules procaryotes, de même que les éléments caractéristiques de ce type de vecteur comme les extrémités cos dans le cosmide.

La présente invention concerne, en outre, des cellules transfectées par les vecteurs selon l'invention, qu'ils soient sous forme de virus ou de plasmides. Il est évident que la transfection primaire sera en général effectuée avec des vecteurs de type plasmidique, c'est-à-dire que l'AEV sera sous forme de l'ADN de son génome ; mais, en présence de l'ADN du virus assistant, des cellules aviaires transfectées vont dans ce cas se transformer et libérer des virus recombinants qui vont pouvoir transfecter d'autres cellules.

Ce processus permet d'avoir un rendement de transformation de cellules très élevé qui sera particulièrement précieux dans les cultures cellulaires « in vitro ». Ce type de processus permet également de constituer des stocks de virus vecteurs utilisables par la suite à la place des vecteurs plasmidiques.

Dans le cas où les cellules sont transfectées par des vecteurs oncogènes, leur sélection est naturelle sans qu'il soit nécessaire d'ajouter un agent de sélection, par contre dans le second type de vecteur non oncogène qui comporte un gène codant pour la résistance à un antibiotique, la culture devra être effectuée en présence dudit antibiotique.

Les cellules en cause peuvent être diverses mais sont, de préférence, des cellules aviaires, en particulier des cellules hématopoïétiques, germinales ou de type fibroblastique.

Les cellules préférées pour la mise en oeuvre des vecteurs de l'invention sont les fibroblastes d'embryon de poulet.

Enfin, l'invention concerne le procédé de préparation d'une protéine déterminée, procédé dans lequel on cultive des cellules modifiées par un vecteur AEV selon l'invention dans lequel le gène étranger code pour ladite protéine et en ce que l'on récupère la protéine de la culture cellulaire.

Il faut remarquer que les cellules dans lesquelles le virus s'est intégré sous forme de provirus peuvent constituer des lignées cellulaires, il peut s'agir d'ailleurs de cellules aviaires ou non-aviaires.

Lorsque l'infection est effectuée au niveau des cellules germinales, notamment au niveau de l'oeuf, on peut obtenir des animaux transformés.

Les exemples ci-après permettront de mettre en évidence d'autres caractéristiques et avantages de la présente invention.

Ces exemples sont décrits en référence aux dessins sur lesquels :

la figure 1 est un schéma des divers stades de développement d'un rétrovirus,

la figure 2 représente la structure du génome d'AEVwt et d'AEVPst124,

la figure 3 représente le génome du virus assistant RAV2 sous forme d'ADN double brin,

la figure 4 représente la formation « in vitro » de génomes viraux à ADN portant une LTR à chaque extrémité ; l'ADN du clone pRAV2 est ouvert par SalI puis soumis à l'action de la ligase, sous des conditions qui favorisent la formation de concatémères entre les molécules ; dans le mélange ainsi réalisé quelques molécules se trouvent entourées par une LTR à chaque extrémité ; elles pourront s'intégrer dans l'ADN du génome de la cellule hôte et donner naissance à des virions, on n'a représenté ici que les arrangements en dimères ;

‎ ▨▨▨ : séquence virale de RAV2

—— : séquence du plamide pBR322 le sens des flèches indique le sens de transcription,

la figure 5 représente la construction d'un génome d'ADN d'AEV comportant des séquences LTR à chaque extrémité (pAEV2LTR),

la figure 6 représente du plasmide recombinant pBRdeltaPst et des divers fragments du gène humain de globine delta utilisés dans les expériences de construction de vecteurs de transfert,

la figure 7 représente la construction de vecteurs recombinants du type pAEV2LTRdelta,

la figure 8 représente la structure de AEVdelta (28),

la figure 9 représente des fragments d'ADN clonés utilisés comme sondes,

la figure 10 représente les cartes théoriques de restriction des génomes proviraux de type sauvage (a), mutant AEVdelta (28) (b), mutant AEVdelta (17) (c),

la figure 11 représente le clonage des fragments Bal31 dans mp11,

la figure 12 représente la construction du mutant de l'AEV pAEVΔerbB (pXJ4),

la figure 13 représente l'introduction du gène Neo$^r$ du transposon Tn5 dans le vecteur rétroviral pXJ5,

la figure 14 représente les structures des clones pAEVΔerbB (pXJ4, pXJ5) et la construction de pXJ5,

la figure 15 représente la structure du génome de l'AEV cloné dans pBR313,

la figure 16 représente le clonage du fragment HincII-BamHI (1,2 kb) contenant la jonction entre v-erbA et v-erbB dans les bactériophages mp8 et mp11 sous forme d'ADN bicaténaire, (1,2 kb) dans les bactériophages mp8 et mp11 sous forme d'ADN bicaténaire,

la figure 17 est une comparaison des séquences nucléotidiques entre le gène v-erbB de l'AEV et celui de l'AEV-H (Yamamoto et al., 1983),

— : nucléotides identiques ;

les nucléotides différents sont indiqués dans la séquence de l'AEV le site BamHI dans la région 5' de v-erbB est souligné,

la figure 18 représente la séquence nucléotidique des fragments Bal31 sélectionnés, la séquence non codante intercalée entre le gène erbA et le gène erbB est numérotée ; les limites des séquences des gènes v-erbA et v-erbB et celle du vecteur de clonage mp11 sont indiquées par des lignes verticales ; les lignes horizontales représentent les séquences du gène v-erbB conservées après la digestion par Bal31 ; les sites de restriction dans les séquences de mp11 sont soulignés ; p15-16, p15-21, p15-25, p15-28 : noms des clones, CAG : site accepteur d'épissage présumé ;

la figure 19 représente la structure du clone pXJ1 et la carte des sites de restriction,

la figure 20 représente la carte des sites de restriction et la structure de pXJ2,

la figure 21 représente la carte des sites de restriction et la structure de pXJ3,

la figure 22 représente les séquences de jonctions entre le gène Neo$^r$ et les vecteurs rétroviraux dans les clones pXJ1 et pXJ3,

la figure 23 représente la préparation du clone mJS21 erb⁻,

la figure 24 représente la préparation du clone mp11-gag,

la figure 25 représente la préparation du clone mp10-J,

la figure 26 représente la préparation du clone pBRgag-2, 7 γ,

la figure 27 représente la préparation du clone pBRgag-J,

la figure 28 représente la préparation du clone pBRgag-J-env LTR,

la figure 29 représente la préparation du clone pBRgag-J-env 2LTR,

la figure 30 représente la préparation du clone pXJ11,

la figure 31 schématise la structure de pXJ12.

Ces figures et les séquences de nucléotides correspondantes font explicitement partie de la présente description mais n'ont pas été répétées pour éviter d'alourdir ladite description.

Les sites de restriction sont abréviés de la façon suivante :

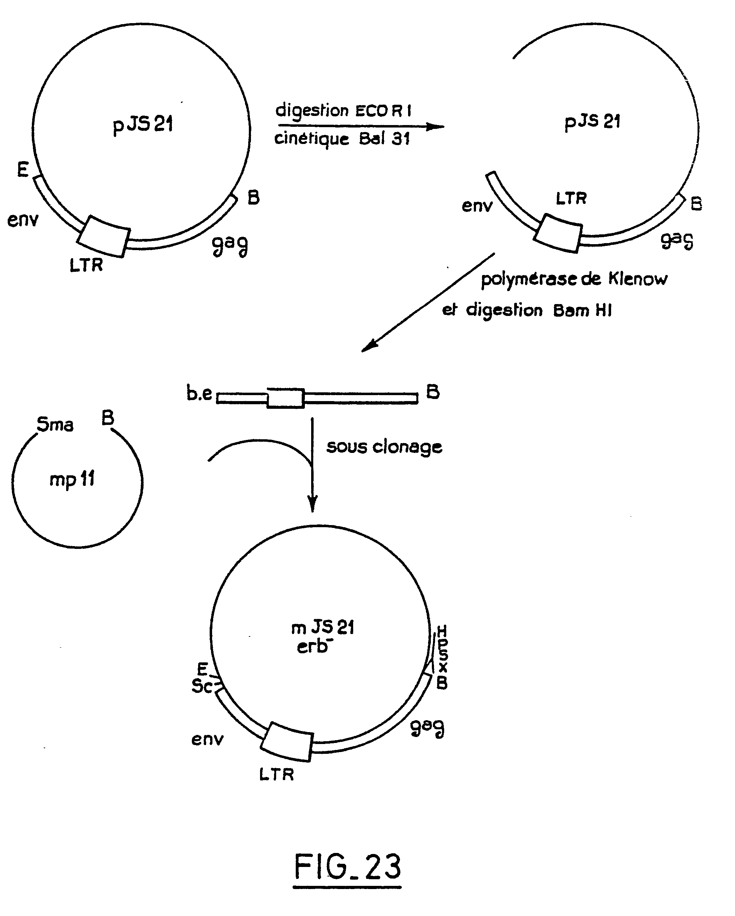

B : BamHI
BII : BglII
E : EcoRI
F : Fkol
P : PstI
C : ScaI
Sm : SmaI
S : SalI
X : Xho  } figures 1 à 22
H : HincII }
X : Xbal } figures 23 à 31
H : HindIII }

## I. Techniques générales

### A) Culture de cellules eucaryotiques

1) Les cellules L (souris) sont maintenues en culture à 37 °C et à 5 % de $CO_2$. Les cellules sont repiquées régulièrement tous les 3 ou 4 jours. Le milieu de culture est composé de DMEM (Gibco) complémenté de 10 % de sérum de veau fœtal, 2 mM de glutamine, 2,2 mg/ml de bicarbonate de sodium, 100 µg/ml de streptomycine, 100 ui/ml de pénicilline.

2) Les fibroblastes de poulet sont obtenus après repiquage de cultures primaires préparées à partir d'embryons de 10 à 11 jours et maintenus en culture dans du milieu HAM F10 (Gibco) complémenté avec 10 % de tryptose phosphate broth (Gibco), 2 µg/ml de polybrène, 5 % de sérum de veau, 1 % de sérum de poule inactivé, 2 mg/ml de bicarbonate de sodium, 100 µg/ml de streptomycine, 100 ui/ml de pénicilline et 60 mg/ml de fungizone.

### B) Préparation des acides nucléiques

#### 1) Préparation des ADN

##### a) Préparation des plasmides

Les souches bactériennes d'E. coli HB101 ou C600RS hébergeant les plasmides utilisés sont cultivées sur milieu L. broth (bactotryptone 10 g/l, extraits de levure 5 g/l, NaCl 5 g/l) complémenté avec 5 ml/l d'une solution de glucose à 20 % et par les antibiotiques appropriés.

Les plasmides sont isolés par la technique du lysat clarifié selon El-Gewely et Helling (1980), et purifiés sur gradient de chlorure de césium. Les ADN plasmidiens purifiés sont resuspendus dans de l'eau bidistillée et conservés à + 4 °C.

b) Préparation d'ADN de fibroblastes de poulet

L'ADN des fibroblastes de poulet est obtenu après homogénéisation des cellules dans du Tris-Hcl 100 mM pH 7,4, EdtaNa$_2$ 10 mM, NaCl 100 mM, SDS 0,5 %. De la pronase est rajoutée à 0,5 mg/ml et le mélange est incubé à 37 °C pendant 8 à 12 heures. L'ADN est extrait au phénol chloroforme et au chloroforme-alcool isoamylique. La solution est ajustée à 200 mM NaCl et précipitée une première fois à l'isopropanol puis 2 fois à l'éthanol. Le culot resuspendu dans du tampon T-E (Tris 10 mM pH 7,4, Edta 1 mM), est traité à la RNase (50 µg/ml) pendant 1 heure à 37 °C, puis à la pronase (100 µg/ml) pendant 1 heure à 37 °C.

La solution d'ADN est extraite une fois au phénol, deux fois au chloroforme-alcool isoamylique et enfin l'ADN est précipité par deux volumes d'éthanol. Le culot d'ADN ainsi purifié est resuspendu dans du tampon T-E et gardé à + 4 °C.

2) Digestion par les enzymes de restriction

Les enzymes de restriction proviennent de Boehringer et sont utilisés sous les conditions d'incubation données par le fabricant. Généralement, 1 à 4 unités d'enzyme sont utilisées pour digérer 1 µg d'ADN pendant 2 heures à 37 °C. La réaction enzymatique est arrêtée par incubation à 65 °C pendant 5 à 10 minutes.

3) Préparation des ARN

La verrerie utilisée pour l'extraction des ARN est préalablement stérilisée par chauffage au four pasteur pendant au moins 2 heures. Les tubes servant à l'extraction sont de plus traités au diéthylpyrocarbonate 1 ‰ (Sigma). Les tampons sont stérilisés à 120 °C pendant 30 à 40 minutes.

a) ARN cellulaire total

L'ARN cellulaire total est extrait des cellules eucaryotiques selon la technique de Auffray et Rougeon (1980) à partir de $20 \cdot 10^6$ à $50 \cdot 10^6$ fibroblastes de poulet infectés par des rétrovirus.

Après centrifugation des cellules à 800 rpm, les culots cellulaires sont congelés dans l'azote liquide 10 à 15 minutes et resuspendus dans un tampon dénaturant (LiCl 3 M, urée 6 M, acétate de sodium 0,01 M pH 5, complexe vanadyl ribonucléique (BRL) 0,01 M). La solution est ajustée à 0,01 % SDS et conservée une nuit à + 4 °C. L'ARN est récupéré après centrifugation à 10 000 rpm pendant 20 minutes à 4 °C. Le culot est lavé par une solution urée 8 M, LiCl 4 M, puis resuspendu dans l'eau bidistillée. La solution d'ARN est extraite une fois au phénol, une fois au phénol-chloroforme, et enfin l'ARN est précipité avec 2 volumes d'éthanol en présence d'acétate de sodium 0,2 M pH 5.

b) Préparation des ARN polyA$^+$

Les ARN polyA$^+$ sont préparés selon la technique décrite par Loncagre et Rutter (1977), par filtration sur colonne.

200 à 500 µg d'ARN total sont déposés sur une colonne contenant 200 à 300 mg d'oligodT cellulose de type 3 (Collaborative Research). La filtration d'ARN est effectuée dans un tampon contenant Tris 10 mM pH 7,4 et KCl 500 mM. L'éluat est recyclé deux fois sur la colonne. L'ARN polyA$^+$ est décroché par le tampon d'élution (Tris 10 mM pH 7,4). Les solutions d'ARN polyA$^+$ sont ajustées à 200 mM acétate de sodium pH 5 et les ARN polyA$^+$ sont précipités par deux volumes d'éthanol. Les culots sont resuspendus dans de l'eau bidistillée et conservés à — 20 °C.

c) Préparation des ARN virions

Les virions sont récupérés dans les surnageants de culture de fibroblastes de poulet infectés par le virus. En pratique, lorsque les cultures infectées par le virus contiennent une majorité de cellules transformées, le milieu de culture est changé par du milieu frais. Les virions sont ensuite collectés 24 heures plus tard. Les surnageants sont alors centrifugés une première fois à 2 000 rpm pendant 10 minutes pour éliminer les débris cellulaires. Les virions sont ensuite sédimentés par ultracentrifugation dans un rotor $50 \cdot 2$ Ti à 16 000 - 18 000 rpm pendant 2 heures à 4 °C. Les culots contenant les virions sont resuspendus dans du Tris 10 mM pH 7,4 contenant Edta 10 mM, NaCl 100 mM et le complexe vanadyl-ribonucléique 10 mM. Du SDS 0,1 % et de la protéinase K (0,5 mg/ml) sont ajoutés et la suspension est incubée pendant 1 heure à 37 °C puis extraite deux fois au phénol, deux fois au chloroforme, et l'ARN est précipité avec 2 volumes d'éthanol.

C) Electrophorèse et transfert des acides nucléiques sur filtre de nitrocellulose

### 1) Electrophorèse et transfert d'ADN

15 à 20 µg d'ADN total préalablement digéré avec des enzymes de restriction sont déposés sur gel horizontal à 1 % d'agarose. L'électrophorèse est effectuée dans du tampon E-T (Tris 40 mM pH 7,5, acétate de sodium 20 mM, Edta 2 mM) contenant 0,5 µg de bromure d'éthydium, pendant 12 à 16 heures. Le gel est dénaturé par immersion dans une solution de NaOH 0,5 N et NaCl 1,5 M pendant 2 à 3 heures, puis neutralisé par du Tris-HCl 500 mM pH 5,6, NaCl 300 mM pendant 3 heures, à température ambiante. Le transfert de l'ADN est effectué en appliquant une membrane de nitrocellulose (Sartorius ou Schleicher et Schüll) sur le gel d'agarose pendant une nuit selon la technique de Southern (1975).

### 2) Electrophorèse et transfert d'ARN

15 à 20 µg d'ARN total ou d'ARN polyA+ ou 2 à 5 µg d'ARN polyA+ sont dénaturés à la formaldéhyde selon Lehrach et al. (1977). Les ARN sont équilibrés par du tampon MOPS (acide morpholinopropane sulfonique 20 mM pH 7, acétate de sodium 5 mM, Edta 1 mM) et dénaturés en présence de 6 % de formaldéhyde et de 50 % de formanide à 65 °C, pendant 5 à 10 minutes, puis refroidis rapidement dans la glace. Le fractionnement des ARN dénaturés est effectué sur gel horizontal de 1 % à 1,5 % d'agarose, contenant 6 % de formaldéhyde, dans du tampon MOPS. La migration s'effectue pendant 16 à 20 heures à 30-40 volts, à température ambiante avec recyclage du tampon. Le transfert des ARN du gel sur filtre de nitrocellulose est réalisé selon la technique de Southern (1975) modifiée par Thomas (1980). Le filtre de nitrocellulose rincé avec l'eau bidistillée est équilibré pendant 5 à 10 minutes dans du SSC × 20 avant d'être appliqué sur le gel. Le transfert dure une nuite à + 4 °C. Le filtre est ensuite récupéré, séché sous une lampe pendant 5-10 minutes.

### D) Préparation de sondes radioactives

0,4 µg à 0,5 µg de divers fragments d'ADN utilisés comme sondes sont marqués « in vitro » par translation de coupure (Rigby et al. 1977). La réaction se déroule dans un volume de 100 µl final contenant du Tris-HCl 50 mM pH 7,8, MgCl$_2$ 5 mM, BSA 500 µg/ml, bétamercaptoéthanol 10 mM, dGTP 3,5 µM, dATP 3,5 µM, 40 µCi de dCTPalpha P$^{32}$ et 40 µCi de TTPalpha P$^{32}$ (activité spécifique 400 Ci/m mole - Amersham), 2 pg de DNaseI (Wortington) et 4 à 5 unités d'ADN polymérase (Boehringer). Le mélange est incubé pendant 2 heures à 15 °C. La réaction est arrêtée par addition de 50 µl d'Edta 250 mM, et l'ADN marqué est séparé des nucléotides non incorporés sur colonne de Séphadex G · 50 ou de biogel (Bio-Rad A. 1,5 m).

### E) Hybridation des filtres

Après transfert soit d'ADN soit d'ARN, les filtres sont séchés à 70-80 °C pendant 2 heures, traités par un tampon de préhybridation (Tris-HCl 50 mM pH 7, SSC × 3, ARN$_t$ 20 µg/ml, ADN de sperme de saumon 20 µg/ml, Denhardts 1x, formamide 50 % pendant 8 à 20 heures à 42 °C. L'hybridation est réalisée dans le même tampon contenant 5-10 · 10$^6$ cpm de la sonde radioactive pendant 24 à 48 heures à 42 °C.

En fin d'hybridation, les filtres sont lavés une fois dans du SSC 2x pendant une heure à 42 °C puis deux fois dans du SSC 0,1x, SDS 0,1 % pendant 45 minutes à 50 °C, et enfin rincés 4 fois dans du SSC 0,1x pendant 5 minutes. Après séchage, les filtres sont exposés pour autoradiographie sur film (Kodak royal X Omat AR) à — 80 °C avec écrans renforçateurs. L'exposition dure de 5 heures à plusieurs jours.

### F) Déshybridation des filtres

Les filtres hybridés une première fois avec une sonde marquée au $^{32}$P sont déshybridés dans du SSC 70-75 °C, pendant 10 à 15 minutes. Ils sont séchés et exposés pendant 48 heures en autoradiographie. Les filtres sont traités par le tampon de préhybridation et utilisés avec une nouvelle sonde.

### II. Techniques utilisées pour la recombinaison génétique « in vitro »

A) Isolement de fragments clones dans des plasmides

### 1) Isolement après électrophorèse sur gel d'agarose

50 µg à 100 µg d'ADN de plasmide contenant le fragment à isoler sont digérés par les enzymes de restriction permettant de relacher le fragment. Les fragments d'ADN sont ensuite séparés par électrophorèse sur gel horizontal d'agarose pendant une nuit à 30-40 volts. La région du gel contenant le fragment à isoler est ensuite découpée. L'ADN est extrait de l'agarose par l'une ou l'autre des deux techniques suivantes :

a) Technique d'électroélution

La région découpée du gel, et contenant le fragment à isoler, est placée dans un sac à dialyse renfermant du tampon E-T (Tris 40 mM pH 7,5, acétate de sodium 20 mM, Edta 2 mM). Le sac est soumis à un champ électrique (30-40 volts) dans une cuve contenant du tampon E-T. L'élution de l'ADN est suivie par illumination brève à la lumière ultraviolette. En fin d'élution, l'ADN est extrait 3 à 4 fois à l'alcool isoamylique puis précipité par deux volumes d'éthanol.

b) Technique dite de « freeze squeeze » (Tautz et Renz, 1983)

Le morceau du gel contenant le fragment à isoler est équilibré dans 10 fois son volume d'une solution contenant de l'acétate de sodium 300 mM pH 7, et de l'EDTA 1 mM, avec agitation pendant 30 à 45 minutes à l'abri de la lumière. Le morceau de gel est placé dans un tube Eppendorf de 0,6 ml, percé à son fond et bouché avec de la laine de verre, et l'ensemble est congelé dans l'azote liquide. Le tube renfermant le morceau de gel est placé dans un tube Eppendorf de 1,5 ml puis centrifugé à 12 000 rpm pendant 10 minutes à température ambiante. La solution récupérée contenant le fragment à isoler est ajustée avec une solution contenant du MgCl$_2$ 1 mM et de l'acide acétique 10 %, puis précipitée avec 2,5 volumes d'éthanol. Le culot est rincé successivement avec un mélange contenant de l'éthanol 70 %. Le culot est séché brièvement, puis resuspendu dans du tampon T-E.

2) Gradient de sucrose

50 μg à 100 μg d'ADN de plasmide préalablement digéré par des enzymes de restriction sont déposés sur un gradient de sucrose 5-20 %. Le gradient est centrifugé pendant 14 à 16 heures, entre 30 000 et 36 000 rpm, dans un rotor SW41 à 20 °C. Les bandes repérées sous lumière ultra-violette sont récupérées à l'aide d'une pipette.

Les ADN sont dialysés dans du tampon T-E pendant une nuit puis purifiés au phénol et au chloroforme et enfin précipités à l'éthanol.

B) Déphosphorylation des extrémités 3' de l'ADN

L'ADN est incubé en présence de phosphatase alcaline bactérienne ou BAP (BRL) pendant une heure à 65 °C. Généralement, on utilise 10 unités de BAP par mole d'extrémité 3' d'ADN à déphosphoryler. L'enzyme est ensuite éliminé par deux extractions successives au phénolchloroforme et l'ADN est récupéré par précipitation à l'éthanol.

C) Ligation

La ligation de fragments d'ADN est effectuée dans un tampon composé de Tris 600 mM pH 7,6, MgCl$_2$ 66 mM, DTT 100 mM, ATP 4 mM, en présence de ligase du phage T4 (Boehringer) à la concentration de 2 μ/μg d'ADN. Les ADN à liguer sont ajoutés en quantité équimolaire à la concentration finale de 5 à 10 μg/ml. Le mélange final qui n'excède pas 60 μl est incubé une nuit à 65 °C.

D) Addition de « linkers »

Les « linkers » PstI ont été utilisés pour la construction des recombinants pAEVdeltaΔHpa. Dans ce cas particulier, les « linkers » sont ajoutés au site HpaI du gène humain de globine delta.

0,5 μg de « linkers » PstI (BRL) sont phosphorylés dans un mélange contenant du Tris HCl 60 mM pH 7,8, du MgCl$_2$ 10 mM, du béta-mercaptoéthanol 15 mM, 4 unités de polynucléotide kinase (Boehringer), de l'ATP 50 μM et 10 μCi d'ATP gamma $^{32}$P. La réaction est incubée à 37 °C pendant 30 minutes. Le mélange de « linkers » PstI phosphorylés est ajouté à 5 μg de plasmide pBRdeltaPst ouvert auparavant au site HpaI et déphosphorylé. Le mélange est soumis à l'action de la ligase T4 pendant une nuit à 15 °C.

E) Identification de bactéries

Les ADN de plasmides recombinants sont introduits dans des bactéries C-600 RS rendues compétentes selon la technique de Maniatis et al. (1982). Les bactéries transformées sont ensuite étalées sur boîte d'agar contenant les antibiotiques appropriés.

F) Identification des plasmides dans les clones bactériens

1) Transfert de colonies bactériennes sur filtre

Cette technique est appliquée dans le cas où les plasmides recombinants recherchés représentent une faible proportion parmi les différents plasmides recombinants possibles. Elle permet un criblage rapide sur un grand nombre de colonies bactériennes. Un papier filtre (Whatman) stérilisé par chauffage à 150 °C pendant 2 heures est appliqué 10 à 15 secondes sur la boîte d'agar contenant des colonies bactériennes bien individualisées. Après transfert, les filtres sont séchés à l'air pendant 5 à 10 minutes, puis traités dans NaOH 0,5 M pendant 5 minutes et placés dans une étuve à 37 °C pendant 1 heure. Les filtres sont ensuite traités deux fois 5 minutes dans chacun des trois bains suivants, avec agitation : NaOH 0,5 M — Tris 0,5 M pH 8 — SSC2x, et enfin rincés dans l'éthanol et séchés à l'air.

Les colonies renfermant les recombinants recherchés sont identifiées par hybridation du filtre avec une sonde d'ADN marquée au dCTPalpha $^{32}$P par translation de coupure.

Les filtres sont préhybridés dans un mélange de formamide 50 % et SSC5x pendant 2 heures à 37-42 °C. L'hybridation s'effectue dans le même mélange en présence de sondes spécifiques appropriées pendant 24 à 48 heures à 42 °C. Les filtres sont lavés respectivement 30 minutes dans du formamide 50 % et SSC5x à 37-42 °C et 4 fois 30 minutes dans du SSC2x à température ambiante. Les filtres sont enfin rincés dans l'éthanol puis séchés à l'air et exposés pour autoradiographie pendant 2 à 4 heures. Les colonies contenant le fragment d'ADN recherché sont identifiées après révélation de l'autoradiographie, puis isolées et analysées par préparation rapide de plasmides.

## 2) Préparation rapide des ADN de plasmides

Les plasmides sont préparés à partir de 1,5 ml de culture selon la méthode de lyse alcaline de Birnboim et Doly (1979) modifée par Maniatis et al. (1982).

Les ADN plasmidiens sont analysés par digestion au moyen d'enzymes de restriction et fractionnement sur gels d'agarose.

## III. Production de particules virales par transfection de fibroblastes de poulet

La technique de transfection utilisée est la technique au phosphate de calcium décrite par Graham et Van der eb (1973), modifiée par Wigler et al. (1977).

### A) Préparation de l'ADN

La quantité d'ADN transfectée par boîte de culture de diamètre 60 mm est de 10 à 15 μg. Cet ADN se compose d'ADN de sperme de saumon (3 à 4 μg) et d'ADN viral. L'ADN viral est lui-même constitué du plasmide contenant le génome AZV' modifié et du plasmide contenant l'ADN du virus assistant RAV-2 dans un rapport molaire de 5/1 à 10/1.

Le mélange d'ADN est suspendu dans un volume de tampon A (HEPES 25 mM, CaCl$_2$ 250 mM, pH 7,15). La précipitation phosphocalcique est réalisée par addition d'un volume de tampon B (HEPES 25 mM, NaCl 280 mM, Na$_2$HPO$_4$ 1,5 mM, ph 7,15) avec agitation rapide (10 à 15 secondes). Un très fin précipité est observé dans le mélange au bout de 20 à 30 minutes à température ambiante.

### B) Préparation des cellules et transfection

$6 \cdot 10^5$ fibroblastes secondaires d'embryons de poulet sont ensemencés dans des boîtes de Pétri de 60 mm de diamètre, 16 heures avant la transfection.

Pour la transfection, le mélange d'ADN précipité est déposé dans chaque boîte de culture et les cellules sont incubées pendant 5 à 6 heures à 37 °C. Le milieu de transfection est remplacé ensuite par du milieu frais et les cellules sont repiquées dans une boîte de Pétri de 100 mm de diamètre. Tous les 3 à 4 jours, chaque boîte de culture est ensuite répartie dans trois boîtes de 100 mm de diamètre.

## Exemple 1

### Clone de l'ADN du virus assistant

Le génome du virus assistant RAV-2 a été obtenu de Boone et Skalka (1981). Il est constitué d'une séquence de 8 kb insérée au site SalI de l'ADN du phage lambda. Cette séquence a été sous-clonée dans le plasmide pBR322 pour fournir le clone pRAV2. Le génome RAV2 présente un seul site SalI situé dans le gène env. De ce fait, dans le plasmide, les séquences du génome sont en partie permutées (figure 3).

Afin de produire des virions RAV-2, l'ADN pRAV2 doit être transfecté sur fibroblastes de poulet. L'ADN viral doit être introduit dans les cellules réceptrices sous la forme d'un génome encadré de LTR et dans lequel l'arrangement des gènes correspond à celui existant dans l'ARN viral. Cet arrangement peut être réalisé « in vitro » après digestion du plasmide pRAV2 par SalI et religation entre eux des fragments obtenus afin de créer des concatémères (figure 4).

La concatémérisation par ligation permet de générer un certain nombre de molécules qui pourront être intégrées dans le génome des cellules hôtes et transcrire des ARN génomiques pouvant générer des

12

virions infectieux. On notera qu'une proportion seulement des concatémères est susceptible de donner naissance à des virions.

Ainsi, si l'on ne considère que l'arrangement en dimères, indispensable pour assurer le fonctionnement du système, on calcule que seulement 1/3 des molécules présenteront la structure d'un génome proviral reconstitué.

Exemple 2

Clone de l'ADN de l'AEV sauvage

a) Clone pAEV11

Le clone de départ est constitué par le plasmide pAEV11 isolé par Vennstrom et al. (1980) et qui est représenté sur la figure 2, a.

Ce clone est constitué du plasmide pBR313 dans lequel le génome d'AEV d'environ 5,4 kb a été inséré au site unique EcoRI. Dans ce clone, le génome présente une permutation due à la présence du site EcoRI situé à 37 pb en amont du codon de terminaison de v-erbB. Il faut souligner que ce clone renferme une structure LTR double.

Comme dans le cas de pRAV2, la production de virions à partir de pAEV11 implique la transfection, sur fibroblastes de poulet, de concatémères reconstituant un génome intégral. Dans ce cas aussi la proportion de concatémères présentant l'arrangement favorable est faible. Afin d'augmenter l'efficacité de production virale par transfection, on recrée par recombinaison génétique « in vitro » un clone dans lequel le génome viral est reconstitué sous une forme identique au provirus.

b) Construction du clone pAEV2LTR

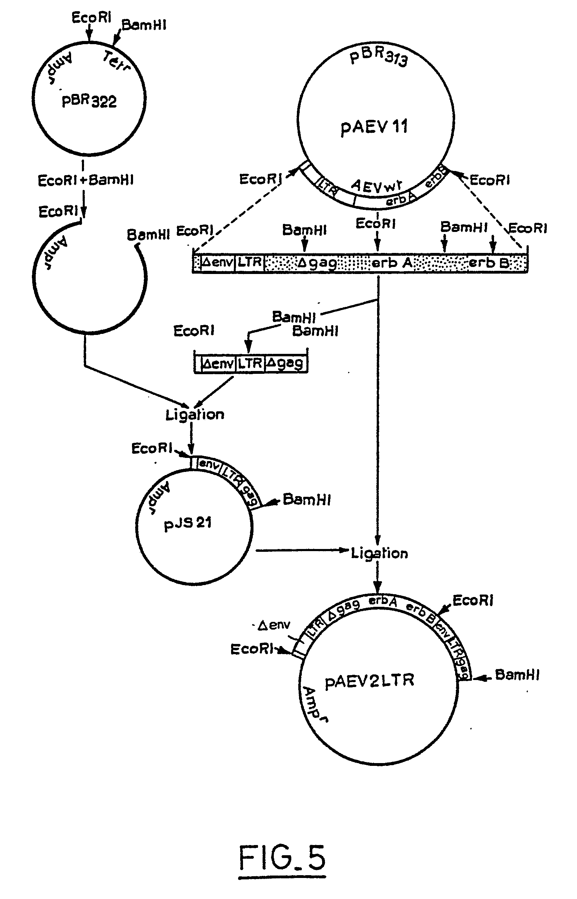

Le réarrangement effectué consiste à isoler le fragment viral EcoRI-BamHI de 1,8 kb (figure 5) couvrant la fin du v-erbB, le résidu 3' terminal du gène env (Δenv), les séquences LTR et le début du gène gag. Ce fragment a été inséré et cloné dans pBR322, délété de son fragment mineur EcoRI-BamHI. Le plasmide obtenu est appelé pJS21.

Le génome entier de l'AEV est isolé de pBR313 après digestion du plasmide pAEV11 par EcoRI. Le fragment portant le génome AEV est ligué au plasmide pJS21 préalablement ouvert au site unique EcoRI. L'un des recombinants obtenus, appelé pAEV2LTR, présente la structure d'un provirus avec une copie LTR à chaque extrémité.

L'ADN pAEV2LTR peut être transfecté sur fibroblastes de poulet sans digestion préalable. Après cotransfection avec l'ADN portant les séquences du virus assistant RAV2, les fibroblastes produisent des virions de type AEV et subissent une transformation oncogène décelable environ 15 jours après la transfection. On note que la transfection avec pAEV2LTR augmente très nettement l'efficacité de la production virale par rapport à la transfection avec pAEV11 concatémérisé.

Ainsi, pour induire la transformation oncogène d'une culture de fibroblaste, environ 25 µg de pAEV11 concatémérisé sont nécessaires, alors que l'on obtient le même résultat avec 10 µg de pAEV2LTR. De plus, la transformation oncogène des fibroblastes transfectés avec pAEV2LTR apparaît beaucoup plus vite qu'avec AEV11 concatémérisé.

Exemple 3

Insertion du gène humain de globine delta dans le génome de l'AEV

a) Nature du fragment inséré

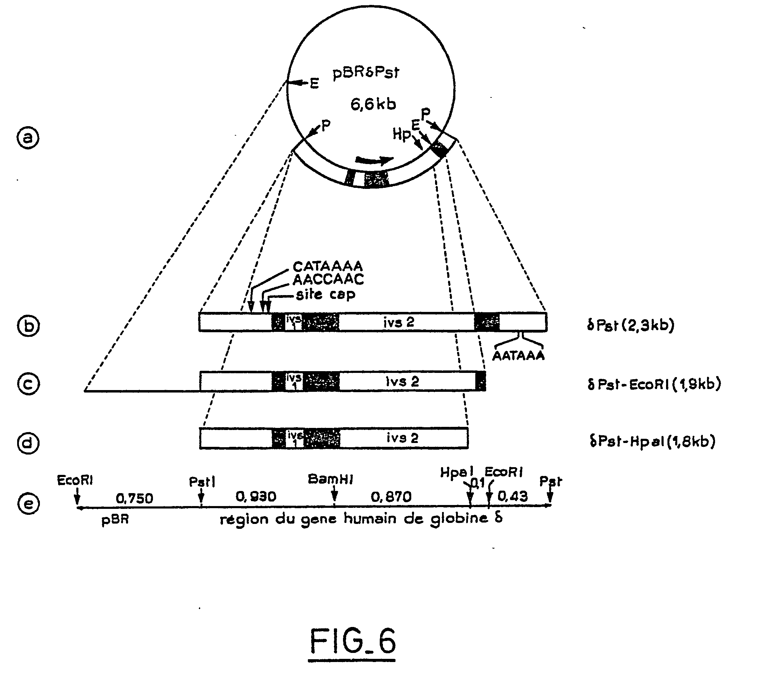

Le fragment de globine delta est obtenu après digestion du plasmide pBrdeltaPst (figure 6) par EcoRI et isolement sur gradient de sucrose. Ce fragment d'ADN commence en amont par une séquence dérivée du plasmide pBR322 (EcoRI-PstI d'environ 0,750 kb). Le fragment pBR est maintenu essentiellement dans le but de conserver un site EcoRI à l'extrémité 5' du fragment delta.

La structure spécifique de la région delta s'étend du site PstI jusqu'au site EcoRI. Dans cette séquence le gène delta proprement dit débute à 460 pb en aval du site PstI. Ce site appelé « cap » est le point d'initiation de la transcription des ARN messagers delta. 30 pb en amont du site « cap » se trouve la séquence CATAAAAG, qui est l'homologue de la séquence « TATA ». Tandis que l'homologue de la deuxième séquence consensus « CAT » rencontrée dans la majorité des gènes d'eucaryotes entre 70 et 80 pb en amont du site cap semble modifiée dans le cas du gène delta (AACCAAC). Les deux séquences consensus TATA et CAT sont impliquées dans la régulation de la transcription des gènes eucaryotiques transcrits par l'ARN polymérase II. La région transcrite comprend respectivement en allant du site cap de 5' vers 3' :

— une séquence « leader » couvrant 50 pb,
— le premier exon (93 pb),
— le premier intron (128 pb),
— le deuxième exon (221 pb),
— le deuxième intron (889 pb),
— les cinquante premières paires de bases du troisième exon.

De ce fait, l'extrémité 3' du gène est écourtée à partir du site EcoRI. La conséquence de cette délétion est l'élimination de 75 pb du 3ème exon et de toute la région 3' non codante portant entre autre le signal de polydénylation (AAUAAA) situé à 18 pb avant le site de terminaison de la tanscription.

b) Clonage du fragment pBRdeltaEcoRI dans pAEV2LTR

L'ensemble des séquences pBR et delta donne lieu à un fragment EcoRI-EcoRI de 2,6 kb qui est inséré dans l'un des sites EcoRI de pAEV2LTR. L'identification des clones pAEV2LTR qui ont subi cette insertion a été réalisée par la technique de transfert des colonies bactériennes sur filtre. Seules les colonies qui présentent une hybridation positive à la fois avec la sonde d'ADN de globine delta et la sonde d'ADN gag- erbA sont retenues. Sur 800 colonies analysées, 24 répondaient à ces critères. Le site d'insertion et l'orientation du fragment delta sont ensuite déterminés par cartographie de restriction des plasmides contenus dans ces bactéries positives.

Parmi les nombreuses combinaisons possibles, seuls les plasmides recombinants ayant inséré le gène de globine au site EcoRI situé à l'extrémité du gène v-erbB, dans les deux orientations possibles, ont été retenus (figure 7). Ces recombinants sont appelés pAEV2LTRdelta (28) dans le cas où le sens de transcription du gène est orienté dans la même direction que celle du virus et pAEV2LTRdelta (17) dans le cas de l'orientation inverse.

Sur les 24 clones ayant incorporé le fragment, deux étaient de type pAEV2LTRdelta (28) et 4 étaient de type pAEV2LTRdelta (17). Les clones pAEV2LTRdelta présentent donc 3 sites EcoRI que nous identifierons de gauche à droite respectivement sous les noms : EcoRI (1), EcoRI (2), EcoRI (3).

c) Structure du plasmide pAEV2LTRdelta (28)

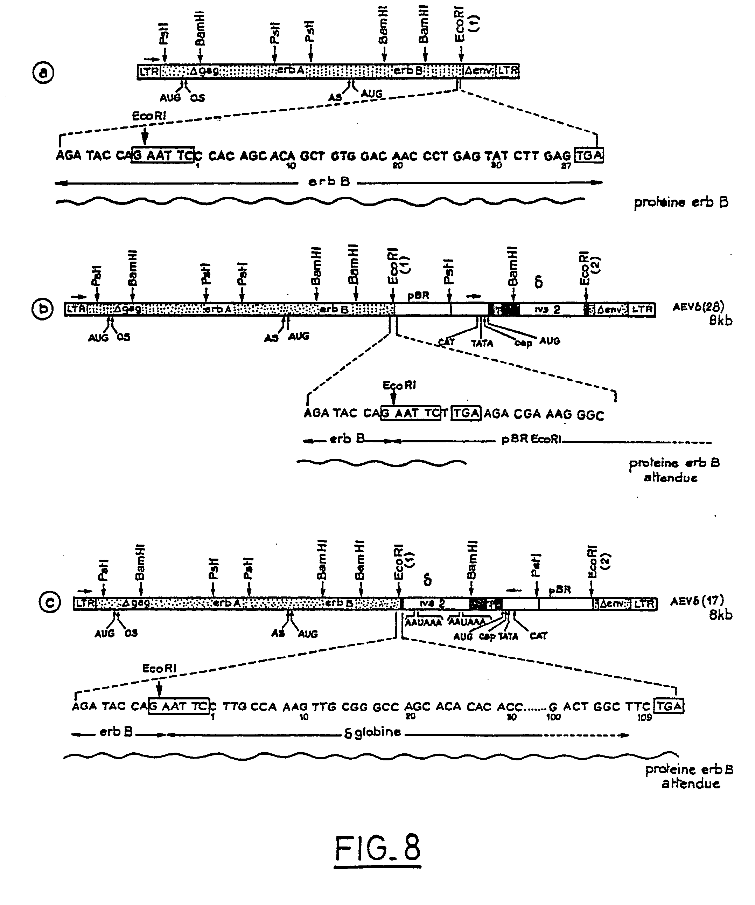

L'insertion du fragment portant le gène delta au site EcoRI unique de l'AEV interrompt la structure normale du gène v-erbB (figure 8,a). Le résultat de cette insertion se traduit par la création d'un nouveau codon de terminaison (UGA) pour la protéine ErbB, situé à 1 pb en aval du site EcoRI (2). En conséquence, la protéine erbB du mutant AEVdelta (28) doit présenter 12 acides aminés de moins dans la région C terminale.

Les signaux d'arrêt de transcription et de polyadénylation étant supprimés dans le fragment PstI-EcoRI ingéré, ce sont les structures virales localisées dans la séquence LTR de droit qui devront vraisemblablement remplir ces fonctions.

d) Structure du plasmide pAEV2LTRdelta (17)

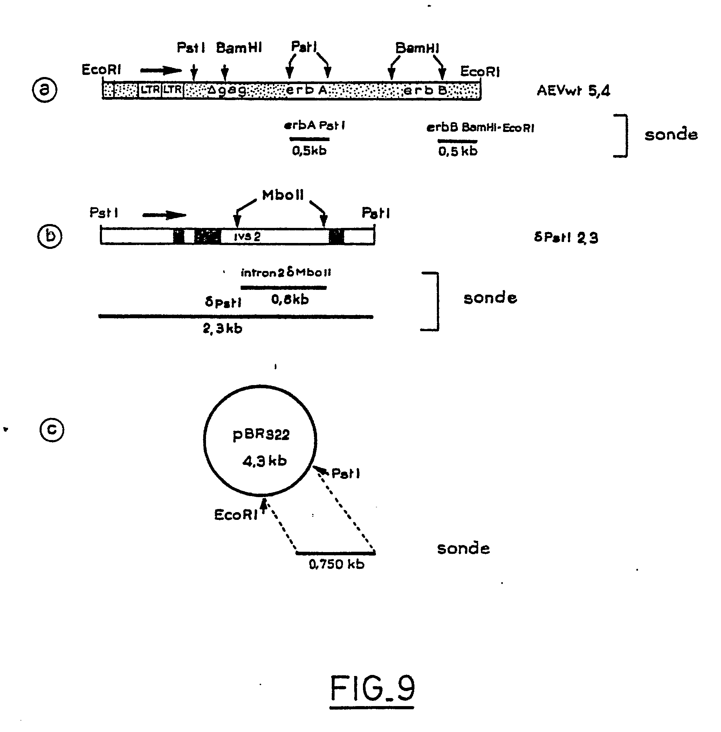

L'insertion du fragment delta en sens inverse de celui de la transcription du virus engendre, là encore, un décalage de la phase de lecture du gène v-erbB. Un nouveau codon de terminaison est créé à 75 pb en aval du site EcoRI (figure 8, b). Il en résulte que la protéine erbB de ce mutant doit théoriquement renfermer dans la région C terminale 75 acides aminés de plus que la protéine erbB du virus sauvage. Dans ce génome viral recombinant, le brin viral qui transcrit l'ARN génomique (brin +) porte le brin anti-codant du gène delta. Dans cette disposition, le brin anti-codant du gène delta fait apparaître deux séquences qui rappellent des signaux de polyadénylation (AAUAAA) à 93 pb et 800 pb en aval du site EcoRI (2) (figure 9b). Dans cette construction, le promoteur propre du gène situé en amont du site cap n'est plus porté sur le brin viral transcrit.

Exemple 4

Caractérisation des différents vecteurs

Tous les plasmides recombinants obtenus par recombinaison « in vitro » sont caractérisés par cartographie de restriction et hybridation avec une sonde spécifique du gène de globine delta. Les ADN plamidiens des différents clones retenus, pAEV2LTR, pAEV2LTRdelta (28), pAEV2LTRdelta (17), sont préparés par la technique de lyse alcaline (Maniatis et al. 1982), digérés respectivement par les 4 enzymes de restriction suivants : EcoRI, BamHI, PstI et SalI. Les fragments de digestion sont séparés sur un gel d'agarose et visualisés en lumière U.V. L'ADN est ensuite dénaturé dans le gel puis transféré sur un filtre. Le filtre est hybridé avec une sonde deltaPst marquée au $^{32}$P par translation de coupure. Les bandes correspondant aux fragments portant le gène sont révélées par exposition en autoradiographie. Pour chaque recombinant les tailles des fragments résultant des 4 digestion (EcoRI, BAmHI, PstI et SalI) sont

déduites des tailles des fragments du marqueur de poids moléculaire utilisé (ADN du phage lambda digéré par HindIII).

A l'issue de ces analyses, on constate que :

1. les tailles des fragments résultant des diverses digestions concordent pour chaque recombinant avec les tailles attendues ;

2. l'hybridation par la sonde deltaPst ne révèle que les fragments porteurs de la séquence delta, présentant là aussi des tailles attendues.

De ces deux constatations, on peut conclure que les clones recombinants retenus sont conformes aux constructions recherchées.

## Exemple 5

### Transfert du gène de globine delta par les particules
### virales AEVdelta (28) et AEVdelta (17)

A) Production de virus

1) Transfection des ADN proviraux dans les fibroblastes de poulet

Pour déterminer si les vecteurs recombinants construit produisent des virus transmissibles, le plasmide recombinant pAEV2LTR et ses dérivés pAEV2LTRdelta (28) et pAEV2LTRdelta (17) ont été transfectés séparément en présence d'ADN du virus assistant (pRAV2) dans des cultures secondaires de fibroblastes embryonnaires de poulet.

Les cellules ont été maintenues en milieu liquide et repiquées régulièrement. Le changement morphologique caractéristique de la transformation des fibroblastes par l'AEV est apparu à partir du 4ème passage (2ème semaine) pour les trois virus. Après ensemencement dans de la gélose molle, ces cellules produisent des colonies de fibroblastes transformés. Les surnageants des cultures transformées ont été récupérés afin de constituer des stocks de virus et utilisés pour infecter des fibroblastes secondaires frais dont ils induisent la transformation oncogène visible au bout d'une semaine environ. Ceci démontre que des particules virales ont été produites par les cellules transfectées par les ADN de virus recombinants, ces particules sont infectieuses et ont conservé leur pouvoir oncogène. On appellera AEVdelta (17) et AEVdelta (28) les virus produits respectivement à partir des plasmides pAEV2LTRdelta (17) et pAEV2LTRdelta (28).

2) Titrage des virus récupérés

Les stocks de virus AEVdelta (17) et AEVdelta (28) ont été titrés pour leur pouvoir transformant selon la technique de dénombrement de foyers de transformation. Pour cela, 1 ml de suspension virale diluée a été inoculé sur des cultures de fibroblastes frais. Les cultures sont ensuite recouvertes d'agar afin de permettre le développement de foyers de transformation. Le titre du virus est donné par le nombre de foyers de transformation par boîte multiplié par la dilution de la suspension de virus inoculé. Le titre est exprimé en F.F.U. (Focus Forming Unit) par ml.

Le virus AEVdelta (28) récupéré à partir des cellules transfectées présentait un titre de $5 \cdot 10^4$ FFU/ml), supérieur à celui du virus AEVdelta (17) ($2 \cdot 10^4$ FFU/ml).

Le virus sauvage AEVwt, produit dans des conditions similaires, présente généralement un titre voisin de $5 \cdot 10^5$ FFU/ml.

Afin de vérifier la stabilité des virus recombinants, les virus sont maintenus sur des cultures de fibroblastes de poulet pendant des durées variables. Pour cela, les stocks originaux, dérivés des cultures transfectées, ont été inoculés à des cultures de fibroblastes frais qui ont subi ensuite deux repiquages à l'issue desquels les surnageants de culture ont été collectés pour constituer les stocks viraux II. Les virus des stocks II ont ensuite été propagés à nouveau sur fibroblastes frais pendant deux repiquages, à l'issue desquels on a collecté le stock viral III.

Les titres des virus dans les stocks II et III ont été déterminés et sont présentés dans le tableau I.

(Voir Tableau I page 16)

Tableau I

| STOCK VIRAL | NOMBRE DE PARTICULES VIRALES TRANSFORMANTES PAR ml | |
| --- | --- | --- |
| | AEVdelta(28) | AEVdelta(17) |
| Original I | $5.10^4$ | $2.10^4$ |
| II | $2.10^4$ | $1.10^4$ |
| III | $2.10^4$ | $0,7.10^4$ |

On constate d'une manière générale une réduction du titre du virus au cours des passages successifs. Bien que l'AEVwt n'ait pas été testé en parallèle l'expérience de l'utilisation de ce virus montre que son titre reste sensiblement stable après plusieurs passages sur culture cellulaire.

B) Analyse des ARN viraux transcrits dans les cellules infectées et encapsidés dans les virions

Des fibroblastes embryonnaires de poulet ont été infectés par les virus AEVwt, AEVdelta (17) et AEVdelta (28). Lorsque les cultures présentaient une majorité de cellules transformées, les surnageants des cultures et les cellules ont été récupérés séparément.

A partir des surnageants, on collecte des virions par centrifugation. Les ARN ont été extraits respectivement des virions et des cellules. Les ARN cellulaires ont été séparés sur oligodT cellulose en ARN polyA$^+$ et polyA$^-$. Les ARN polyA$^+$ cellulaires et les ARN totaux des virions ont été fractionnés en électrophorèse sur agarose et transférés sur filtre de nitrocellulose. Les filtres ont été hybridés successivement avec différentes sondes représentées dans la figure 9. Après exposition autoradiographie et révélation, les tailles des bandes observées ont été résumées dans le tableau II.

Tableau II

| SONDES / VIRUS | erbA | erbB | deltaPst | intron2delta | pBR |
| --- | --- | --- | --- | --- | --- |
| AEVwt | $5,3^*$ | $5,3^*$ | - | - | - |
| | - | $3,2$ | - | - | - |
| AEVdelta(17) (insertion inverse) | 8 | 8 | 8 | 8 | 8 |
| | $6,3^*$ | $6,3^*$ | $6,3^*$ | $6,3^*$ | $6,3^*$ |
| | - | $5,9$ | $5,9$ | $5,9$ | - |
| | - | $4$ | $4$ | $4$ | $4$ |
| | - | $3,7$ | $3,7$ | $3,7$ | - |
| AEVdelta(28) (insertion directe) | $7^*$ | $7^*$ | $7^*$ | - | $7^*$ |
| | $5,2-4,8^*$ | $5,2-4,8^*$ | $5-4,8$ | - | $5-4,8$ |
| | - | $3$ | - | - | - |
| | - | $2,8$ | - | - | - |

Le tableau donne les tailles des bandes identifiées par les différentes sondes, dans les fibroblastes infectés par AEVwt, AEVdelta (17) et AEVdelta (28). Les valeurs signalées par un astérisque (*)

correspondent à des transcrits présents à la fois dans les ARN polyA⁺ cellulaires et dans les ARN des virions.

Ce tableau démontre l'obtention de virions porteurs de l'ARN du gène humain globine delta, de même que d'une partie de l'ARN correspondant au vecteur plasmidique pBR.

C) Analyse des provirus intégrés dans les fibroblastes infectés par les virus recombinants

### 1) But de la recherche de l'ADN proviral

L'ADN proviral représente, à l'exception des LTR, une copie parfaite de l'ARN génomique à partir duquel il est synthétisé. L'analyse du provirus doit donc fournir des informations sur la structure du génome viral transféré dans ces cellules. Cette analyse appliquée aux mutants AEVdelta (17) et AEVdelta (28) doit permettre de vérifier si ces virus sont capables de transférer, dans l'ADN des cellules infectées, les gènes qui ont été greffés dans leur génome. Elle permet aussi de savoir si ces gènes sont transmis intégralement ou si des modifications structurales sont survenues au cours des différents cycles de développement des virus.

### 2) Identification des séquences d'ADN proviral

L'existence dans l'ADN de fibroblastes de poulet de séquences c-erb apparentées aux séquences oncogènes virales est révélée après hybridation avec les sondes d'origine virale. Ces séquences c-erb se distinguent des séquences v-erb par leur cartographie de restriction.

### 3) Analyse des provirus

Des cultures de fibroblastes embryonnaires de poulet ont été infectées séparément par AEVwt, AEVdelta (17) et AEVdelta (28). Après apparition du phénotype transformé, les cellules sont trypsinées et lysées pour en extraire l'ADN total. L'ADN est purifié et digéré par les enzymes de restriction EcoRI ou BamHI. Les produits de digestion ont été séparés sur gel d'agarose, dénaturés puis transférés sur filtres. Les filtres ont été hybridés successivement par les sondes erbB et deltaPst. Les bandes ont été révélées après exposition en autoradiographie de 5 à 10 jours. Les bandes relatives aux génomes proviraux AEVdelta (17) et AEVdelta (28) sont identifiées par comparaison avec les diagrammes d'ADN provenant de cellules infectées par l'AEVwt.

D) Interprétation des résultats : structure des provirus et transcription des ARN viraux

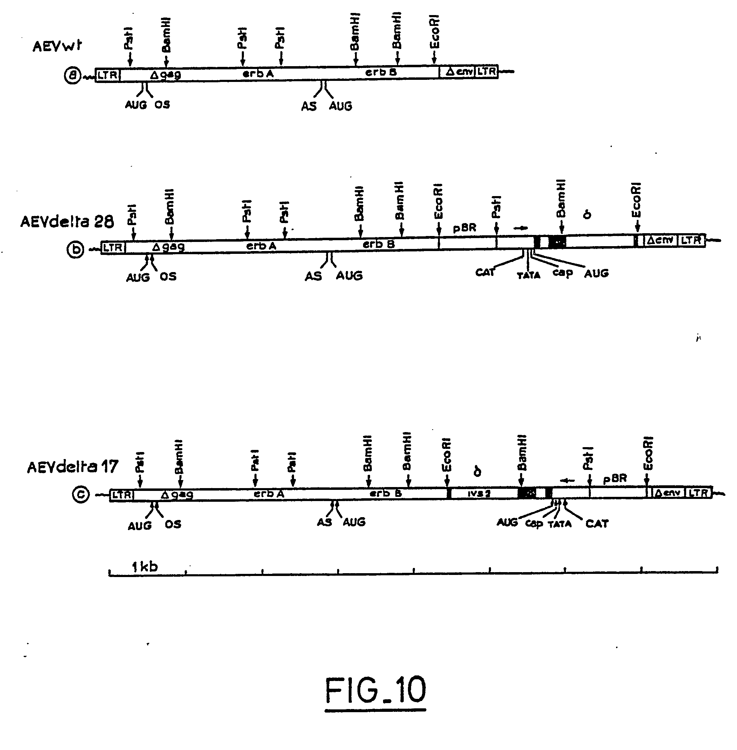

Les tailles des bandes caractéristiques de chaque provirus ont été résumées dans le tableau IV. L'interprétation de ces bandes peut être réalisée en considérant la cartographie de restriction des provirus portée sur la figure 10. Sur le tableau III on a porté la taille théorique des bandes attendues à partir des génomes viraux qui ont été construits. Pour le virus AEVdelta (28), on a considéré que l'intron 2 du gène delta a été éliminé.

### Tableau III

Tailles (kb) théoriques des fragments obtenus après digestion
des provirus AEVdelta (17) et AEVdelta (28) soit par BamHI, soit par
EcoRI et reconnus par les sondes virales erbB ou de globine deltaPst

(Ces tailles ont été calculées à partir des plasmides pAEV2LTRdelta (28) et pAEV2LTRdelta (17). On a considéré que le provirus a perdu l'intron 2 et éventuellement l'intron 1 du gène delta).

| VIRUS | SONDES ENZYMES | erbB | deltaPst |
|---|---|---|---|
| AEVdelta(17) | EcoRI | – | 2,6 |
| | BamHI | 1,5 | 1,5 |
| AEVdelta(28) | EcoRI | – | 1,5-1,6 |
| | BamHI | 2,2 | 2,2 |

Tableau IV

Tailles (kb) des ADN des provirus AEVdelta (17) et AEVdelta (28)
après cartographie de restriction (figure 10)

(Les valeurs signalées par un astérisque (*) correspondent aux tailles des fragments attendus après digestion par EcoRI et par BamHI).

| PROVIRUS | ENZYMES | TAILLES (kb) SONDES | |
|---|---|---|---|
| | | erbA | erbB |
| AEVdelta(17) | EcoRI | − | $2,6^*$ $1,4$ |
| | BamHI | $1,5^*$ | $1,5^*$ |
| AEVdelta(28) | EcoRI | − | − |
| | BamHI | $2,2^*$ $2,1^*$ | $2,2^*$ $2,1^*$ |

Les corrélations entre ces deux tableaux démontrent la présence des segments d'ADN des provirus renfermant des séquences de globine delta humaine.

A partir du génome de l'AEV cloné dans un plasmide, on a donc construit par recombinaison génétique « in vitro » un génome d'AEV renfermant des séquences du gène humain de globine delta. Après transfection de l'ADN viral recombinant dans des fibroblastes de poulet en culture, on obtient des virions infectieux qui renferment un génome viral portant les séquences humaines delta. Après infection de fibroblastes frais de poulet par ces virions, on observe que les cellules intègrent dans leur génome une information génétique nouvelle représentée par le gène humain de globine delta.

Exemple 6

Stabilité des gènes transférés

1) Elimination des introns des gènes transférés

Lorsque le gène delta est inséré dans l'ADN viral, dans une orientation telle que le brin codant du gène delta coïncide avec le brin codant viral, on observe une perte plus ou moins rapide des introns du gène delta qui conduit à la stabilisation de génomes viraux ne portant plus que les séquences codantes de delta.

La perte des introns n'est pas observée lorsque le gène delta est inséré en orientation inverse dans le génome d'AEV. Il faut en conclure que les introns sont vraisemblablement éliminés par des phénomènes classiques d'épissage dans les ARN viraux qui sont transcrits au cours des cycles successifs d'infection virale.

2) Taille des génomes encapsidés

Les virions AEVdelta (28) peuvent encapsider un génome de 7 kb alors que le génome d'AEVwt ne représente que 5,4 kb. Cette expérience démontre qu'il est possible de transférer au moyen de ce vecteur des séquences d'au moins 1,6 kb. On constate que dans les virions AEVdelta (17) l'ARN génomique de 8 kb n'est pas décelable. Ce défaut d'encapsidation peut résulter, soit d'une taille trop importante du génome viral, soit d'une modification de la structure secondaire du génome viral due à la présence du gène delta.

3) Stabilité des gènes transférés

Lorsque le gène delta est inséré en sens direct, on ne discerne pas de remaniements majeurs au niveau des séquences codantes de ce gène dans les provirus intégrés dans les cellules infectées.

Après infection par AEVdelta (17), on observe l'intégration de provirus présentant des remaniements plus ou moins importants dans la région 5′ du gène delta et dans les séquences pBR flanquantes.

Exemple 7

Sélection des virus pour leur pouvoir transformant - Domaine fonctionnel
de la protéine codée par v-erbB

Les virus générés à partir des plasmides pAEV2LTRdelta (17) et pAEV2LTRdelta (28) induisent la transformation oncogène des fibroblastes de poulet « in vitro ». Dans le stock viral AEVdelta (17), les seuls génomes détectés renferment au moins une partie des séquences pBR et delta insérées. Ces virus donnent naissance à une protéine erbB renfermant en région C-terminale 24 acides aminés de plus que la protéine sauvage. Cette mutation n'affecte donc pas le pouvoir transformant du produit du gène erbB.

Dans le stock viral AEVdelta (28), on trouve en abondance un génome viral recombinant ayant conservé les séquences pBR et les séquences codantes du gène delta. On ne peut exclure la présence de génomes ayant délété ces séquences et dont la structure se rapprocherait de celle du génome sauvage. La persistance du génome recombinant dans certains virions suggère que ces virions bénéficient d'un avantage sélectif de production qui pourrait être lié à leur pouvoir oncogène. Dans ces génomes recombinants, le gène v-erbB présente par rapport au gène sauvage, une modification structurale qui provoque un arrêt prématuré de la traduction de la protéine (fig. 8). La protéine erbB produite par AEVdelta (28) ne renferme pas les 12 acides aminés C-terminaux normalement observés dans la protéine sauvage. Si AEVdelta (28) est capable de transformer les fibroblastes « in vitro », il faut en conclure que ces 12 acides aminés C-terminaux ne jouent pas un rôle essentiel dans le pouvoir transformant de la protéine erbB.

Ces observations démontrent qu'il est possible d'insérer des séquences nucléotidiques étrangères au site EcoRI du gène v-erbB tout en conservant le pouvoir oncogène et sélectif du virus.

Dans les génomes d'AEVdelta (17) et d'AEVdelta (28), le gène delta est associé à ses propres séquences renfermant notamment les signaux de contrôle de transcription. Dans les deux types de provirus, on n'observe pas d'initiation de transcription au niveau du promoteur du gène. Tous les transcrits semblent initiés au niveau du promoteur viral situé dans la LTR de gauche.

Pour expliquer ces résultats, plusieurs hypothèses peuvent être proposées parmi lesquelles on retiendra essentiellement les trois suivantes :

1) Le gène de globine delta est peu exprimé dans les cellules érythrocytiques humaines et on peut imaginer que son promoteur est peu actif.

2) La transcription à partir des provirus a été analysée dans des fibroblastes dans lesquels les gènes endogènes de globine ne sont normalement pas transcrits.

3) Des travaux récents de Cullen et al. (1984) montrent que la LTR de gauche fonctionnelle d'un provirus exerce un effet inhibiteur sur l'initiation de transcription à partir d'un promoteur situé à proximité en aval. On peut penser que ce phénomène est identique à celui que nous observons dans le provirus AEVdelta (28).

Les exemples suivants concernent des virus vecteurs non oncogènes, les matériels et les méthodes mis en oeuvre sont légèrement différents de ceux mis en oeuvre dans les exemples précédents

Matériels et méthodes

1) Sousclones des ADN de l'AEV

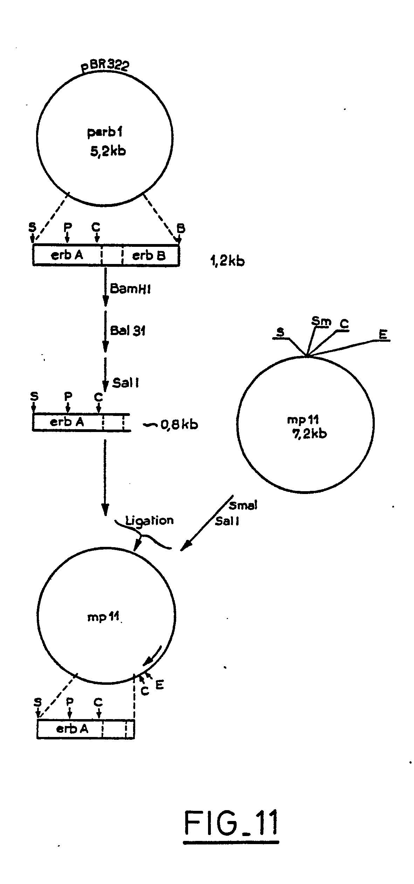

Un fragment SalI-BamHI (1,2 kb) qui couvre la région située entre les gènes v-erbA et v-erbB est inséré entre les sites SalI et BamHI du plasmide pBR322. La structure de ce clone (perb1) est présentée dans la figure 11.

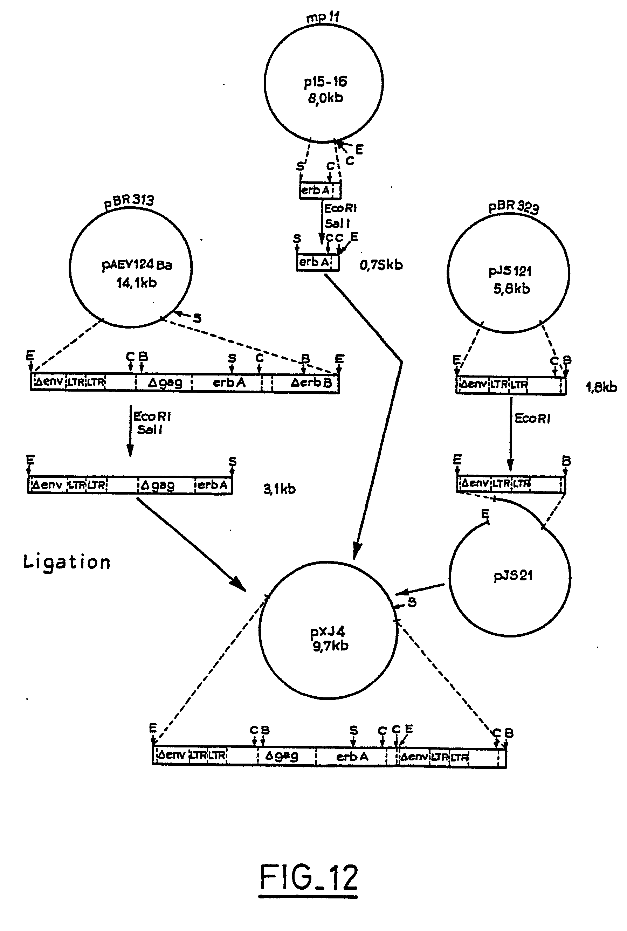

Le clone pJS21 résulte du clonage d'un fragment EcoRI-BamHI (1,8 kb) qui couvre la région 5' virale de pAEV11, entre les sites EcoRI et BamHI de pBR322 (figure 12).

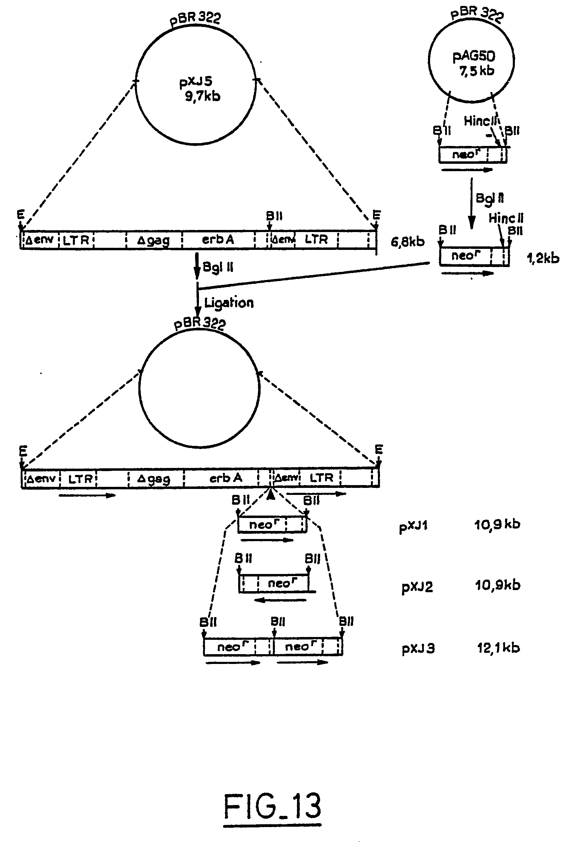

Le recombinant pAG50 a été construit par Colbère-Garapin et al. (1981). Il contient un fragment BglII-HincII (1,1 kb) dérivé du transposon procaryotique Tn5 dans lequel se trouve le gène structural de résistance à la néomycine, Neo$^r$, dépourvu de son propre promoteur (figure 13) (Beck et al., 1982).

2) Digestion des ADN par des enzymes de restriction

La digestion des ADN par les enzymes de restriction est réalisée comme décrit plus haut. Dans certains ca, les ADN digérés sont purifiés par extraction au phénol-chloroforme puis avec un mélange chloroforme-alcool isoamylique puis précipités à l'éthanol.

3) Technique de la digestion des ADN par la nucléase Bal31

La nucléase Bal31 dégrade progressivement l'ADN bicaténaire linéaire à partir des extrémités 5' et 3' de chaque brin. Etant donnée une quantité définie d'ADN, la vitesse de digestion peut être contrôlée en fonction de la concentration de Bal31, de la température de réaction et du temps d'incubation. La spécificité de Bal31 nous permet d'éliminer, d'une façon successive, la séquence nucléotidique au sein de laquelle les sites de restriction utilisables sont absents.

12 µg de l'ADN de perbl linéarisé par BamHI sont incubés à 30 °C en présence d'un tampon d'incubation contenant 12 mM $CaCl_2$, 12 mM $MgCl_2$, 600 mM NaCl, 20 mM Tris-HCl (pH 8), 1 mM Edta, et de 2,2 unités de Bal31. Les aliquotes (2 µg) sont prélevées au bout de 5, 10, 15, 20, 25 et 30 minutes respectivement. L'enzyme est inactivée par addition d'une solution contenant 0,5 % SDS et 25 mM Edta. Ensuite, les ADN digérés sont traités au phénol, précipités à l'alcool absolu froid puis soumis à une digestion par SalI, et, enfin, analysés par électrophorèse sur le gel de 1,6 % agarose. D'après la cinétique de cette réaction obtenue, la vitesse de dégradation d'une molécule est environ 30 nucléotides par minute.

En se basant sur le test préliminaire, 25 µg d'ADN perbl sont soumis à une digestion par Bal31 dans les conditions décrites ci-dessus pendant 15 minutes. Les ADN digérés sont ensuite purifiés et puis leurs extrémités sont réparées par le fragment de « Klenow » dérivé de la DNA polymérase I d'E. coli en présence des 4 nucléotides triphosphates (dATP, TTP, dCTP, dGTP).

### 4) Isolement des fragments d'ADN de différentes tailles

Le mélange des fragments d'ADN de tailles variables est d'abord redissous dans l'eau bidistillée de façon à obtenir une concentration de 100 µg d'ADN par ml. La séparation électrophorétique est réalisée ensuite à 30 volts pendant une nuit (16 heures) sur gel d'agarose préparatif horizontal dont la concentration varie de 0,8 à 1,6 % selon les tailles des fragments à séparer. Des marqueurs de poids moléculaires, en général soit de l'ADN du bactériophage lambda digéré par l'endonucléase de restriction HindIII soit de l'ADN du plasmide pBR322 digéré par l'endonucléase de restriction AluI, sont soumis à électrophorèse parallèlement aux échantillons. La coloration du gel par bromure d'éthidium permet de visualiser les bandes d'ADN sous illumination ultra-violette. La région du gel contenant la bande des fragments d'ADN de tailles déterminée est découpée. Les ADN sont ensuite élués par la méthode de « Freeze Squeeze ». Cette opération conduit à l'obtention des ADN prêts à être soumis à la ligation ou la digestion par les enzymes de restriction.

### 5) Ligation des fragments d'ADN et des vecteurs de clonage

La ligation des fragments d'ADN isolés et des ADN de vecteurs est réalisée selon la technique décrite précédemment.

### 6) Clonage et sélection des plasmides recombinants

La transformation des bactéries, la sélection des bactéries et l'analyse rapide des plasmides sont réalisées selon les techniques décrites précédemment.

### 7) Préparation des ADN plasmidiens

Les ADN de plasmides sont isolés sur gradient de chlorure de césium comme décrit précédemment. Dans certains cas, ces ADN sont ultérieurement purifiés sur coussin de NaCl afin d'éliminer les oligoribonucléotides contaminants (Maniatis, 1982).

### 8) Séquençage

Les fragments à séquencer sont clonés dans les variants mp8 et mp11 du phage M13. Le séquençage des fragments est réalisé selon la méthode de Sanger et al., 1977.

### 9) Attachement d'oligonucléotides synthétiques « linkers »

En cas d'absence de site de restriction compatible avec les extrémités cohésives du fragment d'ADN à cloner, l'emploi des oligonucléotides synthétiques « linkers » contenant la séquence du site de restriction permet de créer de nouveaux sites de clonage dans les vecteurs.

### 9.1) Phosphorylation des « linkers »

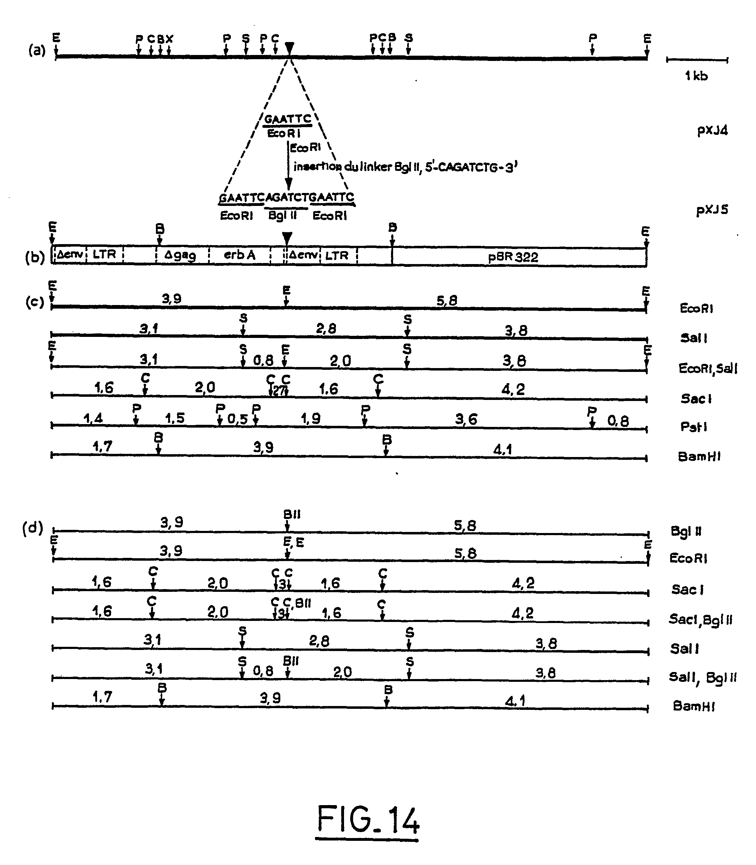

Pour introduire un site de restriction BglII dans le vecteur pXJ4 (figure 14), les « linkers » BglII 5'-d (CAGATCTG)-3' (BRL) sont choisis.

3 µg de « linkers » BglII sont phosphorylés à 37 °C pendant 30 minutes en présence d'une solution de 100 µl composée de 1 µM ATP froid, de 30 µCuries$\gamma$-$^{32}$P-ATP (5 000 µCuries/mole), d'un tampon contenant 60 mM Tris-HCl (pH 7,8), 10 mM $MgCl_2$ et 15 mM 2-mercaptoéthanol et de 80 unités de polynucléotide kinase de phage T4 (BRL). Les « linkers » phosphorylés sont ensuite soumis à divers contrôles de ligase du phage T4 et à être digérés par l'enzyme de restriction BglII.

### 9.2) Modification des extrémités cohésives 5′ sortantes

La digestion des ADN par certaines endonucléases de restriction conduit à l'obtention de molécules portant des extrémités cohésives 5′ sortantes. Pour liguer les bras de vecteur portant les extrémités cohésives 5′ sortantes avec les « linkers » dont les extrémités sont franches, il est donc indispensable de convertir les extrémités cohésives en extrémités franches.

17,5 μg de pXJ4 linéarisé par l'enzyme de restriction EcoRI sont incubés à 25 °C pendant 10 minutes en présence de 39 unités du fragment de « Klenow » dérivé de la DNA polymérase I d'E. coli (BRL), d'un tampon d'incubation contenant 50 mM Tris-HCl (pH 7,4), 10 mM $MgSO_4$, 0,1 mM DTT et 50 μg d'albumine de sérum de bovin par ml, de $3,9 \cdot 10^{-2}$ mM de nucléotides triphosphates froids (dATP, dCTP, dGAP) et de 40 μCuries $^{32}$P-TTP. Au bout de 10 minutes, 10 μl de 2 mM TTP froids sont ajoutés dans 515 μl de milieu réactionnel et l'incubation continue à s'effectuer pendant 10 minutes. Les ADN modifiés sont par la suite purifiés par extraction au phénol et précipités à l'éthanol froid.

### 9.3) Insertion de « linkers » dans les vecteurs

La proportion moléculaire entre les « linkers » et les vecteurs à liguer est en général supérieure à 50/1. 750 μg d'ADN de pXJ4 et 570 ng de « linkers » BglII sont incubés à 15 °C pendant 20 heures en présence de 5 unités de ligase du phage T4 et d'un tampon de ligation identique à celui décrit dans le paragraphe 6), dans un volume final de 60 μl.

Pour éliminer des « linkers » excessifs, le milieu réactionnel de ligation est ensuite incubé en présence de l'enzyme de restriction BglII. Les ADN linéarisés sont ensuite soumis à deux précipitations à — 80 °C pendant 30 minutes en présence d'un mélange d'éthanol et de 1,5 M d'acétate d'ammonium. Ces deux précipitations permettent de récupérer les ADN de hauts poids moléculaires (vecteurs) par centrifugation, tandis que les « linkers » libres et digérés restent dans le surnageant. Les vecteurs ainsi modifiés sont, ensuite, religués et clonés dans les bactéries C600. L'introduction d'un site BglII est identifié par cartographie de restriction.

### 10) Transfection des cellules eucaryotiques

### 10.1) Culture des cellules

Les cellules utilisées pour la transfection sont les suivantes : des cellules L de souris (lignée continue), des cellules de caille QT6 (lignée continue), et des fibroblastes d'embryons de poulet CEF.

Les cultures des cellules L de souris et des fibroblastes d'embryon de poulet sont réalisées selon les techniques décrites précédemment.

Les cellules QT6 de caille sont cultivées à 37 °C en milieu MEM (Gibco) contenant 2,5 mg/ml TPB (tryptose phosphate broth), 10 % de sérum de veau fœtal, 1 % sérum de poulet, 0,19 % bicarbonate de sodium, 100 unités de pénicilline par ml, 100 μg de streptomycine par ml, des acides aminés non essentiels, des vitamines, 4 mM glutamine, et 1 % DMSO (diméthyl sulfoxide).

Ces cellules sont cultivées à 37 °C en atmosphère humide contenant 5 % de $CO_2$.

### 10.2) Transfection des cellules par la méthode de phosphate de calcium

La technique est identique à celle décrite précédemment.

### 10.3) Transfection des cellules par la méthode de polybrène-DMSO

Le principe de cette méthode est basé sur le fait que la présence des polycations (polybrène) conduit à l'attachement des ADN sur les membranes cellulaires. La pénétration des ADN dans les cellules est facilitée par traitement des cellules au DMSO (diméthyl sufoxide) qui augmente la perméabilité des membranes cellulaires (Kawai et al. 1984).

$5 \cdot 10^5$ cellules L de souris sont ensemencées dans une boîte de Pétri dont le diamètre est 60 mm. Après une incubation de 16 heures, les cellules sont mises en contact avec 4 μg d'ADN transformant et incubées à 37 °C en présence d'un ml de milieu de culture frais contenant 25 μg de polybrène. Au bout de 6 heures, les cellules sont traitées avec 2 ml de milieu de culture frais contenant 25 % de DMSO à 37 °C pendant 4 minutes.

### 10.4) Sélection des cellules transformées en présence de G418

2 à 4 jours après la transfection, les cellules sont soumises à la sélection en présence de l'antibiotique G418 dont la concentration est de 800 µg/ml de culture pour les cellules L de souris, de 400 µg/ml de culture pour les cellules QT6, et de 300 µg/ml pour les fibroblastes de poulet.

## Exemple 8

### Localisation de la région 5′ du gène erbB de l'AEV-R

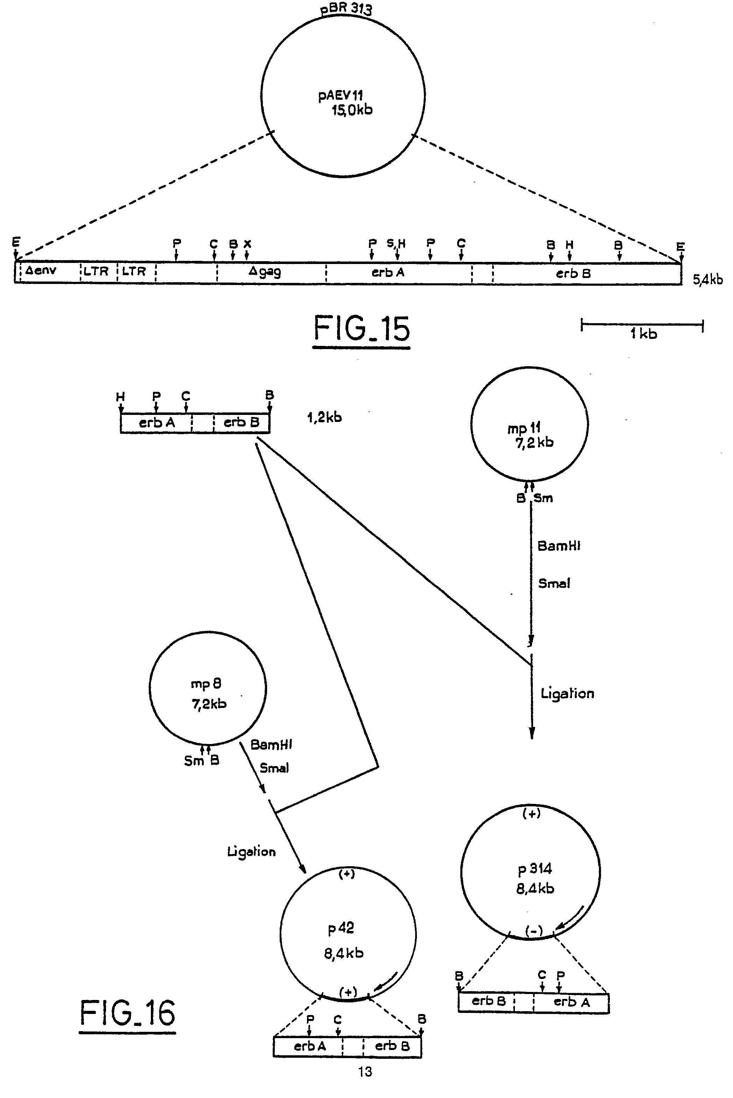

Dans le génome de l'AEV du type sauvage, les deux oncogènes viraux v-erbA et v-erbB occupent la partie majeure des régions codantes (figure 15). Un fragment HincII-BamHI (1,2 kb) renferme la région 3′ du gène erbA, une séquence non codante intercalée entre le gène v-erbA et le gène v-erbB, et la région 5′ du gène v-erbB. Afin de déterminer la position exacte du codon initiateur du gène v-erbB et du site accepteur d'épissage situé entre v-erbA et v-erbB, on a déterminé la séquence nucléotidique de cette région. Le fragment HincII-BamHI est d'abord cloné dans les vecteurs dérivés du bactériophage M13, mp8 et mp11 (figure 16). En état monocaténaire, le brin « plus » du fragment étudié est présent dans le clone p42 dérivé de mp8, tandis que le brin « moins » se trouve dans le clone p314 dérivé de mp11.

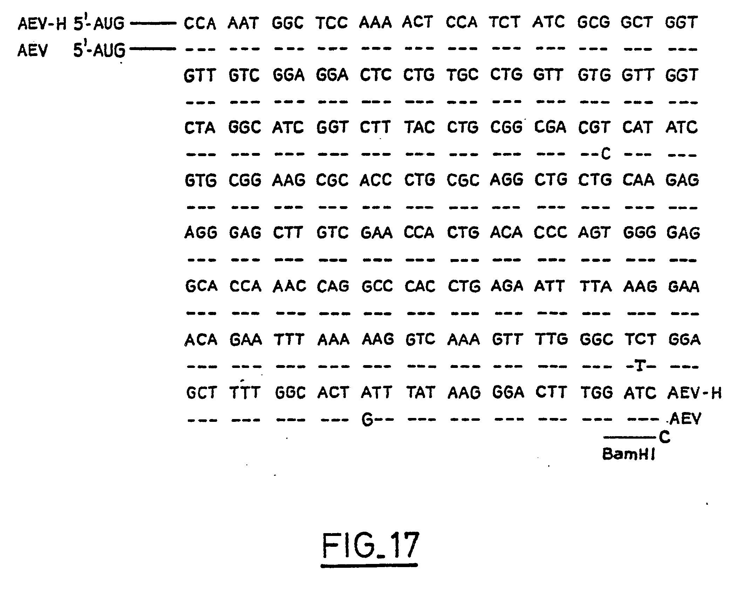

Le séquençage effectué sur le clone p42 permet de déterminer une partie de séquence de la région 5′ du gène v-erbB à partir du site BamHI (figure 17).

La comparaison de la séquence du gène v-erbB de l'AEV et de celle du gène v-erbB du variant AEV-H fournie par Yamamoto et al., 1983 révèle une homologie de 276 nucléotides sur 279. Cette homologie importante permet de déduire que dans le génome d'AEV la distance entre le site BamHI et le codon initiateur « AUG » du gène v-erbB mesure 400 à 500 nucléotides, en supposant que les deux gènes v-erbB sont identiques dans leurs séquences 5′.

## Exemple 9

### Elimination de la séquence nucléotidique de la région 5′
### du gène v-erbB

En considérant l'absence de site de restriction utilisable en amont du site BamHI dans la région 5′ du gène v-erbB, on a employé la nucléase Bal31 pour l'élimination de cette rétgion à partir du site BamHI.

Le clone perbI contenant les séquences de jonction entre v-erbA et v-erbB (figure 11) est digéré par l'enzyme de restriction BamHI puis par la nucléase Bal31 qui opère une dégradation progressive de la région 5′ du gène v-erbB à partir du site BamHI. La digestion est contrôlée de telle façon qu'une séquence nucléotidique d'environ 0,45 kb soit éliminée en amont du site BamHI. La séparation des fragments viraux raccourcis du vecteur pBR322 est réalisée par digestion par l'enzyme de restriction SalI, suivie d'une électrophorèse sur gel d'agarose préparatif.

Une famille de fragments viraux dont les tailles avoisinnent 0,8 kb est isolée du gel. Ces fragments présentent une délétion plus ou moins étendue dans la région 5′ du gène v-erbB.

## Exemple 10

### Etude structurale de la région non codante entre les gènes
### v-erbA et v-erbB

Pour déterminer la délétion introduite dans la région 5′ du gène v-erbB, les fragments raccourcis Bal31 sont d'abord clonés entre les sites SalI et SmaI de mp11 (figure 11).

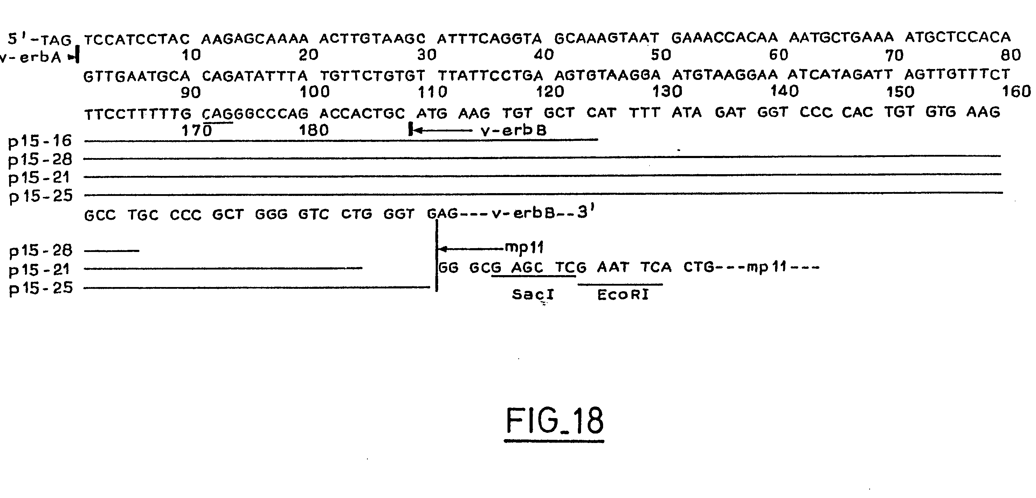

4 clones séquencés montrent des résidus plus ou moins longs de séquence v-erbB (figure 18).

Parmi tous ces clones, le clone p15-16 ne conserve que les 13 premiers nucléotides du gène v-erbB.

L'analyse des séquences en amont du codon « ATG » du gène v-erbB indique qu'il existe un site consensus accepteur d'épissage « CAG » en position 171 précédé par une séquence de 15 nucléotides « TTTCTTTCCTTTTTG » (figure 18) (Sharp, 1981).

Le codon de terminaison « TAG » du gène v-erbA se trouve à 188 nucléotides en amont du codon « ATG » du gène v-erbB. D'autre part, la séquence non codante est caractérisée par une fréquence élevée de doublets et de triplets des nucléotides A et des nucléotides T respectivement.

L'étude comparative avec la séquence de l'AEV-H fournie par Yamamoto et al., 1983 montre que les séquences immédiatement en aval du codon « ATG » du gène v-erbB sont pratiquement identiques entre l'AEV et l'AEV-H.

## Exemple 11

### Construction du mutant pAEV-ΔerbB

Dans le but de remplacer le gène v-erbB par un gène « marqueur », le gène Neo$^r$ dérivé du transposon bactérien Tn5 a été inséré dans le génome modifié de l'AEV.

La stratégie adaptée dans cette construction est schématisée dans la figure 12.

Le clone p15-16 est tout d'abord digéré par les enzymes de restriction EcoRI et SalI. Un fragment SalI-EcoRI (0,75 kb), qui couvre la région 5' du gène v-erbA, la séquence non codante contenant le site accepteur d'épissage, les 13 premiers nucléotides du gène v-erbB et 11 nucléotides dérivés du polylinker de mp11, est isolé par électrophorèse sur gel d'agarose. La double digestion du clone pAEV124Ba par SalI et EcoRI permet d'isoler un fragment de 3,1 kb contenant entre autre les LTR, les séquences résiduelles gag et la moitié 5' du gène v-erbA de l'AEV.

Ces deux fragments sont ligués en tandem et clonés dans le site EcoRI du plasmide pJS21 qui fournit les séquences virales 3'. Le plasmide final contenant un génome viral reconstitué, dépourvu du gène v-erbB, est appelé pXJ4. La structure du plasmide pXJ4 est confirmée par les résultats de la digestion par SalI, SacI, PstI, BamHI et SalI + EcoRI.

Exemple 12

Introduction du gène structural Neo$^r$ de Tn5
dans le vecteur rétroviral pXJ4

L'obtention du vecteur rétroviral pXJ4 offre la possibilité d'insérer un gène de sélection à l'emplacement du gène v-erbB délété sans augmentation de la taille totale du vecteur par rapport à l'AEV du type sauvage.

Le plasmide pAG50, présenté sur la figure 13, comporte le gène structural de résistance à l'antibiotique néomycine (gène Neo$^r$) limité par des sites BglII (Colbère-Garapin et al., 1981). La digestion de pAG50 par BglII permet d'isoler un fragment de 1,2 kb contenant le gène Neo$^r$.

Pour introduire ce fragment dans le plasmide pXJ4 à l'emplacement du gène v-erbB délété, un site de restriction BglII a dû être créé dans le plasmide pXJ4 au niveau du site EcoRI situé en aval du site accepteur d'épissage. Le plasmide pXJ4 a été soumis à une digestion partielle par EcoRI, on a ensuite isolé, sur gel d'agarose préparatif, les molécules linéarisées de 9,7 kb résultant d'une seule coupure par EcoRI. Les extrémités des fragments isolés furent ensuite réparées en extrémités droites puis liées à des « linkers » BglII.

Cette procédure donne naissance à deux sites EcoRI encadrant le site BglII créé (figure 14). Le génome ainsi produit est représenté par le plasmide pXJ5 (figure 14).

La digestion de pXJ5 par BglII prouve l'insertion d'un site BglII. Les doubles digestions par BglII et SacI, ou BglII et SalI, confirment la position de ce site en aval du codon initiateur « AUG » résiduel du gène v-erbB. La restitution du site EcoRI est confirmée par digestion par EcoRI.

L'insertion du gène Neo$^r$ dans le vecteur pXJ5 est réalisée par l'opération suivante : Le plasmide pXJ5 est d'abord linéarisé au site BglII puis lié avec le fragment BglII-BglII contenant le gène Neo$^r$ isolé de pAG50.

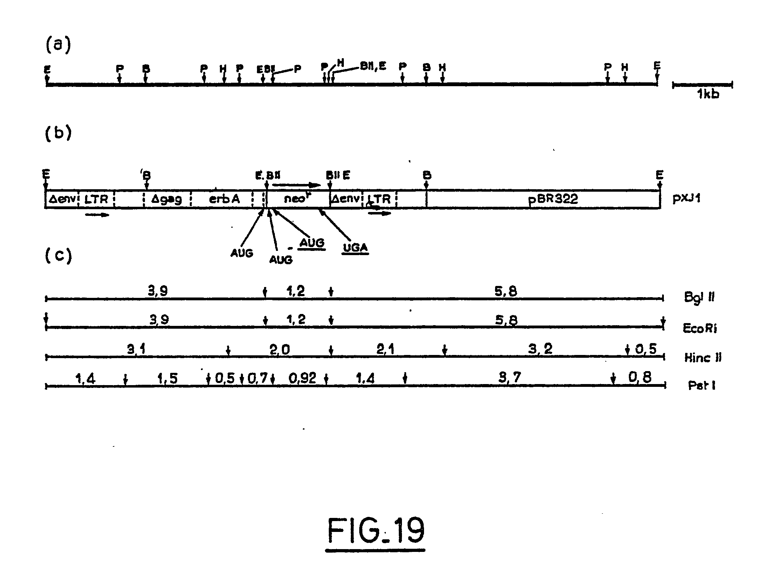

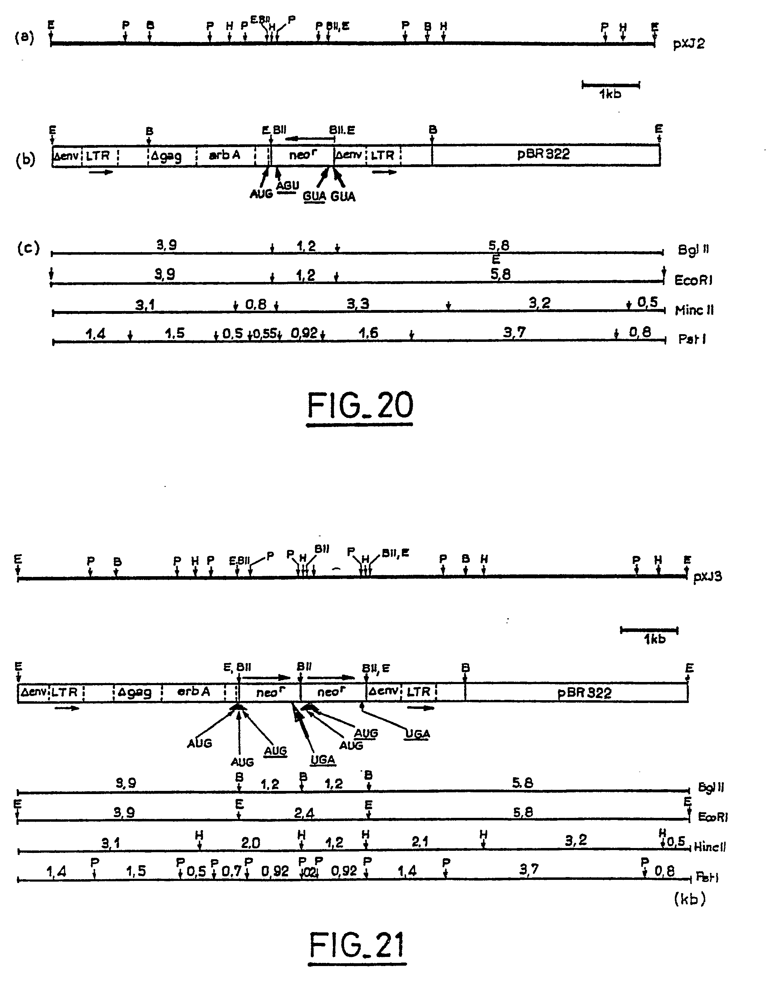

L'analyse des clones recombinants montre que le clone pXJ1 comporte une seule copie du gène Neo$^r$ insérée dans la même orientation transcriptionnelle que celle du vecteur rétroviral (figure 19). Le clone pXJ2 (figure 20) porte une seule copie du gène Neo$^r$ dont l'orientation est opposée à celle du vecteur. Le clone pXJ3 (figure 11) possède deux copies du gène Neo$^r$ associées en tandem, toutes deux orientées dans le sens de transcription du génome viral. Les sites d'insertion, le nombre et l'orientation des gènes Neo$^r$ dans les plasmides pXJ1, pXJ2 et pXJ3, ont été déterminés par digestion des plasmides avec chacune des enzymes BglII, EcoRI, HincII et PstI.

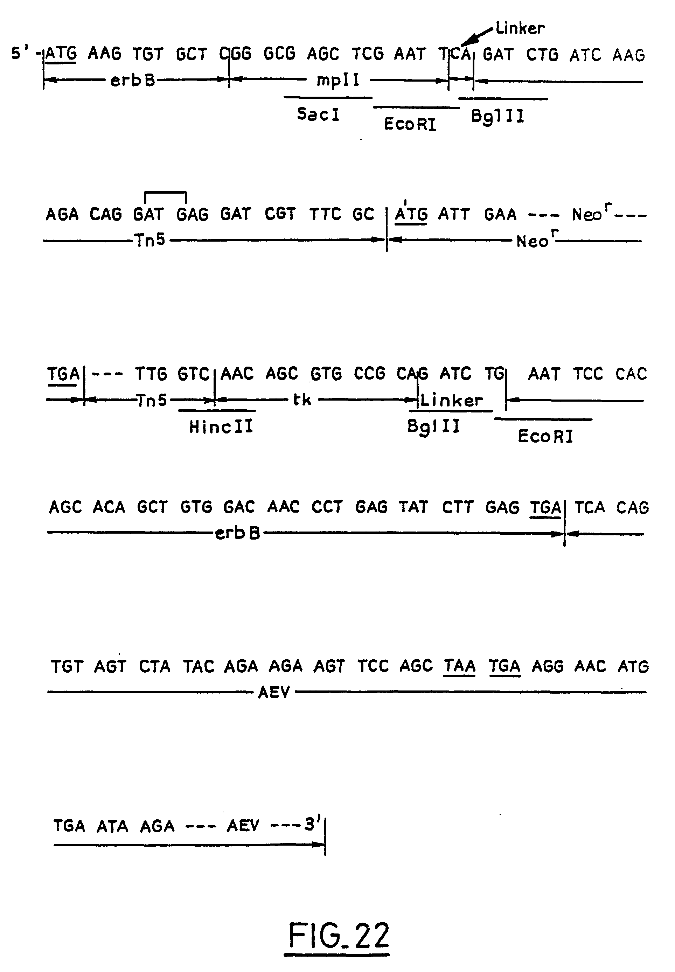

Les séquences de jonction entre le gène Neo$^r$ et les vecteurs rétroviraux dans les clones pXJ1 et pXJ3 sont présentées dans la figure 22.

Exemple 13

Expression du gène Neo$^r$ dans les cellules procaryotiques

Les bactéries du type Kan$^s$ (sensible à la kanamycine) transformées par pXJ1, pXJ2 et pXJ3 sont cultivées à 37 °C pendant 16 heures en présence d'un milieu liquide nutritif contenant de la kanamycine dont la concentration varie de 0 à 600 μg/ml de culture. Les bactéries transformées par le clone pXJ5 sans gène Neo$^r$ sont aussi testées comme témoin.

En présence de 50 μg de kanamycine par ml de culture, seules les bactéries transformées par les recombinants portant le gène Neo$^r$ montrent une croissance normale. Néanmoins, lorsque la concentration de kanamycine est supérieure à 50 μg/ml de culture, les bactéries hébergeant les clones pXJ1 et pXJ3 présentent une croissance au moins 10 fois supérieure à celle des bactéries contenant le plasmide pXJ2. On n'observe aucune différence de sensibilité à la kanamycine entre les bactéries qui renferment pXJ1 et celles qui renferment pXJ3.

23

Exemple 14

Expression du gène Neo[r] dans les cellules eucaryotiques

1) Transfection en présence de phosphate de calcium

1.1) Transfection des cellules L de souris (tableau V)

$5 \cdot 10^5$ cellules L de souris sont soumises à une transfection de 15 µg d'ADN de chacun des vecteurs pXJ1, pXJ2 ou pXJ3. Le plasmide pAG50 dans lequel le gène Neo[r] est sous le commandement des éléments du contrôle de transcription du gène tk du virus Herpès simplex est choisi comme témoin (Colbère-Garapin et al., 1981).

24 heures après la transfection, les cellules sont sélectionnées en milieu liquide en présence de 800 µg de G418 par ml de culture. Au bout de 14 de sélection, les cellules n'ayant pas subi de transfection ainsi que les cellules transfectées avec le plasmide pXJ2 sont toutes mortes. Par contre, les cellules transfectées avec pXJ1 et pXJ3 survivent et commencent à former les colonies. Les nombres de colonies résistantes sont comptés au bout de 19 jours de sélection. L'efficacité de transformation montrée par le clone pXJ3 portant deux copies de gène Neo[r] est inférieure à celle du clone pXJ1 comportant une seule copie.

1.2) Transfection des cellules de caille QT6 (tableau V)

$5 \cdot 10^5$ cellules de caille QT6 sont transfectées avec 5 µg des vecteurs rétroviraux pXJ1, pXJ2 et pXJ3 respectivement. Parallèlement, 15 µg de pAG50 sont utilisés comme témoin. 25 heures après la transfection, les cellules sont soumises à la sélection en milieu liquide en présence de 400 µg de G418 par ml de culture.

Au bout de 7 jours de sélection, les cellules normales non transfectées sont toutes mortes sous la pression d'une telle sélection. Pour faciliter le développement de colonies résitantes, la concentration de G418 est ramenée à 200 µg/ml. Après une sélection continue de 4 jours, 33 colonies résistantes sont formées dans le cas de pXJ1 tandis que les autres clones ne présentent aucune activité de transformation.

1.3) Transfection des fibroblastes d'embryon de poulet (tableau V)

$5 \cdot 10^5$ fibroblastes d'embryon de poulet secondaires sont transfectés avec 5 µg des vecteurs pXJ1, pXJ2 et pXJ3 respectivement. En même temps, 15 µg de pAG50 sont employés comme témoin. 6 jours après la transfection les cellules transfectées sont sélectionnées en milieu liquide contenant 300 µg de G418 par ml de culture. Au bout de deux semaines de sélection, les fibroblastes normaux non transfectés ainsi que les fibroblastes transfectés par pXJ2 et pAG50 sont tous morts. En revanche, les cellules transfectées avec pXJ1 et pXJ3 commencent à former des colonies. Les nombres de colonies résistantes sont comptés à la suite d'une sélection continue de 3 jours. Le clone pXJ1 contenant une copie du gène Neo[r] fournit environ deux fois plus de colonies résistantes que le clone pXJ3. Les fibroblastes transformés par les vecteurs pXJ1 et pXJ3 présentent une morphologie identique à celle des cellules normales.

2) Transfection des cellules L de souris en présence de polybrène (tableau V)

$5 \cdot 10^5$ cellules L de souris sont transfectées avec 4 µg de chacun des ADN transformants pXJ1, pXJ2, pXJ3 et pAG50. 48 heures après la transfection les cellules sont cultivées en milieu liquide contenant 800 µg de G418 par ml de culture. Au bout de 12 jours de sélection, les cellules normales non transfectées ainsi que les cellules transfectées favec les plasmides pXJ2 et pAG50 sont toutes mortes. Par contre, les clones pXJ1 et pXJ3 induisent le développement de colonies résistantes.

L'efficacité de la méthode de tansfection au polybrène-DMSO apparaît beaucoup plus élevée que celle de la méthode au phosphate de calcium.

## Tableau V

### Transfection des cellules eucaryotiques par les vecteurs rétroviraux

| Clone | Structure | ADN (µg) | Nombre de colonie transformée | Méthode de transfection |
|---|---|---|---|---|
| **Nombre de cellule L de souris:5 10⁵** | | | **Selection en présence de G418 800 µg/ml** | |
| pXJ1 | LTR — neo^r — LTR | 15 | 19 | CaPO₄ |
| pXJ2 | LTR — neo^r ← LTR | 15 | 0 | |
| pXJ3 | LTR — neo^r — neo^r — LTR | 15 | 17 | |
| pAG50 | tkpr — neo^r — tkpolyA | 15 | · 9 | |
| pXJ1 | LTR — neo^r — LTR | 4 | 32;36 | POLYBRENE-DMSO |
| pXJ2 | LTR — neo^r ← LTR | 4 | 0;0 | |
| pXJ3 | LTR — neo^r — neo^r — LTR | 4 | 20;24 | |
| pAG50 | tkpr — neo^r — tkpolyA | 4 | 35;32 | |
| **5 10⁵ cellules de caille QT6** | | | **G418:400 µg/ml** | |
| pXJ1 | LTR — neo^r — LTR | 5 | 33 | CaPO₄ |
| pXJ2 | LTR — neo^r ← LTR | 5 | 0 | |
| pXJ3 | LTR — neo^r — neo^r — LTR | 5 | 0 | |
| pAG50 | tkpr — neo^r — tkpolyA | 5 | 0 | |
| **5 10⁵ fibroblastes de poulet embryonnaire(CEF)** | | | **G418:300 µg/ml** | |
| pXJ1 | LTR — neo^r — LTR | 5 | 7 | CaPO₄ |
| pXJ2 | LTR — neo^r ← LTR | 5 | 0 | |
| pXJ3 | LTR — neo^r — neo^r — LTR | 5 | 3 | |
| pAG50 | tkpr — neo^r — tkpolyA | 5 | 0 | |

● L'organisation transcriptionnelle des ADN transformants est montrée dans ce tableau. L'orientation transcriptionnelle de chaque composant est indiquée par une flèche horizontale.

● tkpr : promoteur du gène tk de HSV

● tkpolyA : signal de polyadénylation du gène tk de HSV.

## Exemple 15

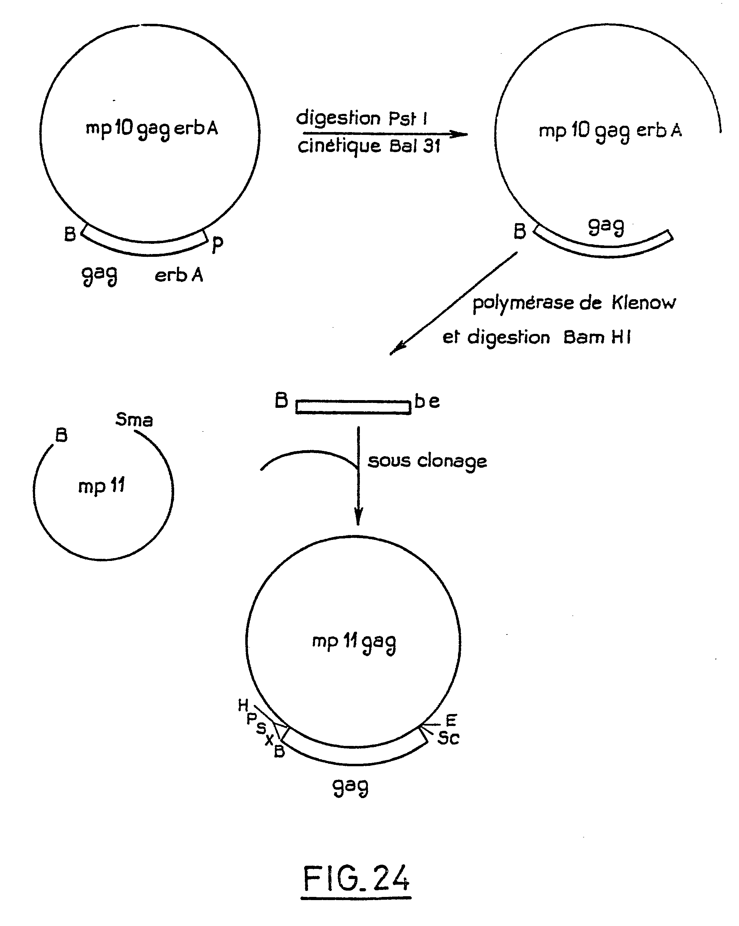

### 1) Préparation du clone mJS21 erb⁻ (figure 24)

Ce clone a été préparé à partir du clone pJS21 (exemple 2, figure 23) qui contient, outre les séquences déjà décrites, une quarantaine de nucléotides appartenant à l'oncogène erbB qui sont situés en 5' du gène env. Cette séquence résiduelle a été éliminée par linéarisation EcoRI et digestion avec Bal31.

Les fragments obtenus sont réparés par la DNA polymérase I. L'insert est relaché au site BamHI par digestion enzymatique et purifié par électroélution après séparation sur gel d'agarose.

Les fragments purifiés sont ensuite clonés dans la forme réplicative du phage M13 préalablement préparée. Après ligation et transformation d'une culture bactérienne JM103 compétente, les phages monocaténaires recombinants sont analysés par hybridation avec un clone contenant le fragment JS21 intégré en sens inverse. Les clones présomptifs sont alors vérifiés par séquençage.

Le clone mJS21 erb⁻ retenu débute dans le gène env 9 nucléotides en 3' du codon terminateur du gène erbB.

2) Préparation du clone mp11gag

mp10gag erbA contient les 400 derniers nucléotides du gène gag ainsi que les 400 premiers nucléotides du gène erbA. De la même manière que pour le clone mJS21 erb⁻, la séquence erbA du clone mp10gag erbA a été éliminée par digestion avec l'exonucléase Bal31 (figure 24).

Dans le clone mp11gag retenu, après analyse par séquençage des clones présomptifs, le gène erbA est totalement éliminé ainsi que 12 nucléotides 3' terminaux du gène gag.

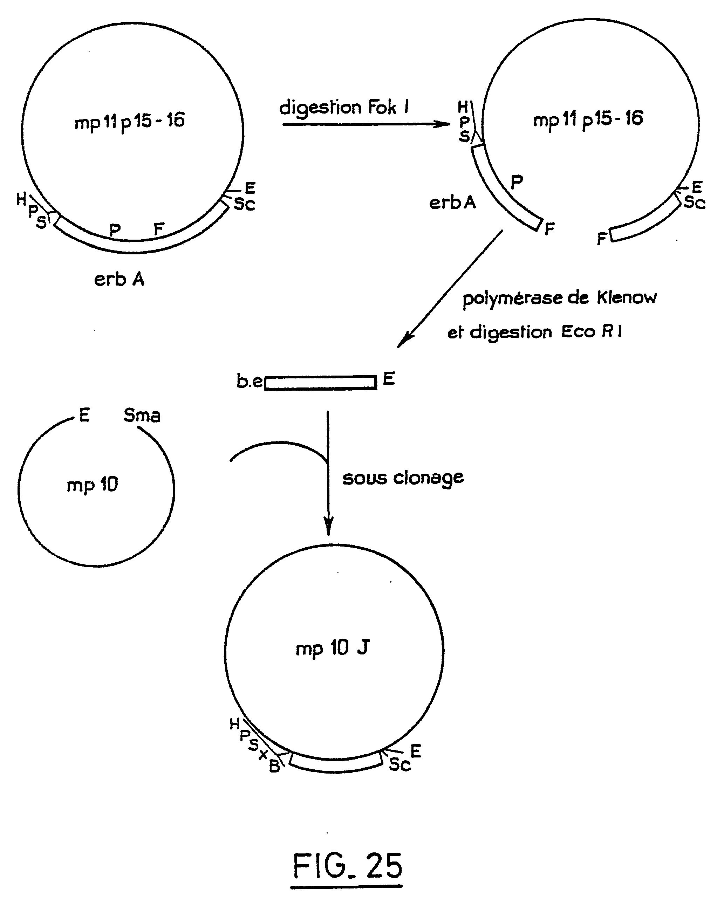

3) Préparation du clone mp10-J (figure 25)

Le fragment J a été préparé à partir du clone mp11 p15-16 (exemple 10) (appelé 15-16). Celui-ci contient, outre la séquence J, les 550 derniers nucléotides du gène erbA. Après analyse de la séquence publiée par Deduire et coll. (1984) concernant cette région de l'AEV, on a identifié un site de restriction FokI unique situé à proximité de la jonction des deux séquences erbA et J. Ainsi, la digestion de l'ADN du clone mp11 p15-16 par cette endonucléase libère le fragment J. En 5' de J, il ne subsiste que 10 nucléotides de la séquence codante du gène erbA.

Exemple 16

Reconstruction du vecteur dépourvu de séquences oncogènes

L'assemblage des différents clones obtenus à l'exemple 15 est réalisé dans le plasmide bactérien pBR328 (Bolivar et coll., 1977). Ce dernier peut, en effet, insérer des fragments de grande taille et être sélectionné pour sa résistance à l'ampicilline.

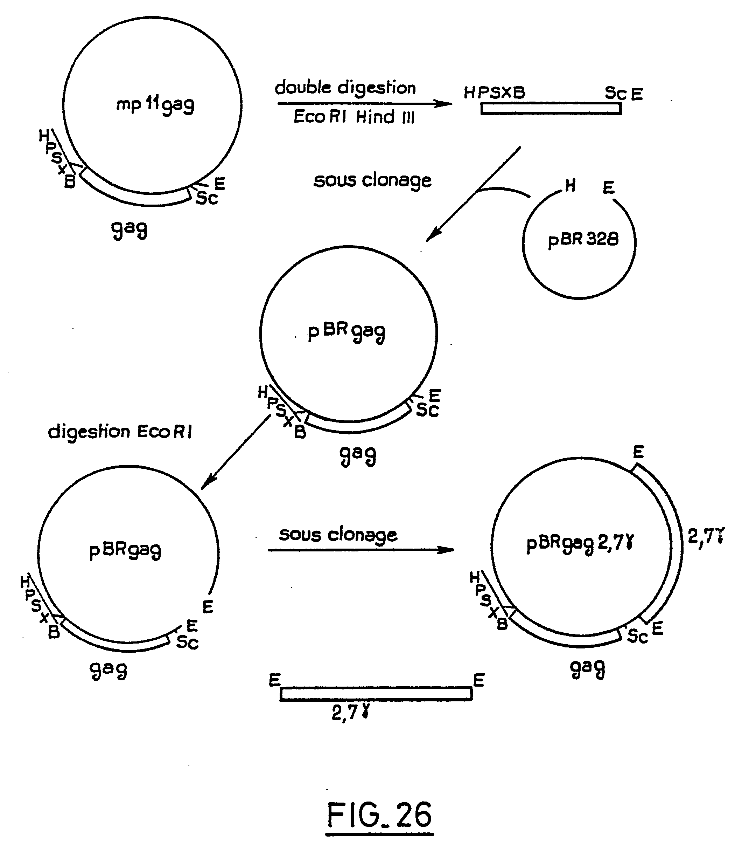

1) Clonage de la séquence gag du mp11gag dans le pBR328 (figure 26)

Le premier fragment introduit dans le plasmide est celui qui contient le gène gag présent sur le clone mp11gag. Le clone pBRgag ainsi obtenu est analysé par cartographie aux endonucléases de restriction.

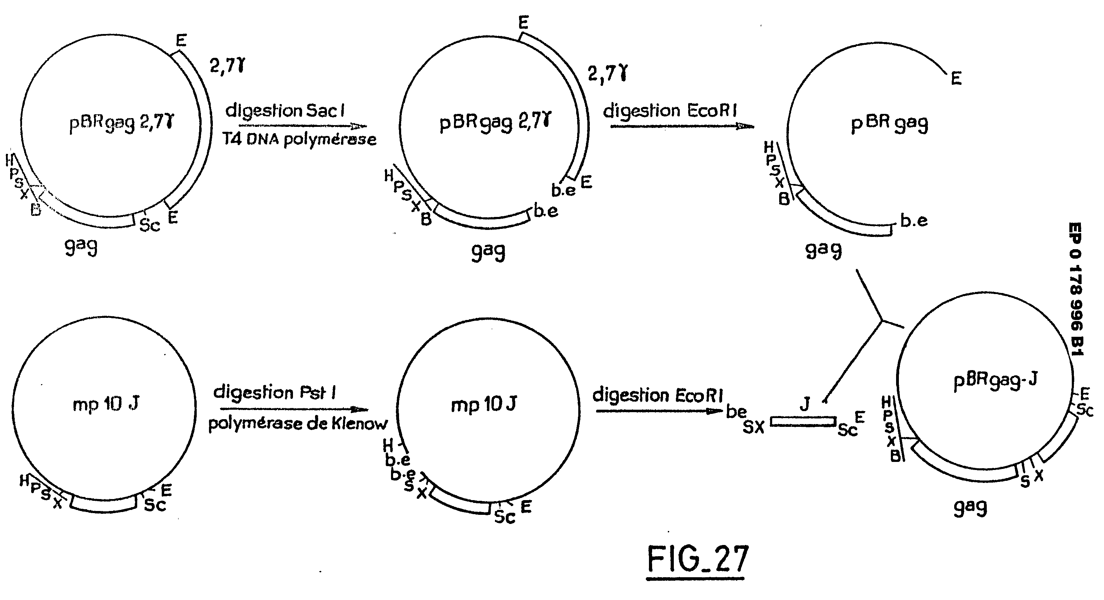

2) Préparation du clone pBRgag-J (figure 27)

a) Préparation du vecteur pBRgag

La préparation du vecteur pBRgag pour l'insertion du fragment J a nécessité une série d'étapes destinées à conserver le site EcoRI situé en 3' de la séquence gag. Ce site EcoRI doit, en effet, dans la construction finale servir de site unique de clonage pour les gènes à insérer dans le vecteur rétroviral.

Pour préparer le vecteur pBRgag, on utilise la T4 DNA polymérase afin d'obtenir des extrémités franches après digestion par l'endonucléase SacI. L'activité de cette T4 DNA polymérase n'est pas rigoureusement contrôlable et compte tenu du fait que les deux sites enzymatiques utilisés pour ce sous-clonage (SAcI et EcoRI) appartiennent au polylinker du phage M13 et sont très proches l'un de l'autre, on risque de perdre le site EcoRI. C'est la raison pour laquelle on introduit dans le vecteur pBRgag, afin d'éloigner le site EcoRI du site SacI, un fragment d'ADN de 2,7 kb appartenant au gène de gamma globine humaine. Cette astuce technique permet, après différentes étapes, de conserver le site EcoRI.

b) Préparation du clone mp10-J

Le fragment J tel qu'il a été préparé est contenu dans le vecteur mp10 entre les sites SmaI et EcoRI du polylinker. Ceci entraîne l'apparition, en 5' du fragment J, de 5 sites de restriction (BamHI, XbaI, SalI, PstI et HindIII). Or, pour la suite de la construction, le site BamHI doit être supprimé. Ceci est réalisé par digestion avec l'endonucléase BamHI, puis remplissage par la DNA polymérase I et ligation. Après analyse par cartographie des formes réplicatives du phage M13, les clones présomptifs sont analysés par séquençage.

Le clone mp10 ainsi préparé est alors cloné dans le pBRgag. Après transformation d'une culture bactérienne C600 R/S compétente, les clones recombinants pBRgag-J sont analysés par cartographie. Le clonage du fragment J dans le pBRgag crée le site unique XbaI situé en 5' de J qui sera utilisé pour le clonage des gènes à insérer dans le vecteur.

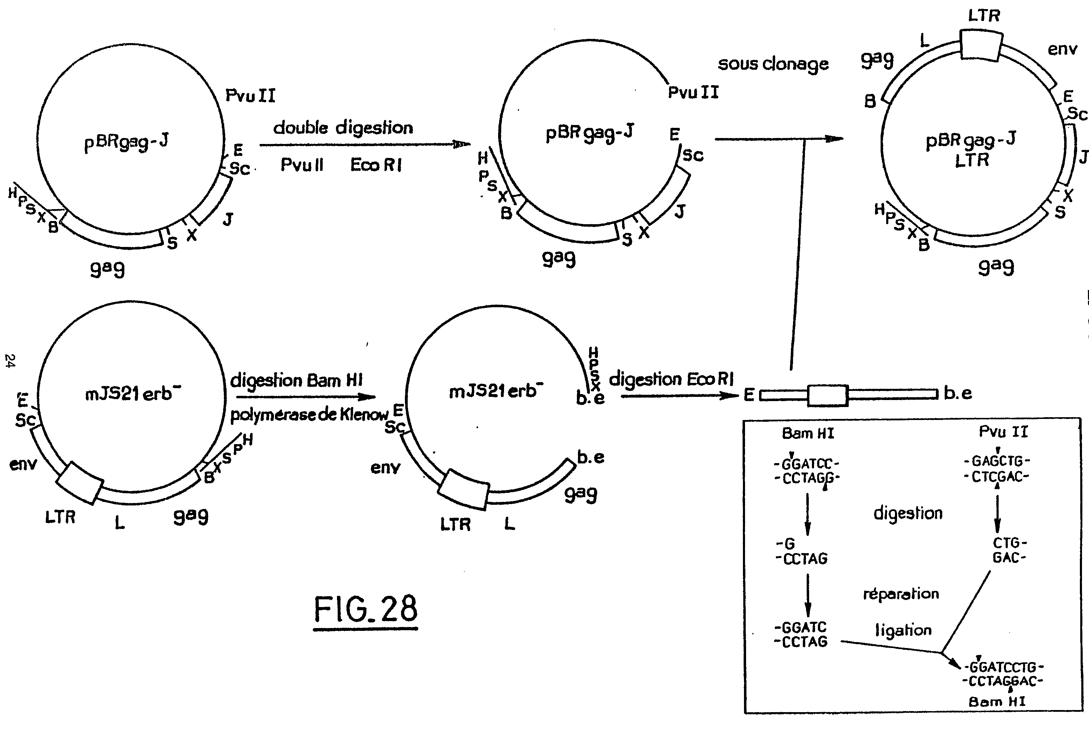

3) Clonage du fragment JS21 erb⁻ en 3' de J dans le vecteur pBRgag-J (figure 28)

Le vecteur pBRgag-J et le fragment JS21 erb⁻ sont séparés comme indiqué dans la figure. Le clonage du fragment JS21 erb⁻ en 3' de J apporte au vecteur la séquence du gène env ainsi que la LTR 3'. Il crée également le site unique EcoRI qui sera utilisé pour le sous-clonage des gènes à insérer dans le vecteur. Les clones pBRgag-J env LTR ainsi obtenus sont analysés en cartographie.

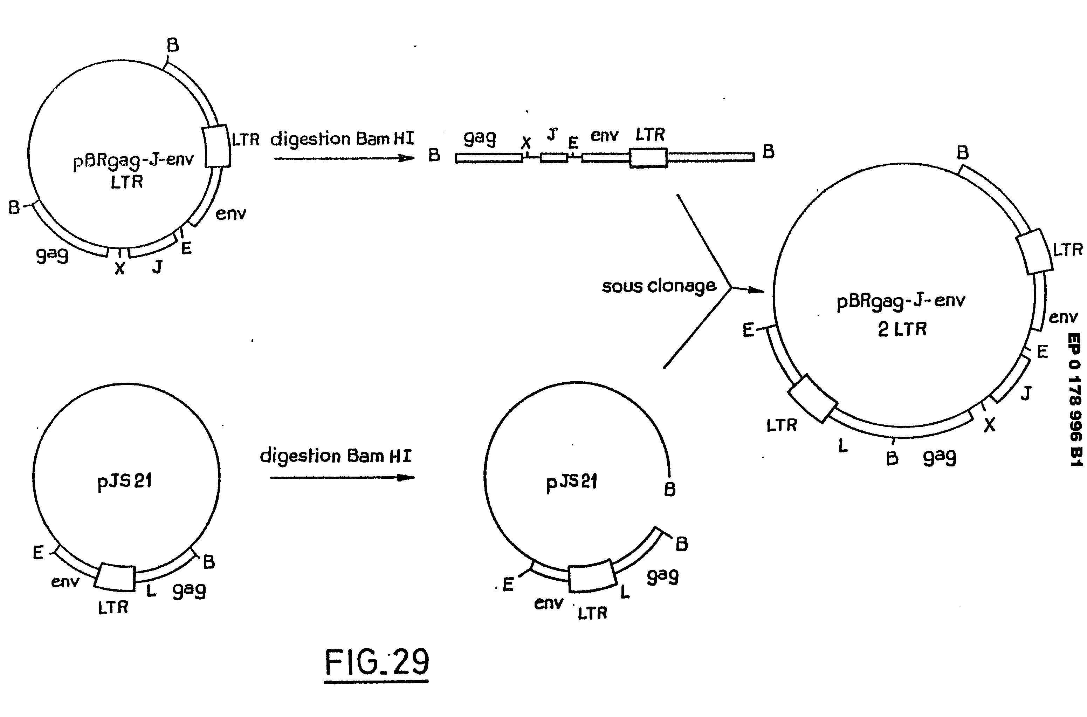

4) Clonage du vecteur pBRgag-J env LTR dans le pJS21 (figure 29)

Le fragment pBRgag-J env LTR et le pJS21 sont préparés comme indiqué dans la figure. Ce sous-clonage apporte au vecteur la LTR 5', la séquence leader, et permet de reconstituer le gène gag tel qu'il existe dans l'AEV sauvage. Le clone pBRgag-J-env 2LTR ainsi obtenu est analysé par cartographie.

Il est à noter que cette étape conduit à l'apparition d'un site EcoRI supplémentaire situé à la jonction des séquences AEV et pBR322. Celui-ci devra être éliminé ultérieurement par digestion partielle afin de n'avoir qu'un site EcoRI situé en 3' du fragment J et qui servira au clonage des gènes à insérer.

Ce clone pBRgag-J-env 2LTR contient entre les 2 LTR le génome de l'AEV sans séquence oncogène, il possède également un site de clonage unique XbaI situé en 5' du fragment J.

Un fragment de restriction XbaI du transposon Tn5 incluant le gène Neo$^r$ est introduit dans le site XbaI de pBRgag-J-env 2LTR, le plasmide résultatn est pAEV TSN qui induit la résistance au G418 pour les cellules aviaires transformées.

Exemple 17

Transfert de gènes sous la dépendance du promoteur viral

Dans une première série de construction, le gène Neo$^r$ du transposon bactérien Tn5 a été inséré à la place du gène v-erbB (exemples 12 et suivants). Les virus produits pouvaient induire la résistance des cellules infectées à la drogue G418 démontrant ainsi la transcription et la traduction correctes du gène inséré. Cette construction laissait cependant subsister plusieurs codons d'initiation potentiels de traduction, dans des cadres de lectures diverses, en amont de la séquence codante du gène Neo$^r$. Afin d'augmenter l'efficacité de traduction du gène et dans le but de produire un vecteur universel de transfet, la séquence du vecteur a été modifiée afin de ne laisser subsister que les codons initiateurs normalement utilisés pour la traduction des gènes viraux.

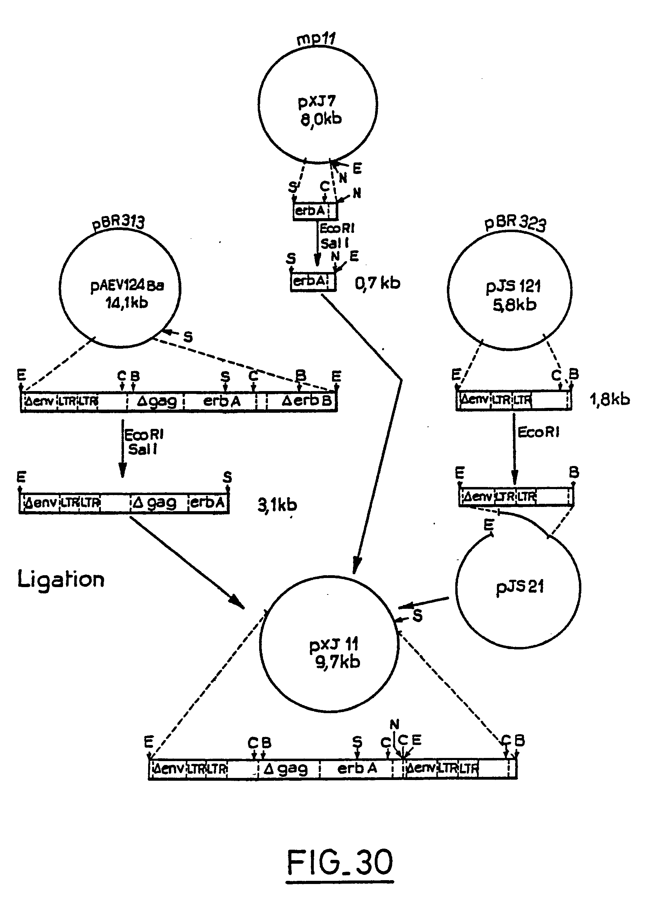

a) Construction d'un vecteur universel portant un site de clonage NdeI au niveau d'un codon initiateur ATG (figure 30)

La région codante du gène v-erbB présente en 5' la séquence suivante :

$$- - - 5' \quad \overline{GGC} \quad \overline{CCA} \quad \overline{GAC} \quad \overline{CAC} \quad \overline{TGC} \quad \underline{\overline{ATG}} \quad \overline{AAG} \quad \overline{TGT} \; 3' \; - - -$$

Après splicing de l'ARN génomique en ARN sous-génomique, cette séquence s'ordonne à la suite de la séquence leader de l'ARN sous-génomique qui porte le codon initiateur de traduction. Cet arrangement fournit la trame codante indiquée ci-dessus (⌐¬).

On a créé un site NdeI recouvrant le codon ATG par la méthode de mutagénèse ponctuelle dirigée, selon Zoller et coll. (1983), en utilisant un oligonucléotide synthétique de séquence :

$$5' \quad CACTTCA\overline{TATGGTGGTCTGG}$$
$$NdeI$$

La modification de séquence a été réalisée sur le clone p15-16 (figure 12) pour fournir le clone pXJ7 dans lequel la séquence en 5' du gène v-erbB est maintenant la suivante :

$$- - - 5' \quad \overline{GGC} \quad \overline{CCA} \quad \overline{GAC} \quad \overline{CAC} \quad \overline{CAT} \quad \overline{ATG} \quad \overline{AAG} \quad \overline{TGT} \; 3' \; - - -$$
$$NdeI$$

Cette séquence a été insérée dans le vecteur selon la construction présentée sur la figure 30 pour fournir finalement le génome viral vecteur porté par le plasmide pXJ11 à partir de pJS21 et PAEV124 obtenu par délétion PstI-PstI dans le gène v-erbA de pAEV11.

Ce clone offre donc un système universel de construction de génomes vecteurs dans lesquels toute séquence insérée au site de clonage NdeI sera automatiquement en phase avec la trame de traduction protéique initiée dans la région leader des ARN viraux transcrits. Les gènes qui devront être intégrés dans ces vecteurs devront par conséquent comporter un site de restriction NdeI au niveau de leur codon ATG initiateur.

b) Construction d'un gène Neo$^r$ portant un site de restriction NdeI au niveau du codon initiateur

La séquence nucléotidique au niveau de la région 5' du gène Neo$^r$ est la suivante :

```
5'GATCTGATCAAGAGACAGGATGAGGATCGTTTCGC ATG ATT GAA --- 3'
```

Cette séquence a été modifiée par mutagénèse ponctuelle dirigée au moyen d'un oligonucléotide de synthèse de séquence :

$$5' \quad \text{GATCGTTTCATATGATTGAAC} \quad 3'$$
$$\text{NdeI}$$

On a ainsi construit le plasmide pXJ10 contenant le gène Neo$^r$ modifié dans lequel un site NdeI apparaît au niveau du codon initiateur. La partie codante du gène peut être isolée de ce plasmide après digestion par NdeI et BglII.

c) Construction d'un vecteur rétroviral portant le gène Neo$^r$ modifié

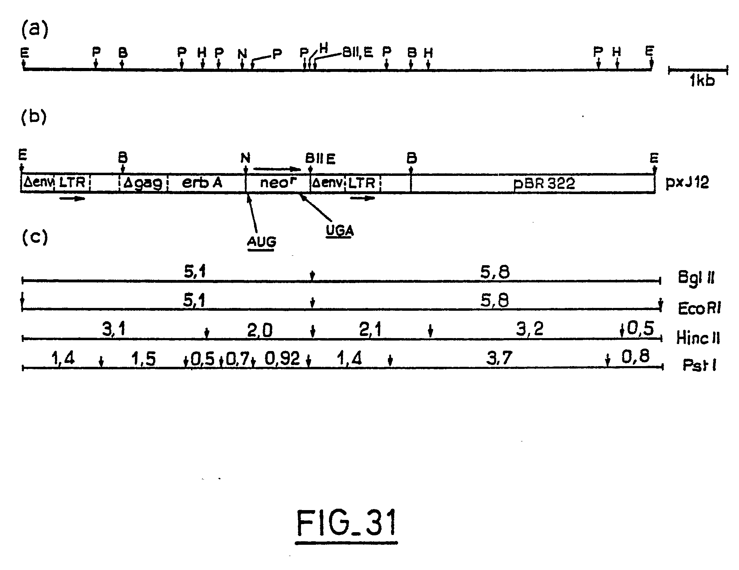

La partie codante du gène Neo$^r$ modifié a été insérée dans le plasmide pXJ11 au niveau du site NdeI de telle façon que la séquence Neo$^r$ soit dans le sens de transcription normal du génome viral. On a obtenu ainsi le plasmide pXJ12. La structure finale du plasmide et du génome viral a été vérifiée par cartographie de restriction notamment pour confirmer la présence du site NdeI et par conséquent la validité de la séquence avoisinant le codon initiateur du gène. La structure de ce plasmide est donnée sur la figure 31.

Ce plasmide, lorsqu'il est transféré dans des bactéries, induit leur résistance à la kanamycine à la concentration de 50 µg/ml. Ce plasmide constitue ainsi une sorte de vecteur navette pouvant être sélectionné aussi bien sur bactéries sous la forme de plasmide que sur cellules aviaires sous forme de provirus.

d) Production de virus dérivés du plasmide pXJ12

Par cotransformation de l'ADN du plasmide pXJ12 et de l'ADN contenant les génomes de virus helper RAV-1 ou RAV-2 sur fibroblastes de poulet, on a pu récupérer des virions AEV-XJ1 (RAV-1) et AEV-XJ12 (RAV-2). Ces virions peuvent infecter des fibroblastes de poulet in vitro et induire leur résistance à la drogue G418.

Le virus AEV-XJ12 constitue donc un vecteur viral sélectionnable. Les gènes à transférer pourront être insérés dans la région encore occupée par les séquences v-erbA.

e) Expression des gènes viraux dans les fibroblastes de poulet infectés par les vecteurs AEV-XJ1 et AEV-XJ12

Afin de vérifier si l'insertion du gène Neo$^r$ dans le génome viral n'altère pas les mécanismes de transcription, on a analysé les ARN produits dans des fibroblastes de poulet infectés par les vecteurs AEV-XJ1 (vecteur contenant plusieurs ATG) et AEV-XJ12.

On retrouve dans ces cellules deux types d'ARN correspondant aux ARN génomiques et sous-génomiques, ce qui démontre que la maturation des ARN est correcte. Cependant, dans les cellules infectées par AEV-XJ1, on observe une prédominance de l'ARN sous-génomique qui code pour la protéine Neo$^r$. Les expressions relatives des ARN génomiques et sous-génomiques ne sont pas sensiblement différentes dans les cellules cultivées en présence ou en l'absence de G418. Par contre, dans les fibroblastes infectés par AEV-XJ12, on observe sensiblement plus d'ARN génomique que d'ARN sous-génomique, dans un rapport qui est identique à celui observé pour l'AEV sauvage.

f) Méthode de titrage de virus aviaires portant le gène Neo$^r$

Le titrage biologique de virus portant un gène Neo$^r$ repose sur l'induction de la résistance au G418 de cellules infectées. Aucune méthode standard n'a jusqu'à présent été proposée pour les virus aviaires. On a développé une méthode qui repose sur l'utilisation de fibroblastes d'embryon de poulet de souche Leghorn Spafas C/O. Ces cellules sont sensibles à tous les sous-groupes rétroviraux aviaires.

En pratique, des fibroblastes secondaires en culture sont infectés avec des dilutions croissantes de suspension de virus, puis cultivés en présence de G418. Le titre du virus en r-ffu/ml (resistant focus forming unit) représente le produit du nombre de foyers résistants par culture par la dilution de la suspension virale utilisée pour l'infection.

Un point critique de ce test est le moment d'addition de la drogue par rapport au moment de l'infection. L'addition de la drogue ne doit pas être trop tardif pour éviter les infections secondaires ce qui conduirait à surestimer le titre du virus, ni trop précoce afin de permettre l'expression de la protéine Neo$^r$ en quantité suffisante dans les cellules infectées afin de les rendre résistantes.

Une analyse cinétique montre que l'estimation du titre par ce test est identique lorsque la drogue est ajoutée dans les 24 heures qui suivent l'infection. La dose de G418 utilisée pour les fibroblastes de poulet Spafas est de 200 μg/ml.

## Exemple 18

### Développement de cellules helper aviaires

Cet exemple concerne des cultures de cellules helper aviaires dans lesquelles les 3 gènes gag, pol et env s'expriment mais ne peuvent pas s'encapsider dans les virions produits.

L'ensemble des gènes gag-pol-env a été isolé en un fragment unique d'ADN à partir d'un plasmide contenant le génome du virus helper RAV-1 fourni par J. M. Bishop (UCSF). Ce fragment est limité en 5' par un site Sacl situé à 146 nucléotides en amont du codon initiateur du gène gag, et en 3' par un site Accl situé à 120 nucléotides en aval du codon de terminaison du gène env. Les séquences responsables de l'encapsidation des génomes viraux aviaires ne sont pas présentes dans le fragment considéré. Ce fragment a été inséré dans des plasmides permettant l'expression de gènes dans des cellules eucaryotiques.

Dans le plasmide HP1, l'ensemble gag-pol-env a été cloné entre le promoteur du gène TK du virus HSVI et le signal de polyadénylation du même virus tous deux isolés du plasmide pAG60 de Colbère-Garapin et al. (1981).

Dans le plasmide HP2, les gènes gag-pol-env ont été insérés entre le promoteur-enhancer du gène précoce du virus SV40 et une séquence contenant l'intron du gène t et le signal de polyadénylation du virus SV40, tous isolés du plasmide pSV2gpt de Mulligan et Berg (1981).

Ces plasmides ont été transfectés sur des cellules de caille, lignée QT6, et des fibroblastes de poulet. Afin de sélectionner les cellules transfectées, les ADN de ces plasmides ont été cotransfectées avec l'ADN de plasmides portant un marqueur de sélection, soit le plasmide pAG60, soit le plasmide pXJ12. Les foyers de cellules résistantes après sélection par G418 ont été prélevés et cultivés isolément. D'autres ont été regroupés et cultivés en mélange. La production de particules virales par ces cellules a été testée en cherchant la présence de réverse transcriptase sous forme particulaire dans les surnageants des cultures. Cette activité a été comparée à celle des surnageants des cellules érythroleucémiques 6C2 de poulet hautement productrices de virus AEV. Sur 9 cultures de cellules QT6 transfectées avec HP1 et HP2, 7 présentent une activité réverse transcriptase significative dont 5 montrent une activité supérieure à celle des cellules 6C2.

Ces résultats démontrent :

— d'une part, que l'expression des gènes gag, pol et env est possible à partir d'un promoteur autre que celui contenu dans les LTR virales,

— d'autre part, que l'on peut produire des virions extra-cellulaires à partir de génomes viraux helper dépourvus des LTR et des séquences d'encapsulation.

Les vecteurs selon la présente invention permettent de préparer des cellules résistantes au G418 et ont permis de cloner et d'exprimer la globine humaine, en particulier la delta globine humaine, les expériences sur les autres globines ayant donné des résultats équivalents.

### Dépôt de souches

Les souches suivantes ont été déposées à la Collection Nationale de Cultures de Microorganismes de l'Institut Pasteur, 28 rue du Docteur-Roux, 75724 PARIS CEDEX 15, sous les numéros suivants :

« Souche E. coli 600 pAEV TSN N° I-492
Souche E. coli 600RS pAEVΔ28 N° I-493
Souche E. coli 600RS pAEVXJ12 N° I-494 »

le 9 octobre 1985.

### Références

Beck E., Ludwig C., Auerswald E. A., Reiss B. et Schaller H. (1982). Nucleotide sequence and exact location of the neomycin. Gene 19 : 327-336.

Kawai S. et Nishizawa M. (1984). New procedure for DNA transfection with polycation and dimethyl sulfoxide. Mol. Cell. Biol. 4 : 1172-1174.

Messing J. et Vicira J. (1982). A new pair of M13 vectors for selecting either DNA strand of double-digest restriction fragments. Gene 19 : 269-276.

Sanger F., Nicklen S. et Coulson A. R. (1977). DNA sequencing with chain terminating inhibitors. Proc. Natl. Acad. Sci. 74 : 5463-5467.

Sharp P. A. (1981). Speculation on RNA splicing. Cell 23 : 643-646.

Auffray C. et Rougeon F. (1980). Purification of Mouse Immunoglobulin Heavy Chain Messenger. RNAs from Total Myeloma Tumor RNA. Eur. J. Biochem. 107 : 303-314.

Birnboim H. C. et Doly J. (1979). A rapid alkaline extraction procedure for screening recombinant plasmid DNA. Nucl. Acid. Res. 7 : 1513-1523.

Boone L. R. et Skalka A. M. (1981). Viral DNA Synthetized in vitro by Avian Retrovirus Particles Permeabilized with Melittin. J. Virology 37 (1) 117-126.

Colbere-Garapin F., Horodniceanu F., Kourilsky P. et Colbere-Garapin A. (1981). A new Dominant Hybrid Selectable Marker for Higher Eukryotic Cells J. Mol. Biol. 150 : 1-14.

Cullen B. R., Lomedico P. T. et Ju G. (1984). Transcriptional Interference in Avian Retroviruses Implication for the promoter Insertion Model of Leukemogenesis. Nature 307 : 241-245.

El-Gewely M. R. et Helling R. B. (1980). Preparative separation of DNA Ethidium Bromide Complexes Zonal Density Gradient Centrifugation. Anal. Biochem. 102 : 423-428.

Graham F. L. et Van der eb A. T. (1973). A new Technique for the Assay of Infectivity of Human Adenovirus 5 DNA. Virology 52 : 456-467.

Lehrach H., Diamon D., Wozney J. M. et Boedtker H. (1977). RNA Molecular Weight Determination by Gel Electrophoresis under Denaturing Conditions a Critical Reexamination. Biochem 16 : 4763-4751.

Longacre S. S. et Rutter W. (1977). Isolation of Specific DNA Sequences by Sulphydryl Sepharose Chromatography of Mercurated Polynucleotides. J. Biol. Chem. 252 : 2742-2752.

Maniatis T., Fritsh E. F. et Sambroock J. (1982). Molecular Cloning. A Laboratory Manual (cold Spring Harbor NEW-YORK : Cold Spring Harbor Laboratory).

McMaster M. et Carmichael G. G. (1977). Analysis of Single and Double stranted Nucleic Acids on Polyacrylamide and Agarose Gels by Using Glyoxal and Acridine Orange. Proc. Natl. Acad. Sci. USA 74 : 4835-4838.

Miller A. D., Jolly D. J., Friedman T. et Verma M. (1983). A Transmissible Retrovirus Expressiong Human Hypoxanthine Phosphoribosyl transferase (HPRT) : Gene Transfer into Cells Obtained from Human Deficient in HPRT. Proc. Natl Acad. Sci. USA 80 : 4709-4713.

Poncz M., Schwartz E., Ballatine M. et Surrey S., (1983). Nucleotide Sequence Analysis of the delta beta Globin Gene Region in Human. J. Biol. Chem. 258 : 11599-11609.

Rigby P. W. J., Diekman M., Rhodes C. et Berg P. (1977). Labelling Deoxyribonucleic Acid to High Specific Activity in vitro by Nick Translation with DNA Polymerase. J. Mol. Biol. 113 : 237-251.

Southern E. M. (1975). Detection of Specific Sequence Among DNA Fragments Separated by Gel Electrophoresis. J. Mol. Biol. 98 : 503-517.

Tautz D. et Renz M. (1983). An Optimized Freeze-Squeeze Method for the Recovery of DNA Fragments from Agarose Gels. Anal. Biochem. 132 : 14-19.

Thomas P. S. (1980). Hybridation of Denatured RNA and Small DNA Fragments Transfered to Nitrocellulose. Proc. Natl. Acad. Sci. USA 77 (9) : 5201-5209.

Vennstrom B., Fanshir L., Moscovici C. et Bishop J. M. (1980). Molecular Cloning of the Erythroblastosis virus Genome and Recovery of Oncogenic Virus by Transfection of Chicken Cells. J. Virols. 36 : 575-587.

Wigler M., Silverstein S., Lee L. S., Pellicer A., Cheng Y. C. et Axel R. (1977). Transfer of Purified Herpes Virus Thymidine Kinase Gene to Cultures Cells. Cell 11 : 223-232.

Yamamoto T., Nishida T., Miyajima N., Kawai S., OOI T. et Toyoshima K. (1983). The erbB gene of Avian Erythroblastosis Virus is a Member of the src Gene Family. Cell 35 : 71-78.

Sanger F., Wicklen J., Coulson A. R. (1977). DNA sequencing with chain terminating inhibitors, Proc. Natl. Acad. Sci. USA 74, 5463-5467.

Sorge J., Wright D., Erdman V. D., Cutting A. E. (1983). Amphotropic retrovirus vector system for human cell gene transfert. Mol. Cell. Biol. 4, 1730-1737.

Yamamoto T., Hinara H., Nishida T., Kawai S., Tayashima K. (1983). A new avian erythroblastoris virus AEV-H carries erb-B gene responsible for the induction of both erythroblastosis and sarcomas. Cell 34, 225-232.

Biggin M. D., Gibson T. J., Hong G. F. (1983). Buffer gradient gels and 35S label as aid to rapid DNA sequence determination. Proc. Natl. Acad. Sci. USA 80, 3963-3965.

Bolivar F., Rodriguez R. L., Green P. J., Betlach M. C., Heynecker H. L., Boyer H. W., Crosa J. H., Falkow S. (1977). Construction and characterization of new cloning vehicules. A multiple purpose cloning system. Gene 2, 95-113.

Debuire B., Henry C., Benaissa M., Biserte G., Claverie J. M., Saule S., Martin P., Stehelin D. (1984). Sequencing the erb-A gene of avian erythroblastosis virus reveals a new type of oncogene. Science 224, 1456-1459.

Dente L., Cesareni G., Cortese R. (1983). pEMBL : a new family of single stranded plasmids. Nucl. Acids Res. 11, 1645-1655.

**Revendications** (pour les Etats contractants : BE, CH, DE, FR, GB, IT, LI, LU, NL, SE)

1. Virus de clonage ou d'expression d'un gène étranger, caractérisé en ce qu'il est constitué par tout ou partie du génome d'un rétrovirus et comporte au moins un gène étranger situé entre les séquences LTR, le rétrovirus étant le virus de l'érythroblastose aviaire ou un virus aviaire apparenté.

2. Virus selon les revendications 1, caractérisé en ce qu'il présente un caractère oncogène.

3. Virus selon la revendication 2, caractérisé en ce que la séquence v-erbB a été conservée.

4. Virus selon l'une des revendications 1 à 3, caractérisé en ce que le gène étranger a été inséré entre la fin du gène v-erbB et la séquence LTR en aval.

5. Virus selon l'une des revendications 1 à 4, caractérisé en ce que le gène étranger est orienté dans le sens de lecture du génome viral.

6. Virus selon l'une des revendications 1 à 5, caractérisé en ce que le gène étranger est orienté dans le sens opposé au sens de lecture du génome viral.

7. Virus selon l'une des revendications 1 à 6, caractérisé en ce que le gène étranger est sous la dépendance d'un promoteur du rétrovirus.

8. Virus selon la revendication 7, caractérisé en ce que le virus vecteur est le virus pAEV2LTR tel que défini à la figure 5.

9. Virus selon la revendication 1, caractérisé en ce qu'il est dépourvu de caractère oncogène.

10. Virus selon la revendication 9, caractérisé en ce que le gène v-erbB a été au moins partiellement délété.

11. Virus selon l'une des revendications 9 et 10, caractérisé en ce qu'au moins un gène étranger est inséré en aval du gène v-erbA.

12. Virus selon la revendication 9, caractérisé en ce que les gènes v-erbB et v-erbA ont été délétés.

13. Virus selon la revendication 12, caractérisé en ce que deux gènes étrangers sont insérés entre les deux séquences LTR.

14. Virus selon la revendication 12, caractérisé en ce que l'un des gènes étrangers est inséré entre les deux sites d'épissage et l'autre gène étranger est inséré à l'extérieur des sites d'épissage et en aval de ceux-ci.

15. Virus selon l'une des revendications 13 et 14, caractérisé en ce que les deux gènes étrangers insérés sont chacun, de façon indépendante et dans un rapport donné, sous le contrôle d'un même promoteur.

16. Virus selon la revendication 15, caractérisé en ce que parmi les deux gènes étrangers insérés, l'un fournit un caractère sélectionnable.

17. Virus selon les revendications 9 à 16, caractérisé en ce qu'il comporte au moins un gène marqueur, en particulier un gène de résistance à un antibiotique, qui s'exprime dans les cellules.

18. Virus selon les revendications 9 à 17, caractérisé en ce que au moins un gène étranger inséré est choisi parmi les gènes humains de globine delta, de globine béta, de globine alpha et le gène d'origine bactérienne Neo$^R$.

19. Virus selon les revendications 1 à 18, caractérisé en ce que le ou les gènes étrangers insérés comportent en amont leurs propres éléments d'expression dans les cellules eucaryotes.

20. Virus selon l'une des revendications 1 à 19, caractérisé en ce que les gènes étrangers insérés sont sous la dépendance d'un élément d'expression viral.

21. Virus selon l'une des revendications 12 à 20, caractérisé en ce que le virus de clonage et d'expression en lui-même comporte au moins un site de restriction unique entre les deux sites d'épissage et un site de restriction unique à l'extérieur et en aval des sites d'épissage.

22. Virus selon la revendication 21, caractérisé en ce que le site de restriction unique à l'extérieur des sites d'épissage est un site NdeI situé au niveau du codon d'initiation du gène v-erbB.

23. Virus selon la revendication 21, caractérisé en ce que les gènes insérés sont sous la dépendance du promoteur de la séquence leader.

24. Plasmide vecteur de clonage ou d'expression d'un gène étranger, caractérisé en ce qu'il comporte une séquence composite d'ADN transcrit réverse d'un virus selon l'une des revendications 1 à 23.

25. Plasmide selon la revendication 24, caractérisé en ce qu'il comporte une séquence d'ADN d'un plasmide bactérien, d'un phage ou d'un cosmide.

26. Plasmide selon la revendication 25, caractérisé en ce que la séquence d'ADN de plasmide bactérien comporte au moins une origine de réplication dans les bactéries et un gène marqueur de sélection dans les bactéries.

27. Procédé de modification de cellules eucaryotes, caractérisé en ce qu'on effectue une infection des cellules avec des virus selon l'une des revendications 1 à 23.

28. Procédé selon la revendication 27, caractérisé en ce qu'on effectue une coinfection avec un « virus assistant » qui comporte les gènes assurant la réplication du virus de clonage ou d'expression.

29. Procédé de modification de cellules eucaryotes caractérisé en ce qu'on effectue une transfection des cellules avec des plasmides, selon l'une des revendications 24 à 26.

30. Procédé selon la revendication 29, caractérisé en ce qu'on effectue une cotransfection des cellules avec un « plasmide assistant » dont les gènes assurent la réplication du vecteur de clonage ou d'expression.

31. Cellule modifiée obtenue par la mise en œuvre d'un procédé selon l'une des revendications 27 à 30.

32. Cellule selon la revendication 31, caractérisée en ce qu'il s'agit de cellules aviaires.

33. Cellule selon la revendication 32, caractérisée en ce qu'il s'agit de cellules hématopoïétiques ou de cellules germinales.

EP 0 178 996 B1

34. Cellule selon la revendication 31, caractérisée en ce qu'il s'agit de fibroplastes embryonnaires de poulet.

35. Cellule selon l'une des revendications 31 à 34, caractérisé en ce que le gène étranger est intégré dans le génome de la cellule sous forme de provirus.

36. Cellule selon la revendication 35, caractérisée en ce qu'il s'agit d'une cellule non-aviaire.

37. Procédé de préparation d'au moins une protéine déterminée par culture de cellules selon l'une des revendications 31 à 36, le gène étranger codant pour ladite protéine, la protéine étant récupérée après culture.

**Revendications** (pour l'Etat contractant AT)

1. Procédé de préparation d'un virus de clonage ou d'expression d'un gène étranger, caractérisé en ce qu'on insère au moins un gène étranger entre les séquences LTR d'un virus constitué par tout ou partie du génome d'un rétrovirus, le rétrovirus étant le virus de l'érythroblastose aviaire ou un virus apparenté.

2. Procédé selon la revendication 1, caractérisé en ce qu'il présente un caractère oncogène.

3. Procédé selon la revendication 2, caractérisé en ce que la séquence v-erbB a été conservée.

4. Procédé selon l'une des revendications 1 à 3, caractérisé en ce que le gène étranger a été inséré entre la fin du gène v-erbB et la séquence LTR en aval.

5. Procédé selon l'une des revendications 1 à 4, caractérisé en ce que le gène étranger est orienté dans le sens de lecture du génome viral.

6. Procédé selon l'une des revendications 1 à 5, caractérisé en ce que le gène étranger est orienté dans le sens opposé au sens de lecture du génome viral.

7. Procédé selon l'une des revendications 1 à 6, caractérisé en ce que le gène étranger est sous la dépendance d'un promoteur du rétrovirus.

8. Procédé selon la revendication 7, caractérisé en ce que le virus vecteur est le virus pAEV2LTR.

9. Procédé selon la revendication 1, caractérisé en ce qu'il est dépourvu de caractère oncogène.

10. Procédé selon la revendication 9, caractérisé en ce que le gène v-erbB a été au moins partiellement délété.

11. Procédé selon l'une des revendications 9 et 10, caractérisé en ce qu'au moins un gène étranger est inséré en aval du gène v-erbA.

12. Procédé selon la revendication 9, caractérisé en ce que les gènes v-erbB et v-erbA ont été délétés.

13. Procédé selon la revendication 12, caractérisé en ce que les deux gènes étrangers sont insérés entre les deux séquences LTR.

14. Procédé selon la revendication 12, caractérisé en ce que l'un des gènes étrangers est inséré entre les deux sites d'épissage et l'autre gène étranger est inséré à l'extérieur des sites d'épissage et en aval de ceux-ci.

15. Procédé selon l'une des revendications 13 et 14, caractérisé en ce que les deux gènes étrangers insérés sont chacun, de façon indépendante et dans un rapport donné, sous le contrôle d'un même promoteur.

16. Procédé selon la revendication 15, caractérisé en ce que parmi les deux gènes étrangers insérés, l'un fournit un caractère sélectionnable.

17. Procédé selon les revendications 9 à 16, caractérisé en ce que le virus comporte au moins un gène marqueur, en particulier un gène de résistance à un antibiotique, qui s'exprime dans les cellules.

18. Procédé selon les revendications 9 à 17, caractérisé en ce que au moins un gène étranger inséré est choisi parmi les gènes humains de globine delta, de globine béta, de globine alpha et le gène d'origine bactérienne Neo^R.

19. Procédé selon les revendications 1 à 18, caractérisé en ce que le ou les gènes étrangers insérés comportent en amont leurs propres éléments d'expression dans les cellules eucaryotes.

20. Procédé selon l'une des revendications 1 à 19, caractérisé en ce que les gènes étrangers insérés sont sous la dépendance d'un élément d'expression viral.

21. Procédé selon l'une des revendications 12 à 20, caractérisé en ce que le virus de clonage et d'expression en lui-même comporte au moins un site de restriction unique entre les deux sites d'épissage et un site de restriction unique à l'extérieur et en aval des sites d'épissage.

22. Procédé selon la revendication 21, caractérisé en ce que le site de restriction unique à l'extérieur des sites d'épissage est un site Ndel situé au niveau du codon d'initiation du gène v-erbB.

23. Procédé selon la revendication 21, caractérisé en ce que les gènes insérés sont sous la dépendance du promoteur de la séquence leader.

24. Procédé de préparation d'un plasmide vecteur de clonage ou d'expression d'un gène étranger, caractérisé en ce qu'on effectue une transcription réverse d'une séquence composite d'ADN d'un virus préparé selon le procédé d'une des revendications 1 à 23.

25. Procédé de préparation d'un plasmide selon la revendication 24, caractérisé en ce que la séquence d'ADN provient d'un plasmide bactérien, d'un phage ou d'un cosmide.

26. Procédé de préparation d'un plasmide selon la revendication 25, caractérisé en ce que la

32

EP 0 178 996 B1

séquence d'ADN de plasmide bactérien comporte au moins une origine de réplication dans les bactéries et un gène marqueur de sélection dans les bactéries.

27. Procédé de modification de cellules eucaryotes, caractérisé en ce qu'on effectue une infection des cellules avec des virus préparés selon l'une des revendications 1 à 23.

28. Procédé selon la revendication 27, caractérisé en ce qu'on effectue une coinfection avec un « virus assistant » qui comporte les gènes assurant la réplication du virus de clonage ou d'expression.

29. Procédé de modification de cellules eucaryotes, caractérisé en ce qu'on effectue une transfection des cellules avec des plasmides, selon l'une des revendications 24 à 26.

30. Procédé selon la revendication 29, caractérisé en ce qu'on effectue une cotransfection des cellules avec un « plamide assistant » dont les gènes assurent la réplication du vecteur de clonage ou d'expression.

31. Procédé de préparation d'au moins une protéine déterminée par culture de cellules modifiées obtenues par la mise en oeuvre d'un procédé selon l'une des revendications 27 à 30, le gène étranger codant pour ladite protéine, la protéine étant récupérée après culture.

32. Procédé selon la revendication 31, caractérisé en ce qu'il s'agit de cellules aviaires.

33. Procédé selon la revendication 32, caractérisé en ce qu'il s'agit de cellules hématopoïétiques ou de cellules germinales.

34. Procédé selon la revendication 31, caractérisé en ce qu'il s'agit de fibroplastes embryonnaires de poulet.

35. Procédé selon l'une des revendications 31 à 34, caractérisé en ce que le gène étranger est intégré dans le génome de la cellule sous forme de provirus.

36. Procédé selon la revendication 35, caractérisé en ce qu'il s'agit d'une cellule non-aviaire.

**Claims** (for the Contracting States : BE, CH, DE, FR, GB, IT, LI, LU, NL, SE)

1. A cloning virus or expression of a foreign gene, characterised in that it comprises all or part of a genome of a retrovirus and comprises at least a foreign gene situated between the sequences LTR, the retrovirus being the virus of avian erythroblastosis or a related avian virus.

2. A virus according to claim 1 characterised in that it presents an oncogene character.

3. A virus according to claim 2, characterised in that the sequence v-erbB is preserved.

4. A virus according to one of claims 1 to 3 characterised in that the foreign gene is inserted between the end of the gene v-erbB and the downstream LTR sequence.

5. A virus according to one of claims 1 to 4 characterised in that the foreign gene is orientated in a sense of reading of the viral genome.

6. A virus according to one of claims 1 to 5 characterised in that the foreign gene is orientated in the opposite sense to the reading sense of the viral genome.

7. A virus according to one of claims 1 to 6 characterised in that the foreign gene is under the control of a retrovirus promoter.

8. A virus according to claim 7 characterised in that the virus vector is the virus pAEV2LTR : such as defined in figure 5.

9. A virus according to claim 1 characterised in that it is devoid of oncogene character.

10. A virus according to claim 9, characterised in that the gene v-erbB is at least partially deleted.

11. A virus according to one of claims 9 and 10 characterised in that at least one foreign gene is inserted downstream of the gene v-erbA.

12. A virus according to claim 9, characterised in that the genes v-erbB and v-erbA are deleted.

13. A virus according to claim 12 characterised in that the two foreign genes are inserted between the two LTR sequences.

14. A virus according to claim 12 characterised in that one of the foreign genes is inserted between two splicing sites and the other foreign gene is inserted outside the spicing sites and downstream of them.

15. A virus according to one of claims 13 and 14 characterised in that the two inserted foreign genes are each, in an independant fashion and in a given relationship, under the control of the same promoter.

16. A virus according to claim 15 characterised in that amongst the two inserted foreign genes one furnishes a selectionable character.

17. A virus according to claims 9 to 16 characterised in that it comprises at least a marker gene, in particular a gene of resistance to an antibiotic, which self expresses in the cells.

18. A virus according to claims 9 to 17 characterised in that at least one inserted foreign gene is chosen amongst the human genes of delta globin, beta globin, alpha globin and the source gene of bacterial Neo[R].

19. A virus according to claims 1 to 18 characterised in that the or each inserted foreign gene comprises upstream their own elements of expression in eucaryotes cells.

20. A virus according to one of claims 1 to 19 characterised in that the inserted foreign genes are dependent on a viral expression element.

21. A virus according to one of claims 12 to 20 characterised in that the cloning virus and the

33

EP 0 178 996 B1

expression of it comprise at least a unique restriction site between the two splicing sites and a unique restriction site outside and downstream of the splicing sites.

22. A virus according to claim 21 characterised in that the unique restriction site outside the splicing sites is an NdeI site situated at the level of the initiation codon of the gene v-erbB.

23. A virus according to claim 21 characterised in that the inserted genes are dependant on a promoter of the sequence leader.

24. A plasmid cloning vector or expression of a foreign gene characterised in that it comprises a composite sequence of reverse transcript ADN of a virus according to one of claims 1 to 23.

25. A plasmid according to claim 24 characterised in that it comprises a sequence of ADN of a bacterial plasmid, a phage or a cosmid.

26. A plasmid according to claim 25 characterised in that the ADN sequence of bacterial plasmid comprises at least a source of replication in the bacteria and a marker gene for selection in the bacteria.

27. Process of modification of eucaryotes cells characterised in that one effects an infection of the cells with the virus according to one of claims 1 to 23.

28. Process according to claim 27 characterised in that one effects a coinfection with « assistant virus » which comprises the genes assuring replication of the cloning virus or expression.

29. Process of modification of eucaryotes cells characterised in that one effects a transfection of cells with plasmids according to one of claims 24 to 26.

30. Process according to claim 29 characterised in that one effects a cotransfections of cells with a « assistant plasmid » in which the genes assure replication of the cloning vector or expression.

31. A modified cell obtained by the putting into operation a process according to one of claims 27 to 30.

32. A cell according to claim 31 characterised in that the cell is a fowl cell.

33. A cell according to claim 32 characterised in that the cell is a haemopoiesis cell or germinal cell.

34. A cell according to claims 31 characterised in that the cell is an embryonic fibroplast.

35. A cell according to one of claims 31 to 34 characterised in that the foreign gene is intergrated in the genome of the cell in the form of a provirus.

36. A cell according to claim 35 characterised in that the cell is a non-fowl cell.

37. Process of preparation of at least a predetermined protein by culture of cells according to one of claims 31 to 36 the foreign gene coding for the said protein, the protein being recovered after culture.


**Claims** (for the Contracting State AT)

1. A process of preparation of a cloning virus or of expressing a foreign gene, characterised in that one inserts at least one foreign gene between the LTR sequences of a virus comprising all or part of a genome of a retrovirus, the retrovirus being the virus of an avian erythroblastosis or a related virus.

2. Process according to claim 1 characterised in that it has an oncogene character.

3. Process according to claim 2 characterised in that the sequence v-erbB is preserved.

4. Process according to one of claims 1 to 3, characterised in that the foreign gene is inserted between the end of gene v-erbB and the downstream LTR sequence.

5. Process according to one of claims 1 to 4, characterised in that the foreign gene is orientated in the sense of reading of the viral genome.

6. Process according to one of claims 1 to 5, characterised in that the foreign gene is orientated in the opposite sense to the reading sense of a viral genome.

7. Process according to one of claims 1 to 6, characterised in that the foreign gene is dependent on a retrovirus promoter.

8. Process according to claim 7, characterised in that the virus vector is the virus pAEV2LTR.

9. Process according to claim 1, characterised in that it is devoid of oncogene character.

10. Process according to claim 9, characterised in that the gene v-erbB is at least partially deleted.

11. Process according to one of claims 9 and 10, characterised in that at least one foreign gene is inserted downstream of the gene v-erbA.

12. Process according to claim 9, characterised in that the genes v-erbB and v-erbA are deleted.

13. Process according to claim 12, characterised in that the two foreign genes are inserted between the two LTR sequences.

14. Process according to claim 12, characterised in that one of the foreign genes is inserted between the two splicing sites and the other foreign gene is inserted outside the splicing sites and downstream of them.

15. Process according to one of claims 13 and 14, characterised in that the two foreign genes inserted are each in an independent fashion and in a given relationship, under the control of the same promoter.

16. Process according to claim 15, characterised in that amongst the two inserted foreign genes, one furnishes a selectionable character.

17. Process according to claims 9 to 16, characterised in that the virus comprises at least a marker gene, in particular a gene of resistance to an antibiotic, which self expresses in the cells.

34

18. Process according to claims 9 to 17, characterised in that at least one inserted foreign gene is chosen amongst the human genes delta globin, beta globin, alpha globin and the source gene of bacterial Neo$^R$.

19. Process according to claims 1 to 18, characterised in that the or each inserted foreign gene comprise upstream their own elements of expression in eucaryotes cells.

20. Process according to one of claims 1 to 19 characterised in that the inserted genes are dependent on a viral expression element.

21. Process according to one of claims 12 to 20, characterised in that the cloning virus and the expression of it comprise at least a unique restriction site between two splicing sites and a unique restriction site outside and downstream of the splicing sites.

22. Process according to claim 21, characterised in that the unique restriction site outside the splicing sites is an NdeI site situated at the level of an initiation codon of the gene v-erbB.

23. Process according to claim 21, characterised in that the inserted genes are dependent on a promoter of the sequence leader.

24. Process preparation of a plasmid cloning vector or expression of a foreign gene, characterised in that one effects a reverse transcription of a composite sequence of a ADN of a virus prepared according to the process of one of claims 1 to 23.

25. Process of preparation of a plasmid according to claim 24, characterised in that ADN sequence originates from a bacterial plasmid, a phage or a cosmid.

26. Process of preparation of a plasmid according to claim 25, characterised in that the bacterial plasmid ADN sequence comprises at least a source of replication in bacteria and a marker gene for selection in the bacteria.

27. Process of modification of eucaryote cells characterised in that one effects an infection of the cell with a virus prepared according to one of claim 1 to 23.

28. Process according to claim 27, characterised in that one effects a coinfection with an « assistant virus » which comprises the genes assuring replication of the cloning virus or expression.

29. Process of modification of eucaryotes cells, characterised in that one effects a transfection of cells with plasmids according to one of claims 24 to 26.

30. Process according to claim 29, characterised in that one effects a cotransfection of the cells with an « assistant plasmid » the genes of which assure replication of the cloning vector or expression.

31. Process of preparation of at least a predetermined protein by culture of modified cells obtained by the use of a process according to one of claims 27 to 30, the foreign gene coding for the said protein, the protein being recovered after culture.

32. Process according to claim 31, characterised in that the cells are fowl cells.

33. Process according to claim 32, characterised in that the cells or haemopoiesis cells or germinal cells.

34. Process according to claim 31, characterised in that the cells are embryonic fibroplasts.

35. Process according to one of claims 31 to 34, characterised in that the foreign gene is integrated into the genome of the cell in the form of a provirus.

36. Process according to claim 35, characterised in that the cell is a non-fowl cell.


**Patentansprüche** (für die Vertragsstaaten : BE, CH, DE, ES, FR, GB, IT, LI, LU, NL, SE)

1. Klonierungs- oder Expressionsvirus eines fremden Gens, dadurch gekennzeichnet, daß es ganz oder teilweise aus dem Genom eines Retrovirus besteht und wenigstens ein fremdes Gen zwischen den Sequenzen LTR aufweist, wobei das Retrovirus das Virus der Geflügelerythroblastose oder ein verwandtes Virus ist.

2. Virus nach Anspruch 1, dadurch gekennzeichnet, daß es einen onkogenen Charakter aufweist.

3. Virus nach Anspruch 2, dadurch gekennzeichnet, daß die Sequenz v-erbB bewahrt wurde.

4. Virus nach einem der Ansprüche 1 bis 3, dadurch gekennzeichnet, daß das fremde Gen stromabwärts zwischen dem Ende des Gens v-erbB und der Sequenz LTR eingesetzt wurde.

5. Virus nach einem der Ansprüche 1 bis 4, dadurch gekennzeichnet, daß das fremde Gen in Leserichtung des viralen Genoms ausgerichtet ist.

6. Virus nach einem der Ansprüche 1 bis 5, dadurch gekennzeichnet, daß das fremde Gen entgegen der Leserichtung des viralen Genoms ausgerichtet ist.

7. Virus nach einem der Ansprüche 1 bis 6, dadurch gekennzeichnet, daß das fremde Gen unter der Kontrolle von einem Promotor des Retrovirus ist.

8. Virus nach Anspruch 7, dadurch gekennzeichnet, daß das Vektorvirus das Virus pAEV2LTR ist, wie es in Fig. 5 definiert wurde.

9. Virus nach Anspruch 1, dadurch gekennzeichnet, daß es keinen onkogenen Charakter hat.

10. Virus nach Anspruch 9, dadurch gekennzeichnet, daß das Gen v-erbB wenigstens teilweise zerstört wurde.

11. Virus nach einem der Ansprüche 9 und 10, dadurch gekennzeichnet, daß wenigstens ein fremdes Gen stromabwärts des Gens v-erbA eingesetzt wurde.

12. Virus nach Anspruch 9, dadurch gekennzeichnet, daß die Gene v-erbB und v-erbA zerstört wurden.

13. Virus nach Anspruch 12, dadurch gekennzeichnet, daß die beiden fremden Gene zwischen den beiden Sequenzen LTR eingesetzt sind.

14. Virus nach Anspruch 12, dadurch gekennzeichnet, daß eines der fremden Gene zwischen die beiden Spleißstellen und das andere fremde Gen außerhalb und stromabwärts der Spleißstellen eingesetzt ist.

15. Virus nach einem der Ansprüche 13 und 14, dadurch gekennzeichnet, daß die beiden Gene jeweils unabhängig in einem gegebenen Verhältnis unter der Kontrolle desselben Promotors eingesetzt sind.

16. Virus nach Anspruch 15, dadurch gekennzeichnet, daß von den beiden fremden eingesetzten Genen das eine ein selektionierbares Merkmal liefert.

17. Virus nach den Ansprüchen 9 bis 16, dadurch gekennzeichnet, daß das Virus wenigstens ein Markierungsgen, speziell ein Antibiotikaresistenzgen, das in den Zellen exprimiert wird, besitzt.

18. Virus nach den Ansprüchen 9 bis 17, dadurch gekennzeichnet, daß wenigstens ein fremdes eingesetztes Gen aus den menschlichen Genen Delta Globin, Beta Globin, Alpha Globin und dem Gen bakteriellen Ursprungs Neo$^R$ ausgewählt ist.

19. Virus nach den Ansprüchen 1 bis 18, dadurch gekennzeichnet, daß das oder die fremden eingesetzten Gene stromaufwärts ihre eigenen Expressionselemente in den eukariotischen Zellen aufweisen.

20. Virus nach einem der Ansprüche 1 bis 19, dadurch gekennzeichnet, daß die fremden eingesetzten Gene unter der Kontrolle eines viralen Expressionselements sind.

21. Virus nach einem der Ansprüche 12 bis 20, dadurch gekennzeichnet, daß das Klonierung- und Expressionsvirus in sich selbst wenigstens eine eindeutige Restriktionsschnittstelle zwischen den beiden Spleißstellen und eine eindeutige Restriktionsschnittstelle außerhalb und stromabwärts der Spleißstellen aufweist.

22. Virus nach Anspruch 21, dadurch gekennzeichnet, daß die eindeutige Restriktionsschnittstelle außerhalb der Spleißstellen eine NdeI-Schnittstelle ist, die auf dem Niveau des Initiations-Codons des Gens v-erbB liegt.

23. Virus nach Anspruch 21, dadurch gekennzeichnet, daß die eingesetzten Gene unter der Kontrolle des Promotors der Leader-Sequenz sind.

24. Plasmider Klonierungs- und Expressionsvektor eines fremden Gens, dadurch gekennzeichnet, daß er eine zusammengesetzte DNA-Sequenz einer reversen Transkription eines Virus nach einem der Ansprüche 1 bis 23 aufweist.

25. Plasmid nach Anspruch 24, dadurch gekennzeichnet, daß es eine Sequenz der DNA von einem bakteriellen Plasmid, von einem Phagen oder einem Cosmid aufweist.

26. Plasmid nach Anspruch 25, dadurch gekennzeichnet, daß die Sequenz der DNA des bakteriellen Plasmids wenigstens einen Ursprungspunkt der Replikation in den Bakterien und ein Selektionsmarkergen in den Bakterien aufweist.

27. Verfahren zur Modifikation eukaryotischer Zellen, dadurch gekennzeichnet, daß man eine Infektion der Zellen mit Viren nach einem der Ansprüche 1 bis 23 durchführt.

28. Verfahren nach Anspruch 27, dadurch gekennzeichnet, daß man eine Koinfektion mit einem « Helfervirus » durchführt, das die Gene aufweist, die die Replikation des Klonierungs- oder Expressionsvirus sicherstellen.

29. Verfahren zur Modifikation eukaryotischer Zellen, dadurch gekennzeichnet, daß man eine Transfektion der Zellen mit Plasmiden nach einem der Ansprüche 24 bis 26 durchführt.

30. Verfahren nach Anspruch 29, dadurch gekennzeichnet, daß man eine Kotransfektion der Zellen mit einem « Helferplasmid » durchführt, dessen Gene die Replikation des Klonierungs- oder Expressionsvirus sicherstellen.

31. Modifizierte Zelle, wie sie durch die Ausführung eines Verfahrens nach einem der Ansprüche 27 bis 30 erhalten wird.

32. Zelle nach Anspruch 31, dadurch gekennzeichnet, daß es sich um Geflügelzellen handelt.

33. Zelle nach Anspruch 32, dadurch gekennzeichnet, daß es sich um blutbildende Zellen oder Keimzellen handelt.

34. Zelle nach Anspruch 31, dadurch gekennzeichnet, daß es sich um embryonale Hühnerfibroplasten handelt.

35. Zelle nach einem der Ansprüche 31 bis 34, dadurch gekennzeichnet, daß das fremde Gen in das Genom der Zelle in der Form eines Provirus integriert ist.

36. Zelle nach Anspruch 35, dadurch gekennzeichnet, daß es sich um eine Nicht-Geflügelzelle handelt.

37. Verfahren zur Herstellung wenigstens eines Proteins, das durch eine Zellkultur nach einem der Ansprüche 31 bis 36, bestimmt ist, wobei das fremde Gen das Protein kodiert und das Protein nach der Kultur gewonnen wird.

**Patentansprüche** (für den Vertragsstaat AT)

1. Verfahren zur Herstellung eines Klonierungs- oder Expressionsvirus eines fremden Gens, dadurch gekennzeichnet, daß man wenigstens ein fremdes Gen zwischen die Sequenzen LTR eines ganz oder teilweise aus dem Genom eines Retrovirus bestehenden Virus einfügt, wobei das Retrovirus das Virus der Geflügelerythroblastose oder ein verwandtes Virus ist.

2. Verfahren nach Anspruch 1, dadurch gekennzeichnet, daß es einen onkogenen Charakter aufweist.

3. Verfahren nach Anspruch 2, dadurch gekennzeichnet, daß die Sequenz v-erbB bewahrt wurde.

4. Verfahren nach einem der Ansprüche 1 bis 3, dadurch gekennzeichnet, daß das fremde Gen stromabwärts zwischen dem Ende des Gens v-erbB der Sequenz LTR eingesetzt wurde.

5. Verfahren nach einem der Ansprüche 1 bis 4, dadurch gekennzeichnet, daß das fremde Gen in Leserichtung des viralen Genoms ausgerichtet ist.

6. Verfahren nach einem der Ansprüche 1 bis 5, dadurch gekennzeichnet, daß das fremde Gen entgegen der Leserichtung des viralen Genoms ausgerichtet ist.

7. Verfahren nach einem der Ansprüche 1 bis 6, dadurch gekennzeichnet, daß das fremde Gen unter der Kontrolle von einem Promotor des Retrovirus ist.

8. Verfahren nach Anspruch 7, dadurch gekennzeichnet, daß das Vektorvirus das Virus pAEV2LTR ist.

9. Verfahren nach Anspruch 1, dadurch gekennzeichnet, daß es keinen onkogenen Charakter hat.

10. Verfahren nach Anspruch 9, dadurch gekennzeichnet, daß das Gen v-erbB wenigstens teilweise zerstört wurde.

11. Verfahren nach einem der Ansprüche 9 und 10, dadurch gekennzeichnet, daß wenigstens ein fremdes Gen stromabwärts des Gens v-erbA eingesetzt wurde.

12. Verfahren nach Anspruch 9, dadurch gekennzeichnet, daß die Gene v-erbB und v-erbA zerstört wurden.

13. Verfahren nach Anspruch 12, dadurch gekennzeichnet, daß die beiden fremden Gene zwischen den beiden Sequenzen LTR eingesetzt sind.

14. Verfahren nach Anspruch 12, dadurch gekennzeichnet, daß eines der fremden Gene zwischen die beiden Spleißstellen und das andere fremde Gen außerhalb und stromabwärts der Spleißstellen eingesetzt ist.

15. Verfahren nach einem der Ansprüche 13 und 14, dadurch gekennzeichnet, daß die beiden Gene jeweils unabhängig in einem gegebenen Verhältnis unter der Kontrolle desselben Promotors eingesetzt sind.

16. Verfahren nach Anspruch 15, dadurch gekennzeichnet, daß von den beiden fremden eingesetzten Genen das eine ein selektionierbares Merkmal liefert.

17. Verfahren nach den Ansprüchen 9 bis 16, dadurch gekennzeichnet, daß das Virus wenigstens ein Markierungsgen, speziell ein Antibiotikaresistenzgen, das in den Zellen exprimiert wird, besitzt.

18. Verfahren nach den Ansprüchen 9 bis 17, dadurch gekennzeichnet, daß wenigstens ein fremdes eingesetztes Gen aus den menschlichen Genen Delta Globin, Beta Globin, Alpha Globin und dem Gen bakteriellen Ursprungs Neo$^R$ ausgewählt ist.

19. Verfahren nach den Ansprüchen 1 bis 18, dadurch gekennzeichnet, daß das oder die fremden eingesetzten Gene stromaufwärts ihre eigenen Expressionselemente in den eukariotischen Zellen aufweisen.

20. Verfahren nach einem der Ansprüche 1 bis 19, dadurch gekennzeichnet, daß die fremden eingesetzten Gene unter der Kontrolle eines viralen Expressionelements sind.

21. Verfahren nach einem der Ansprüche 12 bis 20, dadurch gekennzeichnet, daß das Klonierungs- und Expressionsvirus in sich selbst wenigstens eine eindeutige Restriktionsschnittstelle zwischen den beiden Spleißstellen und eine eindeutige Restriktionsschnittstelle außerhalb und stromabwärts der Spleißstellen aufweist.

22. Verfahren nach Ansprunch 21, dadurch gekennzeichnet, daß die eindeutige Restriktionsschnittstelle außerhalb der Spleißstellen eine NdeI-Schnittstelle ist, die auf dem Niveau des Initiations-Codons des Gens v-erbB liegt.

23. Verfahren nach Anspruch 21, dadurch gekennzeichnet, daß die eingesetzten Gene unter der Kontrolle des Promotors der Leader-Sequenz sind.

24. Verfahren zur Herstellung eines plasmiden Klonierungs- und Expressionsvektors eines fremden Gens, dadurch gekennzeichnet, daß man eine reverse Transkription einer zusammengesetzten DNA-Sequenz eines nach dem Verfahren eines der Ansprüche 1 bis 23 hergestellten Virus durchführt.

25. Verfahren zur Herstellung eines Plasmids nach Anspruch 24, dadurch gekennzeichnet, daß die Sequenz der DNA von einem bakteriellen Plasmid, von einem Phagen oder einem Cosmid herrührt.

26. Verfahren zur Herstellung eines Plasmids nach Anspruch 25, dadurch gekennzeichnet, daß die Sequenz der DNA des bakteriellen Plasmids wenigstens einen Ursprungspunkt der Replikation in den Bakterien und ein Selektionsmarkergen in den Bakterien aufweist.

27. Verfahren zur Modifikation eukaryotischer Zellen, dadurch gekennzeichnet, daß man eine Infektion der Zellen mit nach einem der Ansprüche 1 bis 23 hergestellten Viren durchführt.

28. Verfahren nach Anspruch 27, dadurch gekennzeichnet, daß man eine Koinfektion mit einem « Helfervirus » durchführt, das die Gene aufweist, die die Replikation des Klonierungs- oder Expressionsvirus sicherstellen.

29. Verfahren zur Modifikaticn eukaryotischer Zellen, dadurch gekennzeichnet, daß man eine Transfektion der Zellen mit nach einem der Ansprüche 24 bis 26 hergestellten Plasmiden durchführt.

30. Verfahren nach Anspruch 29, dadurch gekennzeichnet, daß man eine Kotransfektion der Zellen mit einem « Helferplasmid » durchführt, dessen Gene die Replikation des Klonierungs- oder Expressionsvirus sicherstellen.

31. Verfahren zur Herstellung wenigstens eines Proteins, das von einer Kultur modifizierter Zellen wie sie durch die Anwendung eines Verfahrens nach einem der Ansprüche 27 bis 30 erhalten werden, wobei das fremde Gen für das Protein kodiert und wobei das Protein nach der Kultur gewonnen wird.

32. Verfahren nach Anspruch 31, dadurch gekennzeichnet, daß es sich um Geflügelzellen handelt.

33. Verfahren nach Anspruch 32, dadurch gekennzeichnet, daß es sich um blutbildende Zellen oder Keimzellen handelt.

34. Verfahren nach Anspruch 31, dadurch gekennzeichnet, daß es sich um embryonale Hühnerfibroplasten handelt.

35. Verfahren nach einem der Ansrprüche 31 bis 34, dadurch gekennzeichnet, daß das fremde Gen in das Genom der Zelle in der Form eines Provirus intergriert ist.

36. Verfahren nach Anspruch 35, dadurch gekennzeichnet, daß es sich um eine Nicht-Geflügelzelle handelt.

FIG.1

EP 0 178 996 B1

FIG.2

FIG.3

FIG.4

2

FIG_5

FIG_6

FIG. 7

FIG.8

FIG.9

FIG_10

FIG. 11

FIG_12

FIG_13

FIG. 14

EP 0 178 996 B1

FIG.15

FIG.16

13

```
AEV-H 5'-AUG ——— CCA AAT GGC TCC AAA ACT CCA TCT ATC GCG GCT GGT
AEV   5'-AUG ——— --- --- --- --- --- --- --- --- --- --- --- ---
              GTT GTC GGA GGA CTC CTG TGC CTG GTT GTG GTT GGT
              --- --- --- --- --- --- --- --- --- --- --- ---
              CTA GGC ATC GGT CTT TAC CTG CGG CGA CGT CAT ATC
              --- --- --- --- --- --- --- --- --- --C --- ---
              GTG CGG AAG CGC ACC CTG CGC AGG CTG CTG CAA GAG
              --- --- --- --- --- --- --- --- --- --- --- ---
              AGG GAG CTT GTC GAA CCA CTG ACA CCC AGT GGG GAG
              --- --- --- --- --- --- --- --- --- --- --- ---
              GCA CCA AAC CAG GCC CAC CTG AGA ATT TTA AAG GAA
              --- --- --- --- --- --- --- --- --- --- --- ---
              ACA GAA TTT AAA AAG GTC AAA GTT TTG GGC TCT GGA
              --- --- --- --- --- --- --- --- --- --- -T- ---
              GCT TTT GGC ACT ATT TAT AAG GGA CTT TGG ATC AEV-H
              --- --- --- --- G-- --- --- --- --- --- --- .AEV
                                                      ———C
                                                      BamHI
```

# FIG. 17

```
5'-TAG TCCATCCTAC AAGAGCAAAA ACTTGTAAGC ATTTCAGGTA GCAAAGTAAT GAAACCACAA AATGCTGAAA ATGCTCCACA
v-erbA         10        20        30        40        50        60        70        80
       GTTGAATGCA CAGATATTTA TGTTCTGTGT TTATTCCTGA AGTGTAAGGA ATGTAAGGAA ATCATAGATT AGTTGTTTCT
              90       100       110       120       130       140       150       160
       TTCCTTTTTG CAGGGCCCAG ACCACTGC ATG AAG TGT GCT CAT TTT ATA GAT GGT CCC CAC TGT GTG AAG
             170       180       |◄────── v-erbB
p15-16 ─────────────────────────────────────────────────────────────────────────────────────
p15-28 ─────────────────────────────────────────────────────────────────────────────────────
p15-21 ─────────────────────────────────────────────────────────────────────────────────────
p15-25 ─────────────────────────────────────────────────────────────────────────────────────
       GCC TGC CCC GCT GGG GTC CTG GGT GAG---v-erbB--3'

p15-28 ──────────────                         |◄─────mp11
p15-21 ─────────────────────────────────────  |GG GCG AGC TCG AAT TCA CTG---mp11---
p15-25 ─────────────────────────────────────  |      SacI    EcoRI
```

## FIG_18

EP 0 178 996 B1

## (a)

1kb

## (b)

pXJ1

AUG  AUG  AUG  UGA

## (c)

FIG.19

## FIG. 20

## FIG. 21

Linker

5'-|ATG AAG TGT GCT CGG GCG AGC TCG AAT T|CA| GAT CTG ATC AAG

|←———— erbB ————→|←———— mpII ————→|

SacI        EcoRI    BglII

AGA CAG GAT GAG GAT CGT TTC GC | A'TG ATT GAA --- Neo^r ---

————————— Tn5 —————————→|←———— Neo^r ————————

TGA| --- TTG GTC|AAC AGC GTG CCG CAG ATC TG| AAT TCC CAC

———→|←—— Tn5 ——→|←—— tk ———→|Linker |←————

HincII        BglII

EcoRI

AGC ACA GCT GTG GAC AAC CCT GAG TAT CTT GAG TGA| TCA CAG

—————————— erbB ———————————————→|←——

TGT AGT CTA TAC AGA AGA AGT TCC AGC TAA TGA AGG AAC ATG

———————— AEV ————————————

TGA ATA AGA --- AEV ---3'|

————————————————→|

# FIG. 22

18

FIG_23

FIG.24

FIG. 25

FIG. 26

FIG_27

EP 0 178 996 B1

FIG.28

EP 0 178 996 B1

EP 0 178 996 B1

FIG.29

FIG_30

(a)

(b)

(c)

FIG_31